(19) Europäisches Patentamt
European Patent Office
Office européen des brevets

(11) EP 3 654 966 B1

(12) **EUROPEAN PATENT SPECIFICATION**

(45) Date of publication and mention of the grant of the patent:
**04.12.2024 Bulletin 2024/49**

(21) Application number: **18835224.9**

(22) Date of filing: **20.07.2018**

(51) International Patent Classification (IPC):
***A61K 9/14*** $^{(2006.01)}$ ***A61K 31/353*** $^{(2006.01)}$
***A61K 47/34*** $^{(2017.01)}$ ***A61K 47/38*** $^{(2006.01)}$

(52) Cooperative Patent Classification (CPC):
(C-Sets available)
**A61K 9/146; A23L 29/262; A23L 33/105; A23L 33/24; A61K 31/353; A61K 47/38;**
A23V 2002/00; A61K 47/34 (Cont.)

(86) International application number:
**PCT/US2018/043107**

(87) International publication number:
**WO 2019/018774 (24.01.2019 Gazette 2019/04)**

(54) **AMORPHOUS DISPERSIONS OF EPIGALLOCATECHIN GALLATE**

AMORPHE DISPERSIONEN VON EPIGALLOCATECHINGALLAT

DISPERSIONS AMORPHES DE GALLATE D'ÉPIGALLOCATÉCHINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2017 US 201762535075 P**

(43) Date of publication of application:
**27.05.2020 Bulletin 2020/22**

(73) Proprietor: **Amri Ssci, LLC**
**West Lafayette, IN 47906 (US)**

(72) Inventors:
- **TENG, Jing**
**West Lafayette, IN 47906 (US)**
- **CAO, Yizheng**
**West Lafayette, IN 47906 (US)**
- **SELBO, Jon, Gordon**
**West Lafayette, IN 47906 (US)**

(74) Representative: **Potter Clarkson**
**Chapel Quarter**
**Mount Street**
**Nottingham NG1 6HQ (GB)**

(56) References cited:
**WO-A1-2011/156578 WO-A1-2015/171079**
**WO-A1-2017/061627 US-A1- 2008 199 420**
**US-A1- 2012 258 909 US-A1- 2015 265 575**

- **LEUNER C ET AL: "Improving drug solubility for oral delivery using solid dispersions", EUROPEAN JOURNAL OF PHARMACEUTICS AND BIOPHARMACEUTICS, ELSEVIER SCIENCE PUBLISHERS B.V., AMSTERDAM, NL, vol. 50, no. 1, 3 July 2000 (2000-07-03), pages 47 - 60, XP004257179, ISSN: 0939-6411, DOI: 10.1016/S0939-6411(00)00076-X**
- **NIKGHALB ET AL.: "Solid Dispersion: Methods and Polymers to increase the solubility of poorly soluble drugs", JOURNAL OF APPLIED PHARMACEUTICAL SCIENCE, vol. 2, no. 10, October 2012 (2012-10-01), pages 170 - 175, XP055225167**
- **CAO ET AL.: "Amorphous Solid Dispersion of Epigallocatechin Gallate for Enhanced Physical Stability and Controlled Release", PHARMACEUTICALS, vol. 10, 9 November 2017 (2017-11-09), pages 1 - 17, XP055569086**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
A23V 2002/00, A23V 2200/02, A23V 2200/30,
A23V 2250/2116, A23V 2250/51082;
A23V 2002/00, A23V 2200/02, A23V 2200/30,
A23V 2250/2116, A23V 2250/51086

## Description

This application claims priority benefit of U.S. Provisional Patent Application Serial No. 62/535,075 filed July 20, 2017.

## BACKGROUND OF THE INVENTION

Green tea has been one of the most commonly consumed beverages since ancient times (Cabrera, C., et al., 2006). The benefits of green tea include antiarthritic, antibacterial, antiangiogenic, and antioxidative properties (Chowdhury, A., et al., 2016). The benefits are attributed to the polyphenols, of which catechin is the major component, including epicatechin (EC), epigallo-catechin (EGC), epicatechin-3-gallate (ECG), and epigallocatechin gallate (EGCG). Catechins are widely considered a preventive agent against mammary cancer post-initiation, degenerative diseases, oxidative stress, cardiovascular and neurological disorders, and hepatotoxicity (Chowdhury, et al., 2016). Many of these beneficial health effects are credited to the most abundant catechin: epigallocatechin gallate ("EGCG") (Mandel, S., et al., 2004; Moyers, S.B., et al., 2004). Figure 1 summarizes the commonly used hierarchical nomenclature to describe "green tea extracts".

As the most abundant catechin, EGCG accounts for 60-70% of total tea catechins (Katiyar, S., et al., 1996), is often used as a quality indicator (Ananingsih, V.K., et al., 2013), and is claimed to be the most prominent catechin for health benefit purposes (Khan, N., et al., 2007). Due to its strong antioxidant and cancer chemopreventive properties, it is also one of the most extensively explored polyphenolic components of green tea (Kim, H.-S., et al., 2014; Staszewski, M.v., et al., 2011; Du, G.-J., et al., 2012; Singh, B.N., et al., 2011). EGCG is also the only polyphenol present in plasma at high concentration (77-90%) in free form (Manach, C., et al., 2005).

To date, EGCG has been demonstrated to be an anticancer agent (Chung, S., et al., 2015; Ho, Y.-C., et al., 2007; Shankar, S., et al., 2008), antioxidant, and antibacterial agent (Shutava, T.G., et al., 2009), with chemopreventive, anti-inflammatory, and anti-aging properties (Hu, C., et al., 2016). It is also reported to be protective against cardiovascular disease (Cai, Y., et al., 2013; Hong, et al., 2014; Zeng, X., et al., 2015), neurodegenerative diseases (He, M., et al., 2012; Lorenzen, N., et al., 2014), UV-induced photodamage, basal cell carcinomas, melanomas, skin papillomas (Katiyar, S.K., et al., 2000; Wang, Z.Y., et al., 1992), obesity, and diabetes (Chowdhury, et al., 2016). In addition, EGCG has been shown to interact with a number of proteins such as α-synuclein, amyloid-β, and huntingtin (Hora, M., et al., 2017). The structure of EGCG is:

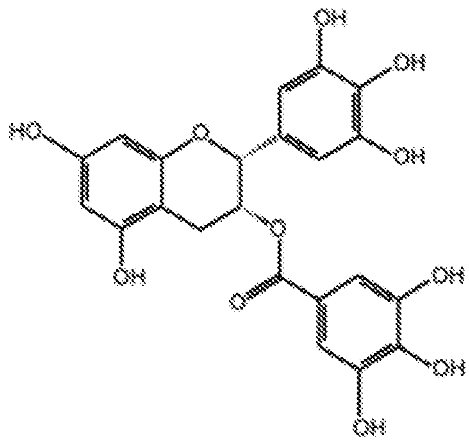

While the vast potential of EGCG in health care has been recognized, two major obstacles have hindered its utilization: high instability and low bioavailability. The instability of green tea catechins has been under study for several decades (Ananingsih, V.K., et al., 2013). EGCG has shown considerable instability and degradability in both the solid state and in solutions (Li, N., et al., 2013; Li, N., et al., 2014). A cow study found that catechins including EGCG are substantially degraded by rumen microorganisms resulting in no detectable catechin in plasma. However intraduodenal administration improved plasma concentration of all catechins with increasing dosage (Wein, S., et al., 2016). The instability of EGCG has crucially affected its processing, storage (Ananingsih, V.K., et al., 2013), and, as expected, dosing in the gastrointestinal (GI) tract. Effects of EGCG take place in a plasma level dependent manner (Mereles, D., et al., 2011) and the necessary plasma concentration, is believed to be relatively high (Smith, A.J., et al., 2013). In the meantime, the poor bioavailability of EGCG has been reported in rodents (Chen, L., et al., 1997) and humans (Chow, H.-H.S., et al., 2001), with values as low as 2~5% (Catterall, et al., 2003; Patel, A.R., et al., 2011b). Although factors responsible for the poor bioavailability of EGCG have not been fully understood, the instability/degradation of EGCG in

the GI tract and its rapid *in vivo* dissolution and elimination (Cai, Y., et al., 2002; Dvorakova, K., et al., 1999; Manach, C., et al., 2005) are believed to be important causes. The reported elimination half-life of EGCG is 3.4 h ± 0.3 h (Lee, M.-J., et al., 2002).

A commonly hypothesized approach to improve bioavailability is to decrease the dissolution rate and therefore establish an extended residence and absorption of EGCG in the GI tract (Smith, A., et al., 2010). An improvement of 30% in bioavailability has been reported with coadministration of piperine, an alkaloid from black pepper, as a result of the extended GI transit, allowing for longer residence time in the intestine (Lambert, J.D., et al., 2004). However, consumption of piperine may influence the metabolism of food and drugs, bringing possible negative effects (Smith, A., Giunta, et al., 2010). Methods such as encapsulation or making insoluble complexes (Patel, A.R., et al., 2011a; Patel, A.R., et al., 2011b) might result in a slower release of EGCG from the capsulated structure/complex which also diminishes its chemical degradation in the GI tract. However, release data were not reported for pure EGCG and it is known that the manufacturing properties of such complexes are challenging to control. Indeed, a layer-by-layer assembly to encapsulate EGCG with gelatin has been done (Shutava, T.G., et al., 2009) and a fabricated colloidal EGCG-methylcellulose complexes in aqueous suspensions resulting in a sustained release spanning two hours in both simulated intestinal and gastric fluids has also been attempted (Patel, A.R., et al., 2011b). However, the release data were not compared with pure EGCG and results after the first two hours were not provided. The oral bioavailability with a nanolipidic formulation was doubled in one report, which however involved an alcohol suspension, and therefore limited the application (Smith, A., et al., 2010). In a later work, the same group attempted to generate different forms of EGCG cocrystals to lower aqueous solubility (Smith, A.J., et al., 2013), whereas it was found that merely decreasing solubility by up to one order of magnitude was not effective in enhancing bioavailability. Patent document US 2015/0265575 discloses compositions comprising EGCG and polyvinylcaprolactanV polyvinylacetate/polyethyleneglycol graft copolymer in a weight ratio of 1:5. There remains, therefore, a need to provide stable and bioavailable EGCG.

## SUMMARY OF THE INVENTION

In one aspect of the invention, a solid dispersion comprising an approximately 1:1 weight ratio of amorphous EGCG and polyvinyl caprolactam-polyvinyl acetate-polyethylene glycol graft co-polymer is provided.

In another aspect of the invention, a pharmaceutical composition comprising a solid dispersion of amorphous EGCG is provided.

In a further aspect of the invention, a pharmaceutical composition for use in a method of sustained release delivery of EGCG is provided.

In yet another aspect of the invention, a sustained release pharmaceutical composition comprising a solid dispersion of amorphous EGCG is provided.

Disclosed, but not claimed, is a solid dispersion comprising EGCG and a micelle-forming polymer.

In another aspect of the invention, a tablet or capsule comprising solid dispersions of EGCG and a polymer according to claim 1 are provided.

In a further aspect of the invention, a foodstuff additive comprising a solid dispersion of EGCG and a polymer according to claim 1 is provided.

## BRIEF DESCRIPTION OF THE FIGURES

Figure 1 is a hierarchical terminology of green tea extracts.
Figure 2 is an x-ray powder diffraction pattern of crystalline EGCG (cEGCG) isolated from Teavigo®.
Figure 3 is a set of scanning electron micrographs of cEGCG.
Figure 4 is a thermogravimetric analysis thermogram of cEGCG.
Figure 5 is a release profile of cEGCG in pH 7.4 Phosphate-buffered saline (PBS) medium at 37°C.
Figure 6 is an x-ray powder diffraction pattern of lyopholized amorphous EGCG (aEGCG).
Figure 7 is a set of scanning electron micrographs of aEGCG.
Figure 8 is a thermogravimetic analysis thermogram of aEGCG.
Figure 9 is a modulated differential scanning calorimetry thermogram of aEGCG.
Figure 10 is an x-ray powder diffraction pattern of aEGCG which has been stressed at 11 days at 40°C/75% relative humidity.
Figure 11 is the release profile of aEGCG in pH 7.4 PBS medium at 37°C.
Figure 12 is an x-ray powder diffraction pattern of an EGCG/HPMC-AS dispersion.
Figure 13 is a set of scanning electron micrographs of an EGCG/HPMC-AS dispersion.
Figure 14 is a thermogravimetric thermogram of an EGCG/HPMC-AS dispersion.

Figure 15 is a modulated differential scanning calorimetry thermogram of an EGCG/HPMC-AS dispersion.
Figure 16 is an x-ray powder diffraction pattern of an EGCG/HPMC-AS dispersion which has been stressed at 11 days at 40°C/75% relative humidity.
Figure 17 is the release profile of an EGCG/HPMC-AS dispersion in pH 7.4 PBS medium at 37°C.
Figure 18 is an x-ray powder diffraction pattern of an EGCG/HPMC-P dispersion.
Figure 19 is a set of scanning electron micrographs of an EGCG/HPMC-P dispersion.
Figure 20 is a thermogravimetric thermogram of an EGCG/HPMC-P dispersion.
Figure 21 is a modulated differential scanning calorimetry thermogram of an EGCG/HPMC-P dispersion.
Figure 22 is an x-ray powder diffraction pattern of an EGCG/HPMC-P dispersion which has been stressed at 11 days at 40°C/75% relative humidity.
Figure 23 is a release profile of a dispersion of EGCG/HPMC-P dispersion in pH 7.4 PBS medium at 37°C.
Figure 24 is an x-ray powder diffraction pattern of an EGCG/Soluplus® dispersion.
Figure 25 is a set of scanning electron micrographs of an EGCG/Soluplus® dispersion.
Figure 26 is a thermogravimetric analysis thermogram of EGCG/Soluplus® dispersion.
Figure 27 is a modulated differential scanning calorimetry thermogram of EGCG/Soluplus® dispersion.
Figure 28 is an x-ray powder diffraction pattern of EGCG/Soluplus® dispersion which has been stressed at 11 days at 40°C/75% relative humidity.
Figure 29 is a release profile of a dispersion of EGCG/Soluplus® dispersion in pH 7.4 PBS medium at 37°C.
Figure 30 is an x-ray powder diffraction pattern of an EGCG/cellulose acetate dispersion.
Figure 31 is a set of scanning electron micrographs of an EGCG/cellulose acetate dispersion.
Figure 32 is a thermogravimetric analysis thermogram of an EGCG/cellulose acetate dispersion.
Figure 33 is a modulated differential scanning thermogram of an EGCG/cellulose acetate dispersion.
Figure 34 is an x-ray powder diffraction pattern of an EGCG/cellulose acetate dispersion which has been stressed at 11 days at 40°C/75% relative humidity.
Figure 35 is a release profile of an EGCG/cellulose acetate dispersion in pH 7.4 PBS medium at 37°C.
Figure 36 is an overlay of x-ray powder diffraction patterns of the four dispersions, aEGCG, and cEGCG.
Figure 37 is an overlay of thermogravimetric analysis thermograms of the four dispersions, aEGCG, and cEGCG.
Figure 38 is an overlay of modulated differential scanning calorimetry thermograms of the four dispersions and aEGCG.
Figure 39 is an overlay of x-ray powder diffraction patterns of the four dispersions and aEGCG after stressing at 11 days at 40°C/75% relative humidity.
Figure 40 is an overlay of release profiles of the four dispersions, aEGCG, and cEGCG at pH 7.4 PBS medium at 37°C.
Figure 41 is an x-ray powder diffraction overlay of post-dissolution solids from EGCG/Soluplus® and EGCG/cellulose acetate dispersions.
Figure 42 is a comparison of EGCG release from the dispersions, and amorphous and crystalline EGCG in the first 20 minutes in pH 7.4 PBS medium at 37°C.
Figure 43 is a kinetic release model fit for cEGCG.
Figure 44 is a kinetic release model fit for aEGCG.
Figure 45 is a kinetic release model fit for EGCG/HPMC-AS.
Figure 46 is a kinetic release model fit for EGCG/HPMC-P
Figure 47 is a kinetic release model fit for EGCG/cellulose acetate.
Figure 48 is a kinetic release model fit for EGCG/Soluplus®.
Figure 49 is a release profile of EGCG/Soluplus® fitted with a biphasic model.
Figure 50 is an XRPD pattern for a 91:9 (w/w) EGCG/Soluplus® dispersion.
Figure 51 is an XRPD pattern for an 83:17 (w/w) EGCG/Soluplus® dispersion.
Figure 52 is an XRPD pattern for a 67:33 (w/w) EGCG/Soluplus® dispersion.
Figure 53 is an XRPD pattern for a 50:50 (w/w) EGCG/Soluplus® dispersion.
Figure 54 is an XRPD pattern for a 9:91 (w/w) EGCG/Soluplus® dispersion.
Figure 55 is an XRPD pattern for a 91:9 (w/w) EGCG/HPMC-AS dispersion.
Figure 56 is an XRPD pattern for an 83:17 (w/w) EGCG/HPMC-AS dispersion.
Figure 57 is an XRPD pattern for a 67:33 (w/w) EGCG/HPMC-AS dispersion.
Figure 58 is an XRPD pattern for a 91:9 (w/w) EGCG/HPMC-P dispersion.
Figure 59 is an XRPD pattern for an 83:17 (w/w) EGCG/HPMC-P dispersion.
Figure 60 is an XRPD pattern for a 67:33 (w/w) EGCG/HPMC-P dispersion.
Figure 61 is an XRPD pattern for a 91:9 (w/w) EGCG/ cellulose acetate dispersion.
Figure 62 is an XRPD pattern for an 83:17 (w/w) EGCG/cellulose acetate dispersion.
Figure 63 is an XRPD pattern for a 67:33 (w/w) EGCG/cellulose acetate dispersion.
Figure 64 is an XRPD pattern for amorphous EGCG.

Figure 65 is an XRPD pattern for the post-stressing solids of a 91:9 (w/w) EGCG/Soluplus® dispersion at about 40°C/75% RH/30 days.

Figure 66 is an XRPD pattern for the post-stressing solids of an 83:17 (w/w) EGCG/Soluplus® dispersion at about 40°C/75% RH/30 days.

Figure 67 is an XRPD pattern for the post-stressing solids of a 67:33 (w/w) EGCG/Soluplus® dispersion at about 40°C/75% RH/30 days.

Figure 68 is an XRPD pattern for the post-stressing solids of a 50:50 (w/w) EGCG/Soluplus® dispersion at about 40°C/75% RH/30 days.

Figure 69 is an XRPD pattern for the post-stressing solids of a 9:91 (w/w) EGCG/Soluplus® dispersion at about 40°C/75% RH/30 days.

Figure 70 is an XRPD pattern for the post-stressing solids of a 91:9 (w/w) EGCG/HPMC-AS dispersion at about 40°C/75% RH/30 days.

Figure 71 is an XRPD pattern for the post-stressing solids of an 83:17 (w/w) EGCG/HPMC-AS dispersion at about 40°C/75% RH/30 days.

Figure 72 is an XRPD pattern for the post-stressing solids of a 67:33 (w/w) EGCG/HPMC-AS dispersion at about 40°C/75% RH/30 days.

Figure 73 is an XRPD pattern for the post-stressing solids of a 91:9 (w/w) EGCG/HPMC-P dispersion at about 40°C/75% RH/30 days.

Figure 74 is an XRPD pattern for the post-stressing solids of an 83:17 (w/w) EGCG/HPMC-P dispersion at about 40°C/75% RH/30 days.

Figure 75 is an XRPD pattern for the post-stressing solids of a 67:33 (w/w) EGCG/HPMC-P dispersion at about 40°C/75% RH/30 days.

Figure 76 is an XRPD pattern for the post-stressing solids of a 91:9 (w/w) EGCG/ cellulose acetate dispersion at about 40°C/75% RH/30 days.

Figure 77 is an XRPD pattern for the post-stressing solids of an 83:17 (w/w) EGCG/cellulose acetate dispersion at about 40°C/75% RH/30 days.

Figure 78 is an XRPD pattern for the post-stressing solids of a 67:33 (w/w) EGCG/cellulose acetate dispersion at about 40°C/75% RH/30 days.

Figure 79 is an XRPD pattern for the post-stressing solids of amorphous EGCG at about 40°C/75% RH/30 days.

## DETAILED DESCRIPTION OF THE INVENTION

Amorphous materials have been used in the pharmaceutical industry due to their high solubility. However pure amorphous compounds, such as active pharmaceutical ingredients ("APIs"), are rarely marketed as such because of physical instability, i.e., the tendency towards crystallization during storage and processing. Amorphous solid dispersions kinetically stabilize amorphous APIs via the presence of excipients, typically polymers, to help prevent crystallization and maintain the supersaturation (Brouwers, J., Brewster, et al., 2009). Owing to the substantial length and flexibility of the polymer chains in a solid dispersion, an API therein is separated into interstitial solid solutions with the molecules in the interstices. Via this confinement and immobilization, an amorphous API may be significantly stabilized compared to its neat form in the appropriate polymer. As further disclosed herein, Applicants have prepared solid dispersions of amorphous EGCG (where EGCG is the API) and various polymers as vehicles for delivery of EGCG in a pharmaceutical setting. In particular, the amorphous solid dispersions reported herein address the instability and low bioavailability issues which have previously limited the use and development of EGCG.

While the use of solid dispersions to improve the solubility of poorly-soluble drugs is known in the literature, solid dispersions have not, therefore typically been viewed as a vehicle for delivering highly soluble APIs. In this instance, the Applicants have utilized a solid dispersion to provide sustained release formulation with an aim towards enhancing the bioavailability of a water-soluble, but low bioavailability compound, while at the same time addressing the instability challenges plaguing EGCG.

Amorphous dispersions may be made by several methods. These include spray drying, lyophilization, melt extrusion, and co-precipitation. For the solid dispersions prepared in the Examples, lyophilization was used to generate the EGCG amorphous solid dispersions.

The polymers used herein are those that are capable of forming a dispersion with EGCG. While some polymers may not form a dispersion with lyophilziation, such polymers may form a dispersion under different methods or under different lyophilziation conditions than those herein described. Examples of suitable EGCG: polymer weight ratios include about 10:90 to about 90:10 including all ranges in between such as about 10:90 to about 20:80, about 20:80 to about 30:70, about 30:70 to about 40:60, about 40:60 to about 50:50, about 50:50 to about 60:40, about 60:40 to about 70:30, about 70:30 to about 80:20, and about 80:20 to about 90:10.

Examples of polymers which form solid dispersions with EGCG include those that contain a cellulose functionality

such as with HPMC-AS, HPMC-P, and cellulose acetate. Other polymer systems that form dispersions with EGCG include those that contain caprolactam functionalities such as polyvinyl caprolactam-polyvinyl acetate-polyethylene glycol graft co-polymer, one embodiment of which is Soluplus®.

The structure of Soluplus® is

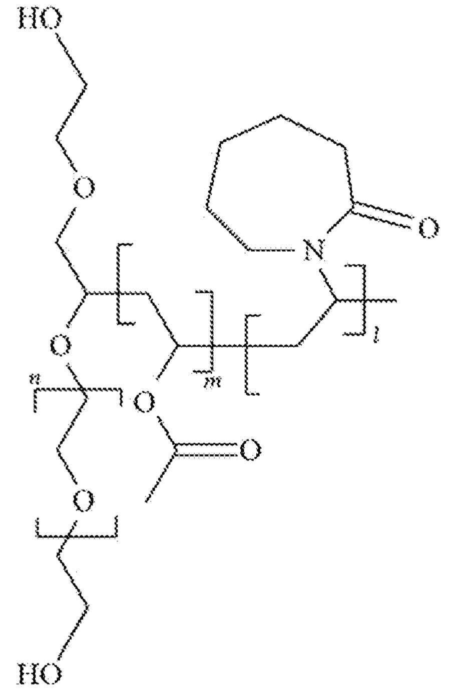

Where *l*, *m,* and *n* are values such that the average molecular weight, as determined by gel permeation chromatography is in the range of 90,000 - 140,000 g/mol.

The solid dispersions of EGCG and one or more polymers contain varying amounts of polymer. These weights range from about 9% to about 91% EGCG with the balance being polymer. All weights and weight ranges are reported as inputs prior to forming a solid dispersion.

Disclosed, but not claimed, are solid dispersions comprising amorphous EGCG and HPMC-AS.

In many of these embodiments, the solid dispersion has a glass transition temperature of less than about 80°C including at about 72°C. In these and other embodiments, the solid dispersions exhibit an x-ray amorphous powder diffraction pattern, which remains after eleven days under accelerated stress conditions of 40°C and 75% relative humidity. The term "x-ray amorphous" means that a powder diffraction pattern reveals one or more amorphous halos and does not contain sharp crystalline peaks. In some embodiments, which are not according to the claims, the mass of EGCG and the mass of HPMC-AS is approximately the same in the solid dispersion (~1:1 or equal to 1: 1). In other embodiments, which are not according to the claims, the weight of EGCG to HPMC-AS ranges from about 50:50 to about 91:9.

Disclosed, but not claimed, are solid dispersions of amorphous EGCG and HMPC-P.

In many of these embodiments, the solid dispersions exhibit an x-ray amorphous powder diffraction pattern and an x-ray amorphous diffraction pattern is seen after eleven days under accelerated stress conditions of 40°C and 75% relative humidity. In some embodiments, which are not according to the claims, the mass of EGCG and the mass of HPMC-P is approximately the same in the solid dispersion (~1:1 or equal to 1:1). In other embodiments, which are not according to the claims, the weight of EGCG to HPMC-P ranges from about 50:50 to about 91:9.

Disclosed, but not claimed, are solid dispersions of EGCG and cellulose acetate. In many of these embodiments, the solid dispersions exhibit an x-ray amorphous powder diffraction pattern and an x-ray amorphous diffraction pattern is seen after eleven days under accelerated stress conditions of 40°C and 75% relative humidity. In some embodiments, which are not according to the claims, the mass of EGCG and the mass of cellulose acetate is approximately the same in the solid dispersion (~1:1 or equal to 1:1). In other embodiments, which are not according to the claims, the weight of EGCG to cellulose acetate ranges from about 50:50 to about 91:9.

In many embodiments of the invention, solid dispersions comprising amorphous EGCG and polyvinyl caprolactam-polyvinyl acetate-polyethylene glycol graft co-polymer is provided. In many of these embodiments, the solid dispersions have a glass transition temperature of less than about 150°C including at about 143°C. In these and other embodiments, the solid dispersion exhibits an x-ray amorphous powder diffraction pattern and an x-ray amorphous diffraction pattern is seen after eleven days under accelerated stress conditions of 40°C and 75% relative humidity. One

tradename of such a polymer is Soluplus®. In these and other embodiments, the amount of EGCG released within 20 minutes in a pH 7.4 medium containing PBS is less than 60% and in some embodiments is about 50%. The amount of EGCG released in many of the solid dispersions of the invention is less than that found in corresponding crystalline EGCG. In some embodiments, the mass of EGCG and the mass of polyvinyl caprolactam-polyvinyl acetate-polyethylene glycol graft co-polymer is approximately the same in the solid dispersion (~ 1:1 or equal to 1:1). In many embodiments, which are not according to the claims, the ratio of polyvinyl caprolactam-polyvinyl acetate-polyethylene glycol graft co-polymer to EGCG is between about 10:1 to about 1:10 including between about 91:1 to about 9:91. In certain of the embodiments, polyvinyl caprolactam-polyvinyl acetate-polyethylene glycol graft co-polymer is Soluplus®.

It was previously reported that EGCG tends to bind with polyvinyl pyrrolidone and polyvinyl polypyrrolidone, resulting in colloids and aggregates (Patel et al., 2011). These colloids are typically undesirable, but may find applications of special formulations in food or drugs, whereas they could also raise difficulties in formulation and reduce efficacy of polyphenols (Patel et al., 2011). This may explain why precipitates were observed upon mixing EGCG solutions with polymer solutions of PVP, PVA, PEG, and PVAC. Furthermore, such binding may occur, though likely weaker, between EGCG and the other five polymers of HPMC-AS, HPMC-P, Soluplus®, cellulose acetate, and Gelucire® 50/13 listed in Table 5 since during mixing of solutions, samples can be cloudy but clear again upon addition of more solvent and vortexing. One intrinsic difference between amorphous dispersions and colloidal complexes is the molecular-level mixing of the drug and the polymer. With colloidal complexes, cloudy solutions with precipitates form. To reduce the chance of colloid formation, a slow and stepwise addition approach was employed for the dispersions, along with adding additional fresh solvents and vortexing for the purpose of acquiring clear solutions.

In many embodiments, clear solutions containing EGCG and polymer were lyophilized to yield solids. The lyophilized materials were often fluffy and lightweight. For example, the Soluplus® 50:50 dispersion in one embodiment showed a white color while all the other dispersions and the EGCG displayed some yellowness. Solids were then characterized by XRPD, and the results are included in Table 5. Among them, Gelucire® 50/13 as prepared was not a suitable candidate to generate amorphous dispersion since the lyophilized solids displayed a disordered x-ray diffraction pattern with characteristic peaks consistent with Gelucire® 50/13. As a result, four polymers, i.e., HPMC-AS, HPMC-P, Soluplus®, and cellulose acetate, were selected as candidate excipients to generate amorphous dispersions with EGCG for further study.

EGCG amorphous solid dispersions generated by lyophilization were characterized by XRPD and the data compared to the lyophilized EGCG and the starting material of EGCG, as shown in Figure 36. Based on the results, the four dispersions and the lyophilized EGCG show clear x-ray amorphous patterns while the EGCG isolated from Teavigo™ is a crystalline material. The individual x-ray powder diffractions of cEGCG, aEGCG, EGCG/HPMC-AS, EGCG/HPMC-P, EGCG/Soluplus®, and EGCG/cellulose acetate can be found at Figures 2, 6, 12, 18, 24, and 30 respectively.

SEM images of cEGCG, aEGCG, and the four dispersions are provided in Figures 3, 7, 13, 19, 25, and 31 respectively. The cEGCG and aEGCG SEM images exhibit disparate morphologies. The cEGCG micrograph contains long thin flat laths while the aEGCG micrograph has continuous structure consisting of round-ended fibers and small spheres with a broad distribution of particle sizes down to tens of nm. The four dispersions all show continuous structures with different morphologies as well. For the one with HPMC-AS (Figure 13), EGCG particles appear intimately interconnected with the polymer forming a porous layered network. The dispersions with HPMC-P (Figure 19) and cellulose acetate (Figure 31) exhibit relatively separated phases of EGCG and polymer. The Soluplus® dispersion shows a higher level of homogeneity and with no apparent separation between EGCG and polymer (Figure 25).

Figure 37 provides the TGA data of the four vacuum-dried disperions and both aEGCG and cEGCG. Certain amounts of volatiles are observed in all of samples. For EGCG, the water is the volatile while for the dispersions, volatiles could be water, dioxane or a mixture of both. The amount of volatiles are estimated and accounted for when calculating the EGCG equivalency for dissolution testing. Starting from approximately 220°C, all materials show apparent decomposition. Individual TGA curves are shown at Figure 4, 8, 14, 20, 26, and 32 for cEGCG, aEGCG, EGCG/HPMC-AS, EGCG/HMPC-P, EGCG/Soluplus®, and EGCG/cellulose acetate respectively.

Figure 38 shows the mDSC data for aEGCG and the four dispersions. Glass transition temperatures are observed at about 163°C, 72°C, and 143°C for aEGCG, the dispersions of EGCG/HPMC-AS and EGCG/Soluplus® respectively (see Figures 9, 15, and 27 respectively). No clear glass transition is observed for the EGCG/HPMC-P dispersion (see Figure 21). For the EGCG/cellulose acetate dispersion, the glass transition spanned a large range of 59-119°C (see Figure 33). The presence of one (miscible) or two (phase separation) glass transition is often used to evaluate drug-polymer miscibility (Baird and Taylor, 2012). For example, the EGCG/HPMC-AS and EGCG/Soluplus® dispersions display only one apparent $T_g$, suggesting that EGCG and the polymers are likely miscible in the materials.

The physical stability of aEGCG and the four dispersions were evaluated at different conditions including a typical stress condition of 40°C/75%RH for 11 days, and in media of SGF and SIF for 24 h. Table 1 summarizes the visual observations on the materials after they have been stressed at 40°C/75%RH for 11 days. The aEGCG became a hard material after stress, while all the dispersions were particles or soft aggregates.

**Table 1. Observation of the materials after 40 °C/75% RH stress for 11 days** (only the sample EGCG/Soluplus® is according to the claims)

| Material | Visual observation after stress |
|---|---|
| aEGCG | pink hard material, agg. |
| EGCG/HPMC-AS | pink agg. and particles |
| EGCG/HPMC-P | pink agg. and particles |
| EGCG/Soluplus® | white free flowing agg. and particles |
| EGCG/cellulose acetate | dark pink agg. and particles |

The post-stress samples were characterized by XRPD and the results are presented in Figure 39. The aEGCG turned into a highly crystalline material while the four dispersions remained x-ray amorphous, suggesting the dispersions are physically stable at this stress condition. In addition, the Soluplus® dispersion retained its white color after stress while all the others presented different levels of color changes. It has been well established that the color change of EGCG directly correlated with its chemical instability, i.e., degradation and oxidation (Li et al., 2013; Sang et al., 2005). This difference in appearance after stress likely indicates that the Soluplus® dispersion has a higher chemical stability than other dispersions and aEGCG. This improved physicochemical stability may have significant impacts on the whole cycle of storage, transportation, and processing of EGCG. Individual diffraction patterns for such stressed conditions can be found at Figures 10, 16, 22, 28, and 34 for aEGCG, EGCG/HPMC-AS, EGCG/HPMC-P, EGCG/Soluplus®, and EGCG/cellulose acetate respectively.

For the four polymers identified, further stress tests were carried out on dispersions with varying weight ratios of EGCG to polymer as set forth in Example 8. In these experiments, stressing was done for 30 days at 75% relative humidity at 40°C for 30 days. A total of 14 dispersions were made in $H_2O$ and dioxane as shown in Table 9. Figures 50-63 are the XRPD patterns of the prepared dispersions prior to stressing. Figures 75-78 are the XRPD patterns after stressing and these too show the samples to amorphous by XRPD. Figure 82, 83, and 85 show a peak indicative of NaCl, which is used in the salt both of the humidity chamber storing the samples. Thus, such a peak is not indicative of any crystalline EGCG in the corresponding dispersions. As a control, an EGCG was prepared (Figure 64) and similarly stressed and it showed conversion to EGCG as seen in Figure 79. Based on the XRPD results, the post-stressing dispersions were x-ray amorphous (other than the NaCl peaks).

Table 10 shows color changes after stressing. A color change may be indicative of chemical degradation to some degree. With regards to the tested dispersions, all the dispersions prepared had a very light pinkish/ off white hue. After stressing, the 50:50 EGCG:Soluplus® dispersion remained unchanged and the 9:91 EGCG:Soluplus® dispersion had a slight change to light yellow. The other Soluplus® dispersions changed to a dark red whereas the dispersions with other polymers changed to a very dark red which was almost black in hue. The color change suggested that the Soluplus® dispersions had the least degradation of the dispersions tested. The non-dispersed amorphous EGCG converted to crystalline EGCG.

For stress testing in media, solids of aEGCG and the dispersions were suspended in SGFs and SIFs (see Example 6), and the suspensions were observed under polarized light microscope from time to time to look for the evidence of birefringence and extinction (B/E) of the materials, which is the indication of the occurrence of crystallization in a material. The observations are summarized in Table 2. Amorphous EGCG crystallized quickly, within 15 minutes in both SGF and SIF, while no occurrence of crystallization was noticed for the four dispersions in both media for at least 24 h, suggesting improved physical stability in the GI environment of the dispersions compared to aEGCG.

**Table 2. Observations under PLM for the dispersions and aEGCG in SGF and SIF media** (only the sample EGCG/Soluplus® is according to the claims)

| Material | in SGF | in SIF |
|---|---|---|
| EGCG | B/E observed in 15 min | |
| EGCG/HPMC-AS | no B/E observed in 24 h | |
| EGCG/HPMC-P | | |
| EGCG/Soluplus® | | |
| EGCG/cellulose acetate | | |

The dissolution tests for the EGCG and dispersions were carried out in Example 7 to determine the release profiles of EGCG in a GI tract model. During tests, cEGCG, aEGCG, EGCG dispersions prepared with HPMC-AS or HPMC-P were all dissolved without visible solids, while residual solids were present for dispersions of EGCG/Soluplus® and EGCG/cellulose acetate after 24 hours. The solids were isolated, dried under $N_2$ purge and then analyzed by XRPD. The XRPD patterns of the post-dissolution solids of EGCG/Soluplus® and EGCG/cellulose acetate dispersions, along with the pattern of NaCl are shown in Figure 41. The peaks which appeared in the XRPD patterns of both solids are consistent with NaCl which came from the dissolution medium. No evidence of crystalline peaks consistent with crystalline EGCG is observed. In fact, based on the dissolution data, for both samples all EGCG (~20 mg) was released into the medium within the period of dissolution tests. Therefore, the solids isolated after dissolution are un-dissolved polymers, Soluplus® or cellulose acetate, respectively.

The release profiles for each material are presented in Figure 40 and the data in the first 20 minutes are presented in the inset. During this 20 minutes, the releases of EGCG were approximately 93% for cEGCG, 100% for aEGCG, 82% for EGCG/HPMC-AS, 90% for EGCG/HPMC-P, 50% for EGCG/Soluplus®, and 85% for EGCG/cellulose acetate, respectively, as shown in Figure 42. The individual release profiles are found at Figures 5, 11, 17, 23, 29, and 35 for cEGCG, aEGCG, EGCG/HPMC-AS, EGCG/HPMC-P, EGCG/Soluplus®, and EGCG/cellulose acetate respectively.

As expected, aEGCG exhibits an immediate dissolution, at a much higher rate than all the other materials. The three dispersions with HPMC-AS, HPMC-P, and cellulose acetates show slower release compared to cEGCG. For dispersions with HPMC-AS and HPMC-P, since the polymers are quickly dissolved in the medium, they are no longer able to prevent EGCG molecules from releasing. Under this situation, the interactions between polymers and EGCG do not significantly prolong the release of EGCG in the intestine (high pH). Based on the release profiles for the first 20 minutes (Figure 40), the dispersions of HPMC-P and cellulose acetate have the faster dissolutions than the other two dispersions. For the Soluplus® dispersion, a distinct release profile is observed compared to the other materials. Only half of the EGCG is released in the first 20 minutes and the dissolution continues up to approximately 24 hours.

Without being bound by theory, it is believed that the micelle-forming ability of polyvinyl caprolactam-polyvinyl acetate-polyethylene glycol graft co-polymer or a Soluplus® based polymer enhances the performance of the resulting solid dispersion which provides for the sustained release of EGCG. In support of this theory, the release kinetics of the EGCG/Soluplus® dispersion was modeled and compared with the other three dispersions as well as aEGCG and cEGCG.

As an initial proposition, the dispersions, aEGCG and cEGCG were modeled with respect to pseudo-second order models according to equation 1 where t is time, Q is cumulative release, and $Q_e$ the final release and, and $k_2$ the rate constant of pseudo-second order kinetics.

$$\frac{t}{Q} = \frac{1}{k_2 Q_e^2} + \frac{t}{Q_e} \qquad \text{Eq. (1)}$$

Pseudo-second order kinetics is often used to model the release and/or adsorption of materials in aqueous media. In this case, all of the dispersions and aEGCG and cEGCG fit this model as evidenced by very high correlation coefficients (Figures 43-47). The outlier was EGCG/Soluplus® which fit the model poorly with a correlation coefficient of only 0.9613 showing substantial nonlinear behavior with this model (Figure 48).

Soluplus®, as a polyvinyl caprolactam-polyvinyl acetate-polyethylene glycol graft copolymer, has an amphiphilic structure with a high lipophilicity, and possesses a very low critical micelle concentration (7.6 mg/L) (Reintjes, 2011). Soluplus® has a bifunctional character, which can be used as a matrix polymer for solid solutions and also an active solubilizer through micelle formation in water (Shamma and Basha, 2013). A number of reports have demonstrated the use of self-assembled polymer micelles based on amphiphilic block copolymers as vehicles to improve delivery and bioavailability (Bontha et al., 2006; Huynh et al., 2009; Kim et al., 2009; Liu et al., 2006; Siddiqui et al., 2009).

During the dispersion generation with EGCG, Soluplus® reached a concentration of 7353 mg/L in water-dioxane solution prior to lyophilization. This concentration is much higher than its critical micelle concentration, and therefore Soluplus® micelles likely formed in the solution with EGCG molecules inside the micelles. Micelle formation would result in a tight wrapping of the EGCG molecules and/or the high miscibility between Soluplus® and EGCG, which may explain why they yield lyophilized solids with white color while the other dispersions show yellowness originating from the color of EGCG (see Table 1). In the dissolution test, it is hypothesized that the tightly wrapped EGCG is released after the dissolution or at least partial dissolution of Soluplus®, which could form micelles again in the dissolution medium and further delay the release of EGCG.

With the strong tendency to form micelles, the interaction between EGCG and water is mitigated. Thus, while the hydroxyls of EGCG render an adequate binding with the hydrophilic chains in Soluplus®, the high lipophilicity of Soluplus® lowers the wettability of EGCG. Indeed, these features are consistent with a bi-phasic release of EGCG in a Soluplus® dispersion (see Figure 49). In the first portion of the release, EGCG molecules that are not fully enveloped

by the dispersive protection of the micelles of EGCG are released in the same manner as the other dispersions whereas EGCG that is within the micelles are released at a slower rate due to the water-inhibition properties of the micelles.

This biphasic model that combines pseudo-second-order and first-order kinetics is used to describe the release profile of the EGCG/Soluplus® dispersion as set forth in equation 2:

$$Q_t = \frac{t}{1/k_2 Q_e^2 + 1/Q_e} + Q_0(1 - \exp(-k_1 t)) \qquad \text{EQ. (2)}$$

The first order portion of equation (2) is also a special case of the Weibull model:

$$Q = Q_0\left(1 - \exp\left(-\frac{(t-T)^b}{a}\right)\right) \qquad \text{EQ. (3)}$$

Other polymers which exhibit micelle forming properties include amphiphilic polymers such as poly(ethylene oxide)- poly (propylene oxide) - poly(ethylene oxide) polymers. Other micelle-forming polymers include those set forth in Table 3.

**Table 3 Micelle-Forming Polymers** (not claimed)

| | | | | |
|---|---|---|---|---|
| **PEO-PBLA**<br>polyethylene oxide)-poly (J3-benzyl-L-aspartate) | **PEO-P(Lys)**<br>polyethylene oxide)-poly(L-lysine) | **PEO-P(Asp)**<br>polyethylene oxide)-poly (aspartic acid) | **PEO-PE**<br>polyethylene oxide)-phosphatidyl ethanolamine | **PEO-PDLLA**<br>polyethylene oxide)-poly(DL-lactic acid) |
| **PNIPA-PBMA**<br>poly(N-isopropylacrylamide)-poly (butylmethacrylate) | **PAA-PMMA**<br>poly(acrylic acid)-poly (methyl methacrylate) | **PEO-PPO-PEO**<br>polyethylene oxide)-poly (propylene oxide)-poly (ethylene oxide) | **PEO-PCL**<br>polyethylene oxide)-poly(E-caprolactone) | **PEO-($C_{16}$,BLA)**<br>polyethylene oxide)-$C_{16}$, (J3-benzyl-L-aspartate) |
| **PEO-P(Asp,BLA)**<br>polyethylene oxide)-poly (aspartic acid),(J3-benzyl-L-aspartate) | **LCC**<br>N-laurel-carboxymethyl-chitosan | | | |

Suitable excipients for use in the composition of the present invention include, without limitation, any excipient which is relatively non-toxic and innocuous to a patient at concentrations consistent with effective activity of the solid dispersion of the invention so that any side effects ascribable to the excipients do not vitiate the beneficial effects of the solid dispersion. Such excipients include, without limitation, solvents, diluents, or other liquid vehicles, dispersion or suspension aids, surface active agents, isotonic agents, thickening or emulsifying agents, preservatives, solid binders, lubricants, adjuvants, vehicles, delivery systems, disintegrants, absorbents, preservatives, surfactants, colorants, flavorants, or sweeteners and the like, as suited to the particular dosage form desired.

The amount of solid dispersion that may be included in a composition of the present invention is that amount which produces a result or exerts an influence on the particular individual receiving the solid dispersion. Solid dispersions of the present invention can be administered with, e.g., pharmaceutically-acceptable excipients well known in the art using any effective conventional dosage unit forms, including immediate, slow and timed release preparations, orally, parenterally, topically, nasally, ophthalmically, optically, sublingually, rectally, vaginally, and the like.

The composition comprising the solid dispersion may be used alone or in combination with other compositions to improve animal or human health or nutrition. Likewise, the solid dispersion itself may be combined with other compositions to improve animal health or nutrition.

The dispersions according to the present invention are suitable as dietary supplements, or as components in dietary supplements.

In some embodiments, the solid dispersion thereof is incorporated into a capsule.

In other embodiments, the solid dispersion thereof is incorporated into a tablet.

In yet other embodiments, the solid dispersion is added to a foodstuff. The solid dispersion may be added to

any foodstuff, including, without limitation coffee, tea, soda, fruit drink, water, sauce, candy, cereal, bread, fruit mixes, fruit salads, salads, snack bars, fruit leather, health bars, granola, smoothies, soups, juices, cakes, pies, shakes, ice cream, health drinks.

EGCG can be used for prevention or therapy of various diseases or conditions, based on its antioxidant effects. It is useful for treatment or prevention of diseases including, but not limited to neurodegenerative diseases or conditions, such as Alzheimer's disease; upper respiratory diseases, including those caused by an infection; dementia, such as AIDS-dementia; oncological disorders, such as cancer; inflammatory or auto-immune diseases, such as rheumatoid arthritis or diabetic neuropathies; or a disease or condition caused by an infection by virus or bacteria.

The solid dispersions of the present invention may be administered in a single unit dosage form that contains an amount of solid dispersion effective to treat a subject. The solid dispersions can also include suitable excipients or stabilizers, and can be in solid or liquid form such as, tablets, capsules, powders, solutions, suspensions, or emulsions. Typically, the composition comprising the solid dispersion will contain from about 0.01 to 99% w/w, or from about 5 to 95% w/w solid dispersion.

The solid dispersion, when combined with any suitable excipients or stabilizers, and whether administered alone or in the form of a composition, can be administered orally. For most therapeutic purposes, the solid dispersion can be administered orally as a solid or as a solution or suspension in liquid form.

The solid unit dosage forms of the solid dispersion can be of a conventional type. The solid form can be a capsule, such as an ordinary gelatin type containing the solid dispersion and a carrier, for example, lubricants and inert fillers such as, lactose, sucrose, or cornstarch. In another embodiment, the solid dispersion is tableted with conventional tablet bases such as lactose, sucrose, or cornstarch in combination with binders like acacia or gelatin, disintegrating agents such as cornstarch, potato starch, or alginic acid, and a lubricant such as stearic acid or magnesium stearate.

The solid dispersion may be administered in combination with other therapeutic regimens that are known in the art, whether now known or hereafter developed.

## EXAMPLES

### Example 1

**Table 4. Raw materials and vendors**

| Material | Vendor |
|---|---|
| Teavigo™ (150 mg/capsule, EGCG ≥ 94%) | Healthy Origins |
| NF Grade hydroxypropyl methylcellulose acetate succinate (HPMC-AS, MG) | Shin-Etsu Chemical |
| hydroxypropyl methylcellulose phthalate (HPMC-P, HP-55) | Shin-Etsu Chemical |
| Soluplus® | BASF |
| cellulose acetate, average Mn ~30000 by GPC | Sigma-Aldrich |
| polyvinylpyrrolidone (PVP K-90), average Mw 360000 | Sigma-Aldrich |
| polyvinyl alcohol (PVA) | Sigma-Aldrich |
| polyvinyl acetate (PVAc) | Sigma-Aldrich |
| polyethylene glycol (PEG), average Mn 10000 | Sigma-Aldrich |
| Gelucire® 50/13 | Gattefosse |
| dioxane | Sigma-Aldrich |
| water (HPLC grade) | Sigma-Aldrich |
| simulated gastric fluid (SGF) | RICCA Chemical Company |
| simulated intestinal fluid (SIF) | RICCA Chemical Company |

All chemicals were obtained from commercial sources and used without further purification unless otherwise indicated.

XRPD patterns were collected with a PANalytical X'Pert PRO MPD diffractometer using an incident beam of

Cu radiation produced using an Optix long, fine-focus source. An elliptically graded multilayer mirror was used to focus Cu Kα X-rays through the specimen and onto the detector. Prior to the analysis, a silicon specimen (NIST SRM 640e) was analyzed to verify the observed position of the Si 111 peak is consistent with the NIST-certified position. A specimen of the sample was sandwiched between 3-μm-thick films and analyzed in transmission geometry. A beam-stop, short antiscatter extension, and antiscatter knife edge were used to minimize the background generated by air. Soller slits for the incident and diffracted beams were used to minimize broadening from axial divergence. Diffraction patterns were collected using a scanning position-sensitive detector (X'Celerator) located 240 mm from the specimen and Data Collector software v. 2.2b. The data acquisition parameters for each pattern are displayed above the image in the Data section of this report including the divergence slit (DS) before the mirror and the incident-beam antiscatter slit (SS).

Polarized light microscopy (PLM) was performed using a Leica DM LP microscope with cross polarizer. SEM was performed using a FEI Quanta 200 scanning electron microscope equipped with an Everhart Thornley (ET) detector. Images were collected and analyzed using xTm (v. 2.01) and XT Docu (v. 3.2) software, respectively. The thermogravimetric analyses (TGA) were performed using a TA Instruments Q5000 IR thermogravimetric analyzer. Each sample was placed in an aluminum sample pan and inserted into the TGA furnace. The furnace was first equilibrated at 25°C, and then heated under $N_2$ at a rate of 10°C/min, up to a final temperature of 350°C. Modulated differential scanning calorimetry (mDSC) data were obtained on a TA Instruments Q2920 differential scanning calorimeter equipped with a refrigerated cooling system (RCS). The test was progressed by modulating temperature ±0.8°C every 60 seconds from 0 to 180°C. The reported glass transition temperature ($T_g$) is obtained from the inflection point of the step change in the reversing heat flow versus temperature curve.

**Example** 2 - **Preparation of raw** EGCG

36 capsules of Teavigo® were broken and the solids were suspended in ~260 mL HPLC grade water and filtered to remove the solids. The resultant solution was clear with no evidence of solid particles observed. The solution was then dried under $N_2$ purge until water was fully removed based on visual observation. Such generated EGCG solids were directly used for generating amorphous EGCG and solid dispersions.

**Example 3 - Screening for Dispersions**

Nine polymers were investigated for performance in making solid dispersions with EGCG. For each polymer, individual solutions of polymer (200 mg in 3~10 mL dioxane depending on solubility) and EGCG (200 mg in 11.5 mL water) were prepared and then slowly mixed together with the procedures described generally in Example 4. The initial results are provided in Table 5. Upon mixing, precipitates were observed in the samples containing EGCG along with PVP, PVA, PEG and PVAc, and therefore these samples did not move forward with dispersion generation. Slight amount of precipitates were also noticed in the samples containing EGCG and other five polymers of HPMC-AS, HPMC-P, Soluplus®, cellulose acetate, and Gelucire® 50/13. These samples were clear again upon addition of more solvent and vortexing.

**Table 5. Initial screen results of nine polymers with EGCG** (only the sample EGCG/ Soluplus® is according to the claims)

| Polymer | Visual observation upon mixing | Visual observation after lyophilization | XRPD results |
|---|---|---|---|
| HPMC-AS | clear solution | light yellow fluffy particles | x-ray amorphous |
| HPMC-P | clear solution | yellow fluffy particles | x-ray amorphous |
| Soluplus® | clear solution | white fluffy particles | x-ray amorphous |
| Cellulose acetate | clear solution | light yellow fluffy particles | x-ray amorphous |
| Gelucire® 50/13 | clear solution | light yellow fluffy particles | disordered |
| PVA | flocculated suspension | - | - |
| PVP | flocculated suspension | - | - |
| PEG | flocculated suspension | - | - |
| PVAc | flocculated suspension | - | - |

These clear solutions containing EGCG and polymer were lyophilized to yield solids.

**Example 4 - Preparation of Dispersions**

EGCG solid dispersions were prepared by lyophilization using four polymers including HPMC-AS, HPMC-P, Soluplus®, and cellulose acetate. Individual solutions of polymer (in dioxane) and EGCG (in water) were prepared. The EGCG aqueous solution was added into the polymer-dioxane solution slowly and stepwisely, with each step about 1~2 mL of aqueous EGCG solution were added. During this step, additional fresh dioxane or water may be added into the samples followed by vigorous vortexing to reach a clear solution without visible solids or colloids. The approximate ratios of water to dioxane for the final solution of the dispersions are provided in Table 6 (per gram of EGCG).

**Table 6. Final mixtures proportions of the four dispersions** (only the sample EGCG/ Soluplus® is according to the claims)

| | EGCG (g) | Polymer (g) | $H_2O$ (mL) | Dioxane (mL) |
|---|---|---|---|---|
| HPMC-AS | 1 | 1 | 50 | 80 |
| HPMC-P | 1 | 1 | 58 | 100 |
| Soluplus® | 1 | 1 | 58 | 78 |
| Cellulose acetate | 1 | 1 | 58 | 140 |

For lyophilization, each EGCG/polymer final solution was frozen into a thin layer on the sides of the vial by rotating the sample vial in a cold bath of dry ice/acetone (at a temperature of -78°C). The sample vial was then attached to a Flexi-Dry manifold lyophilizer (SP Industries, Stone Ridge, NY) at -50°C for 3 days. Pure EGCG aqueous solution was lyophilized as well to generate amorphous EGCG (aEGCG) as a reference material for comparison with the dispersions. After lyophilization was done, samples were secondary dried under vacuum at 40°C/5 days for dispersions, and under vacuum at RT/8 days for aEGCG, to remove the residual dioxane and water from samples. The lyophilized materials are fluffy and lightweight; the Soluplus® dispersion shows a white color while all the others (including the pure EGCG) display some yellowness. All dispersions were prepared at 50:50 (w/w, EGCG/polymer) composition.

**Example 5 - Stress Tests at about 40°C/75% RH stress**

Solids of dispersion or amorphous EGCG (approximately 15~30 mg each) were exposed to about 40°C/75% RH by placing solids into an uncapped clear glass vial inside a sealed container in a 40°C oven. 75% RH were maintained by saturated salt solution of NaCL (ASTM Standard E, 104-85, 1996). Samples were stressed for 11 days, and then characterized by XRPD for evidence of crystallization.

**Example 6 - Stress Tests in the GI Tract (Simulated)**

Solids were suspended in media including simulated gastric fluid (SGF) and simulated intestinal fluid (SIF), and observed under PLM from time to time up to 24 hours for evidence of crystallization indicative by the appearance of birefringence/extinctions. The weights of the solids and the volumes of the media are listed in the following table. Because of the high solubility of aEGCG, more materials were suspended into the media compared with the dispersions.

**Table 7. The amounts of the solids and media used for the stress testings** (only the sample EGCG/Soluplus® is according to the claims)

| | In SGF | | In SIF | |
|---|---|---|---|---|
| | Weight of solids (mg) | SGF volume (μL) | Weight of solids (mg) | SIF volume (μL) |
| aEGCG | 49.5 | 50 | 46.8 | 40 |
| EGCG/HPMC-AS dispersion | 4.6 | 100 | 4.4 | 100 |
| EGCG/HPMC-P dispersion | 3.5 | 100 | 4.2 | 100 |

(continued)

| | In SGF | | In SIF | |
|---|---|---|---|---|
| | Weight of solids (mg) | SGF volume ($\mu$L) | Weight of solids (mg) | SIF volume ($\mu$L) |
| EGCG/Soluplus® dispersion | 3.0 | 100 | 3.5 | 100 |
| EGCG/cellulose acetate dispersion | 3.5 | 100 | 4.4 | 100 |

**Example 7 - Dissolution Tests**

Dissolution testing was performed in 100 mL of standard phosphate-buffered salines (pH 7.4) at 37°C in a jacketed beaker. The temperature was precisely controlled by a Julabo Heating Circulator. EGCG concentration in the solution was in-situ monitored via an Ocean Optics USB2000+ UV-VIS spectrometer equipped with an RT-2MM Dip Probe. The testing was performed with continuous stirring until all solids were dissolved and the medium was free of observable particles, or up to 24 hours. For early period of dissolution, the data acquisition density was relatively large to capture all sudden changes; while at later periods, the data acquisition frequency was reduced due to slow evolution in the medium. For each material, an equivalency of 20 mg EGCG was tested and the experiment was monitored until all solids were dissolved or up to 24 hours. Based on the dissolution data, all EGCG (~20 mg) were released into the medium within the period of dissolution tests for all samples. The actual amounts of materials tested are listed in following table.

**Table 8. Weight of each sample used for dissolution test** (only the sample EGCG/ Soluplus® is according to the claims)

| **Material** | **Weight of sample (mg)** |
|---|---|
| cEGCG | 20.3 |
| aEGCG | 23.0 |
| EGCG/HPMC-AS dispersion | 46.7 |
| EGCG/HPMC-P dispersion | 47.3 |
| EGCG/Soluplus® dispersion | 42.7 |
| EGCG/cellulose acetate dispersion | 45.3 |

**Example 8- Stability Studies on Dispersions of Varying Weight Rates**

20 capsules of Teavigo™ were broken and the solids were suspended in ~300 mL HPLC grade water and filtered to remove the solids. The resultant solution was clear with no evidence of solid particles observed. The solution was then dried under $N_2$ purge until water is fully removed based on visual observation. Such generated EGCG solids were directly used for generating amorphous EGCG and solid dispersions.

EGCG solid dispersions were prepared by lyophilization using four polymers including HPMC-AS, HPMC-P, Soluplus®, and cellulose acetate. Individual solutions of polymer (in dioxane) and EGCG (in water) were prepared. The EGCG aqueous solution was added into the polymer-dioxane solution slowly. Additional fresh dioxane were added into the samples followed by vigorous vortexing to reach a clear solution without visible solids or colloids. The approximate volumes of water and dioxane for the final solution of the dispersions are provided in Table 9 (per gram of EGCG).

**Table 9. The volumes of $H_2O$ and dioxane per gram of EGCG in the final mixtures for lyophilization** (only the sample 50:50 (w/w) EGCG/Soluplus® dispersion is according to the claims)

| | $H_2O$ (mL) | Dioxane (mL) |
|---|---|---|
| 91:9 (w/w) EGCG/Soluplus® dispersion | 63.1 | 65.4 |
| 83:17 (w/w) EGCG/Soluplus® dispersion | 65.5 | 76.0 |

(continued)

| | $H_2O$ (mL) | Dioxane (mL) |
|---|---|---|
| 67:33 (w/w) EGCG/Soluplus® dispersion | 61.9 | 92.9 |
| 50:50 (w/w) EGCG/Soluplus® dispersion | 62.0 | 134.3 |
| 9:91 (w/w) EGCG/Soluplus® dispersion | 66.0 | 707.5 |
| 91:9 (w/w) EGCG/HPMC-AS dispersion | 64.0 | 104.2 |
| 83:17 (w/w) EGCG/HPMC-AS dispersion | 57.3 | 110.7 |
| 67:33 (w/w) EGCG/HPMC-AS dispersion | 61.7 | 150.6 |
| 91:9 (w/w) EGCG/HPMC-P dispersion | 59.5 | 96.9 |
| 83:17 (w/w) EGCG/HPMC-P dispersion | 59.9 | 115.8 |
| 67:33 (w/w) EGCG/HPMC-P dispersion | 54.8 | 164.4 |
| 91:9 (w/w) EGCG/ cellulose acetate dispersion | 61.3 | 90.5 |
| 83:17 (w/w) EGCG/cellulose acetate dispersion | 62.1 | 126.3 |
| 67:33 (w/w) EGCG/cellulose acetate dispersion | 62.3 | 162.1 |

For lyophilization, each EGCG/polymer solution was frozen in a cold bath of dry ice/acetone (at a temperature of -78 °C). The sample vial was then kept in a lyophilizer at -30 °C for 1 day, -10 °C for 2 days, 0 °C for 1 day, and then 20 °C for 4 hours. Pure EGCG aqueous solution was lyophilized as well to generate amorphous EGCG (EGCG) as a reference material for comparison with the dispersions. The lyophilized materials are free flowing materials. Table 10 shows the color results, based on visual observation, before and after such stressing.

**Table 10. Color Observations Before and After Stress** (only the sample 50:50 (w/w) EGCG/Soluplus® dispersion is according to the claims)

| Material | As generated | After stress |
|---|---|---|
| 91:9 (w/w) EGCG/Soluplus® dispersion | very light pink, off white | dark red |
| 83:17 (w/w) EGCG/Soluplus® dispersion | very light pink, off white | dark red |
| 67:33 (w/w) EGCG/Soluplus® dispersion | very light pink, off white | dark red |
| 50:50 (w/w) EGCG/Soluplus® dispersion | very light pink, off white | light pink |
| 9:91 (w/w) EGCG/Soluplus® dispersion | very light pink, off white | light yellow |
| 91:9 (w/w) EGCG/HPMC-AS dispersion | very light pink, off white | very dark red |
| 83:17 (w/w) EGCG/HPMC-AS dispersion | very light pink, off white | very dark red |
| 67:33 (w/w) EGCG/HPMC-AS dispersion | very light pink, off white | very dark red |
| 91:9 (w/w) EGCG/HPMC-P dispersion | very light pink, off white | very dark red |
| 83:17 (w/w) EGCG/HPMC-P dispersion | very light pink, off white | very dark red |
| 67:33 (w/w) EGCG/HPMC-P dispersion | very light pink, off white | very dark red |
| 91:9 (w/w) EGCG/ cellulose acetate dispersion | very light pink, off white | very dark red |
| 83:17 (w/w) EGCG/cellulose acetate dispersion | very light pink, off white | very dark red |

(continued)

| Material | As generated | After stress |
|---|---|---|
| 67:33 (w/w) EGCG/cellulose acetate dispersion | very light pink, off white | very dark red |
| amorphous EGCG | very light pink, off white | pink |

## Claims

1. A solid dispersion comprising an approximately 1:1 weight ratio of amorphous EGCG and polyvinyl caprolactam-polyvinyl acetate-polyethylene glycol graft co-polymer.

2. A pharmaceutical composition comprising the solid dispersion of claim 1.

3. The pharmaceutical composition of claim 2, further comprising one or more pharmaceutically acceptable excipients.

4. A pharmaceutical composition according to claim 3, for use in a method of sustained release delivery of EGCG.

5. A sustained release pharmaceutical composition comprising a solid dispersion of claim 1.

6. The solid dispersion of claim 1 wherein the glass transition temperature is less than 150°C, optionally wherein the glass transition temperature is 143°C.

7. The solid dispersion of claim 1, wherein the x-ray powder diffraction pattern is x-ray amorphous after being stressed at 40°C and 75% relative humidity after 11 days.

8. The solid dispersion of claim 1, wherein the EGCG released within 20 minutes is less than that of amorphous EGCG in a pH 7.4 PBS medium at 37°C or is less than that of crystalline EGCG in a pH 7.4 PBS medium at 37°C.

9. The solid dispersion of claim 1, wherein less than 60% of the EGCG is released within 20 minutes, optionally wherein 50% of the EGCG is released within 20 minutes.

10. A supplement comprising a solid-dispersion of claim 1.

11. A pharmaceutical composition according to claim 2 or 3, for use in a method of delivering EGCG to a human.

12. The pharmaceutical composition of claims 2, 3, and 5 in tablet or capsule form.

13. A foodstuff additive comprising the solid dispersion as defined in claim 1.

## Patentansprüche

1. Feste Dispersion, die ein Gewichtsverhältnis von etwa 1:1 von amorphem EGCG und Polyvinylcaprolactam-Polyvinylacetat-Polyethylenglykol-Pfropfcopolymer umfasst.

2. Pharmazeutische Zusammensetzung, umfassend die feste Dispersion nach Anspruch 1.

3. Pharmazeutische Zusammensetzung nach Anspruch 2, ferner umfassend einen oder mehrere pharmazeutisch verträgliche Hilfsstoffe.

4. Pharmazeutische Zusammensetzung nach Anspruch 3 zur Verwendung in einem Verfahren zur verzögerten Freisetzung von EGCG.

5. Pharmazeutische Zusammensetzung mit verzögerter Freisetzung, die eine feste Dispersion nach Anspruch 1 umfasst.

**6.** Feste Dispersion nach Anspruch 1, wobei die Glasübergangstemperatur weniger als 150 °C beträgt, optional wobei die Glasübergangstemperatur 143 °C beträgt.

**7.** Feste Dispersion nach Anspruch 1, wobei das Röntgenpulverbeugungsmuster nach 11 Tagen bei 40 °C und 75 % relativer Luftfeuchtigkeit röntgenamorph ist.

**8.** Feste Dispersion nach Anspruch 1, wobei das innerhalb von 20 Minuten freigesetzte EGCG geringer ist als das amorphe EGCG in einem PBS-Medium mit einem pH-Wert von 7,4 bei 37 °C oder geringer ist als das kristalline EGCG in einem PBS-Medium mit einem pH-Wert von 7,4 bei 37 °C.

**9.** Feste Dispersion nach Anspruch 1, wobei weniger als 60 % des EGCG innerhalb von 20 Minuten freigesetzt werden, optional wobei 50 % des EGCG innerhalb von 20 Minuten freigesetzt werden.

**10.** Nahrungsergänzungsmittel, umfassend eine Feststoffdispersion nach Anspruch 1.

**11.** Pharmazeutische Zusammensetzung nach Anspruch 2 oder 3 zur Verwendung in einem Verfahren zur Abgabe von EGCG an einen Menschen.

**12.** Pharmazeutische Zusammensetzung nach Anspruch 2, 3 und 5 in Tabletten- oder Kapselform.

**13.** Lebensmittelzusatzstoff, umfassend die feste Dispersion wie in Anspruch 1 definiert.

## Revendications

**1.** Dispersion solide comprenant un rapport pondéral d'environ 1:1 d'EGCG amorphe et de copolymère greffé de polyvinyl caprolactame-polyacétate de vinyle-polyéthylène glycol.

**2.** Composition pharmaceutique comprenant la dispersion solide selon la revendication 1.

**3.** Composition pharmaceutique selon la revendication 2, comprenant en outre un ou plusieurs excipients pharmaceutiquement acceptables.

**4.** Composition pharmaceutique selon la revendication 3, destinée à être utilisée dans un procédé d'administration à libération prolongée d'EGCG.

**5.** Composition pharmaceutique à libération prolongée comprenant une dispersion solide selon la revendication 1.

**6.** Dispersion solide selon la revendication 1, dans laquelle la température de transition vitreuse est inférieure à 150 °C, éventuellement dans laquelle la température de transition vitreuse est de 143 °C.

**7.** Dispersion solide selon la revendication 1, dans laquelle le diagramme de diffraction des rayons X sur poudre est amorphe aux rayons X après avoir été contraint à 40 °C et 75 % d'humidité relative après 11 jours.

**8.** Dispersion solide selon la revendication 1, dans laquelle l'EGCG libéré dans les 20 minutes est inférieure à celle de l'EGCG amorphe dans un milieu PBS à pH 7,4 à 37 °C ou est inférieure à celle de l'EGCG cristallin dans un milieu PBS à pH 7,4 à 37 °C.

**9.** Dispersion solide selon la revendication 1, dans laquelle moins de 60 % de l'EGCG est libéré en 20 minutes, éventuellement dans laquelle 50 % de l'EGCG est libérée en 20 minutes.

**10.** Complément comprenant une dispersion solide selon la revendication 1.

**11.** Composition pharmaceutique selon la revendication 2 ou 3, destinée à être utilisée dans un procédé d'administration d'EGCG à un humain.

**12.** Composition pharmaceutique selon les revendications 2, 3 et 5 sous forme de comprimé ou de capsule.

**13.** Additif alimentaire comprenant la dispersion solide telle que définie dans la revendication 1.

Hierarchical terminology of green tea extracts

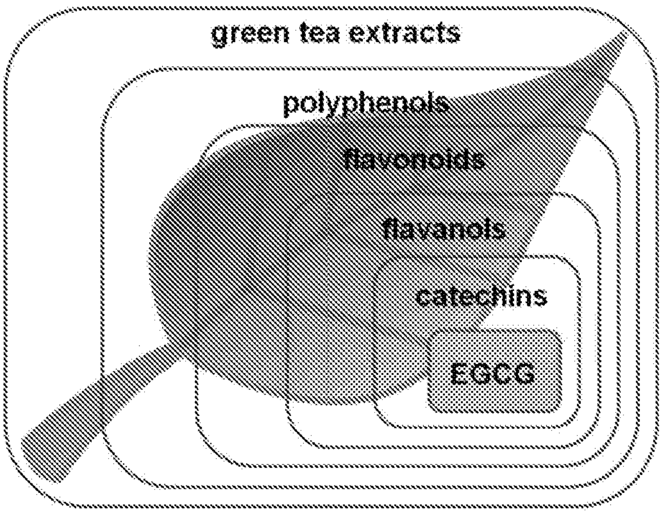


FIG. 1

XRPD pattern of cEGCG

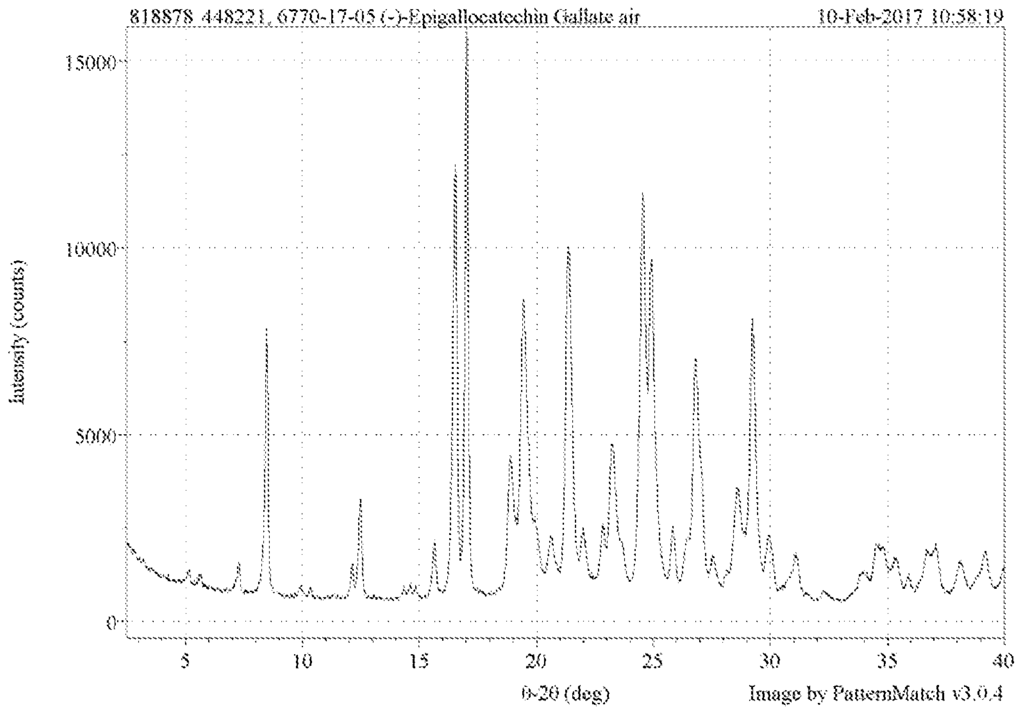


FIG. 2

SEM images of cEGCG

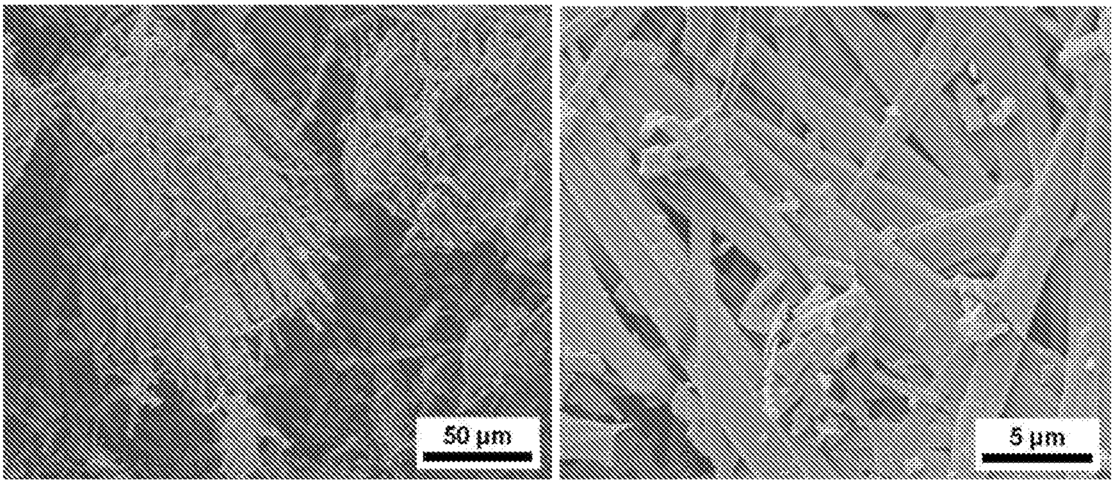


FIG. 3

TGA thermogram of cEGCG

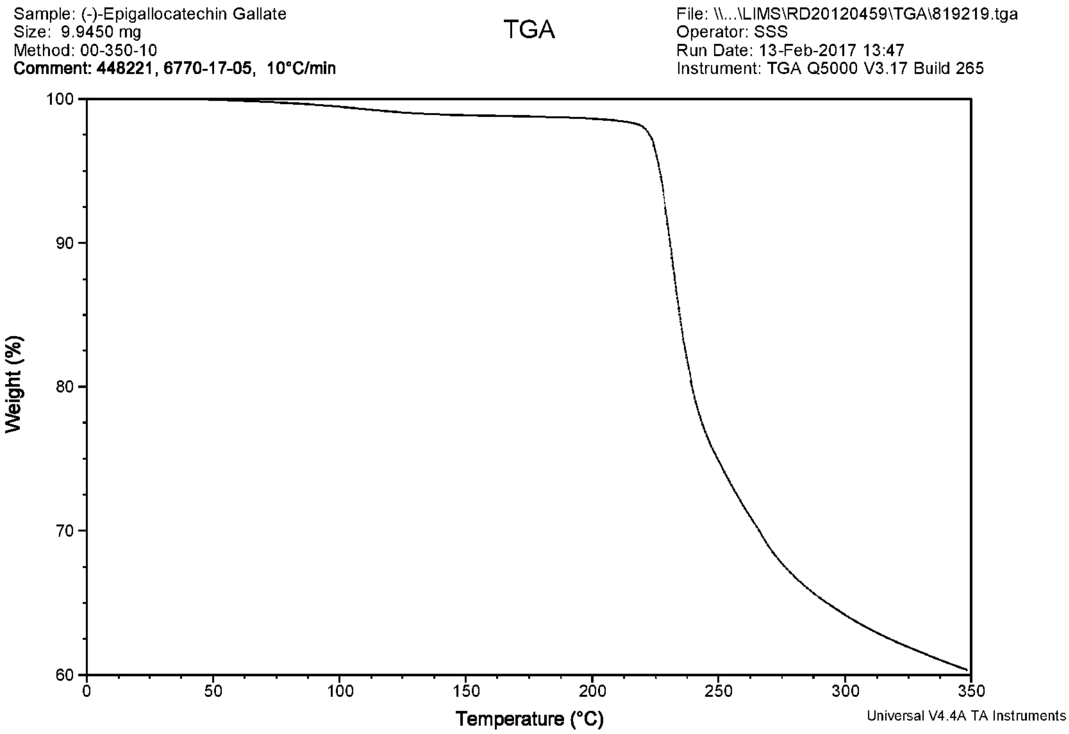


FIG. 4

EGCG release profile of cEGCG in pH 7.4 PBS medium at 37°C

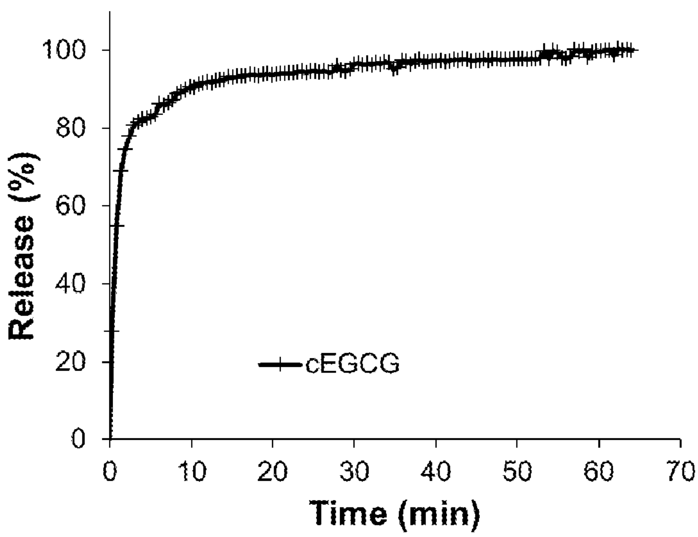


FIG. 5

XRPD pattern of aEGCG

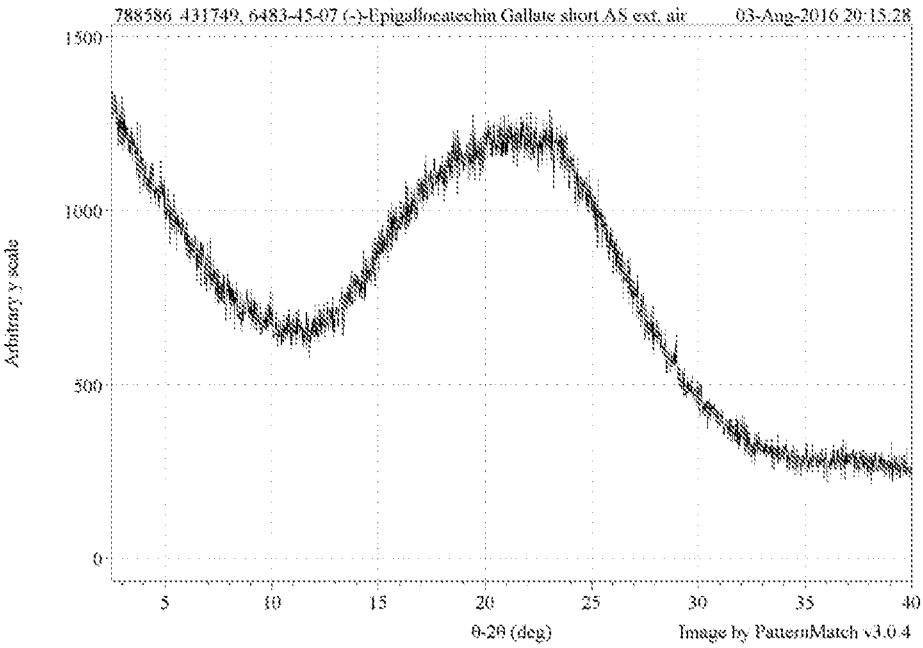


FIG. 6

SEM images of aEGCG

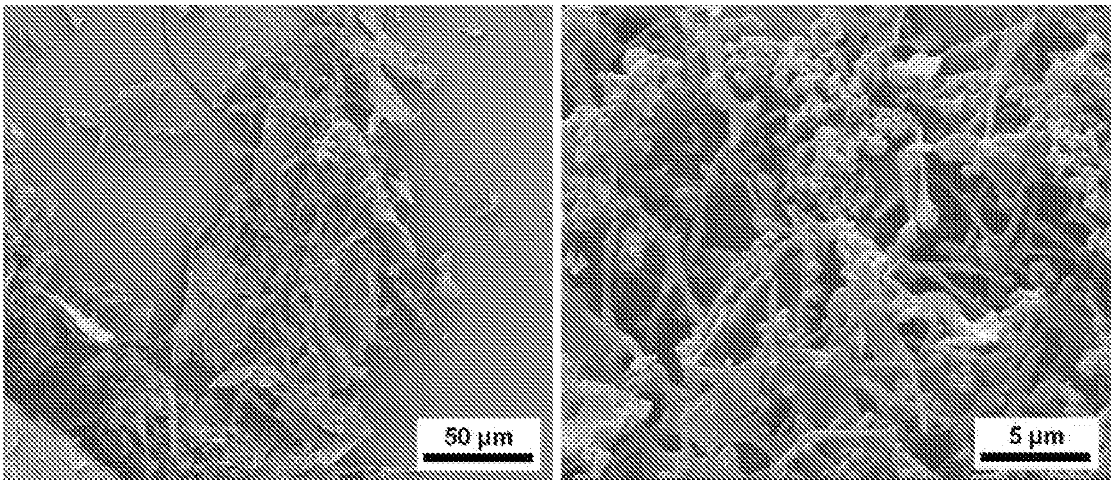


FIG. 7

TGA thermogram of aEGCG

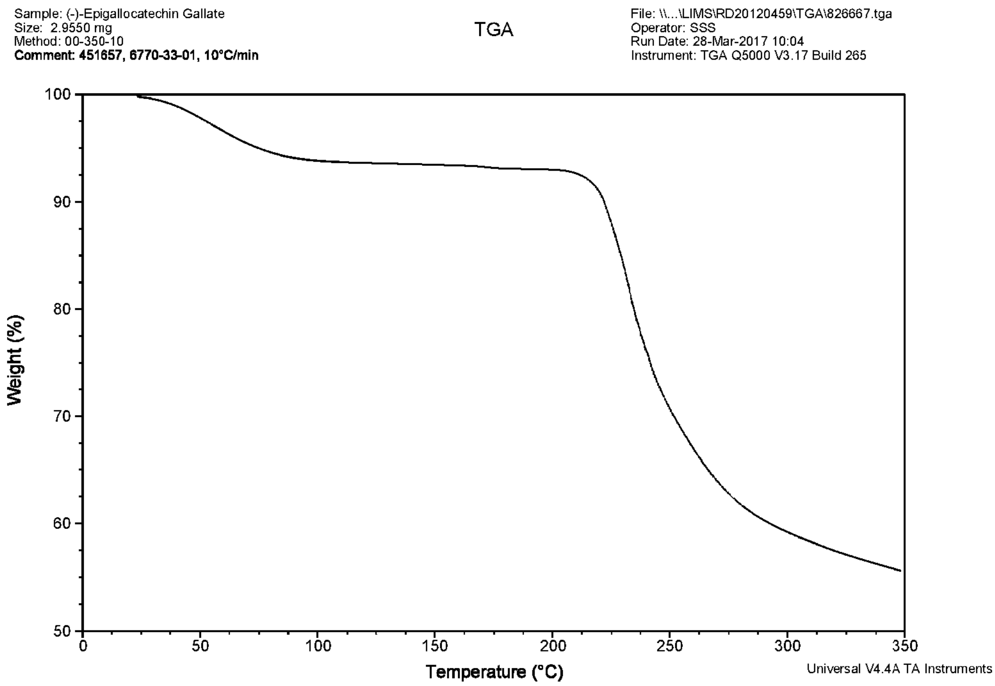


FIG. 8

mDSC of aEGCG

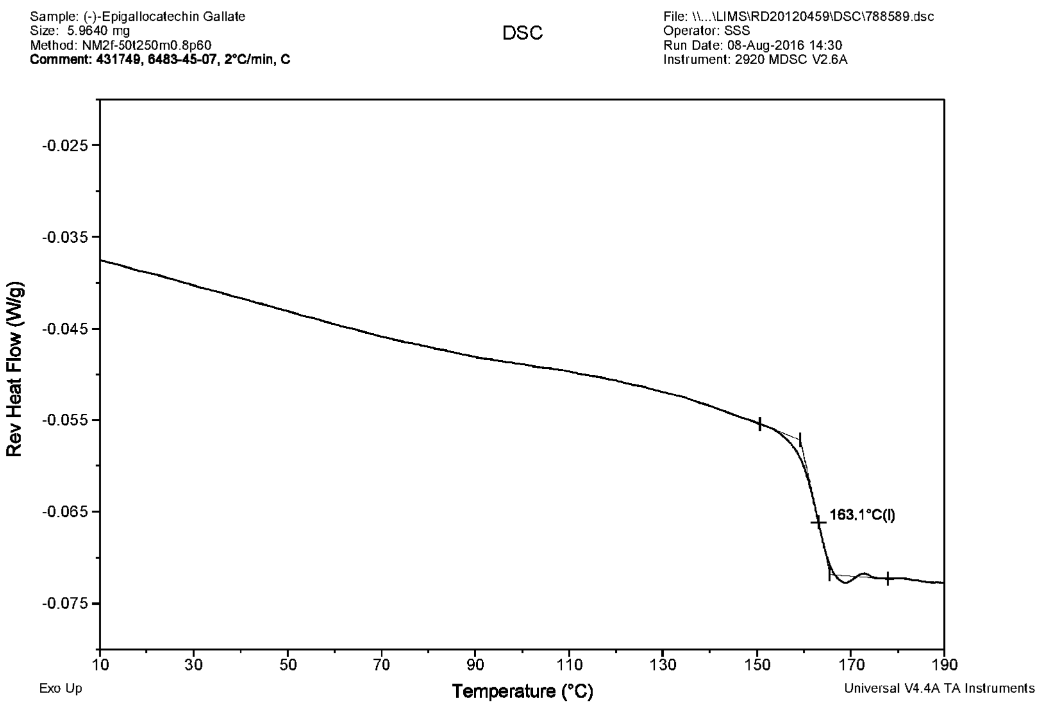


FIG. 9

XRPD pattern of aEGCG stressed at about 40 °C/75%RH for 11 d

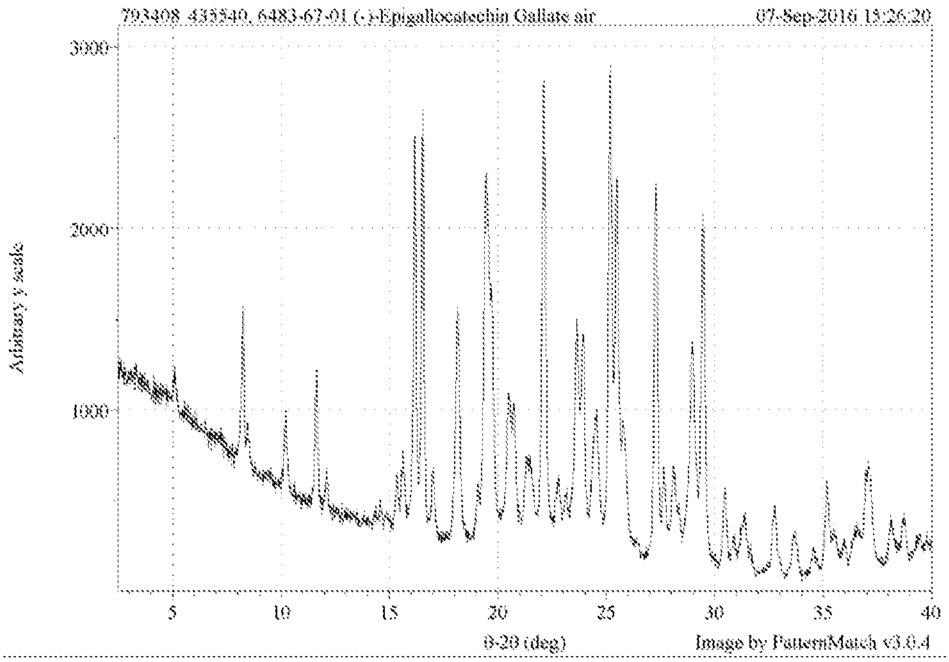


FIG. 10

EGCG release profile of aEGCG in pH 7.4 PBS medium at 37°C

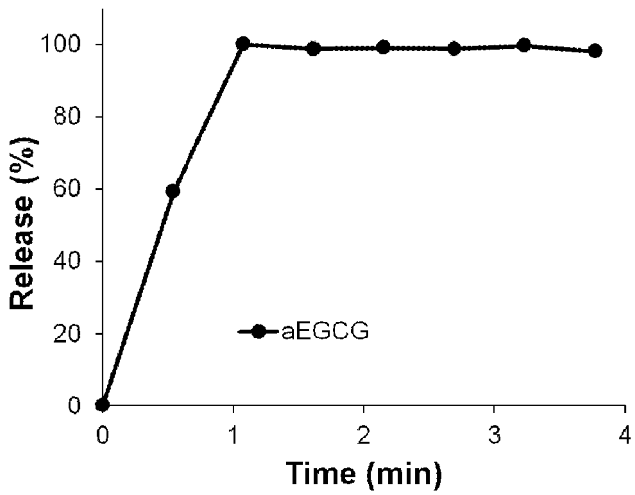


FIG. 11

XRPD pattern of EGCG/HPMC-AS dispersion

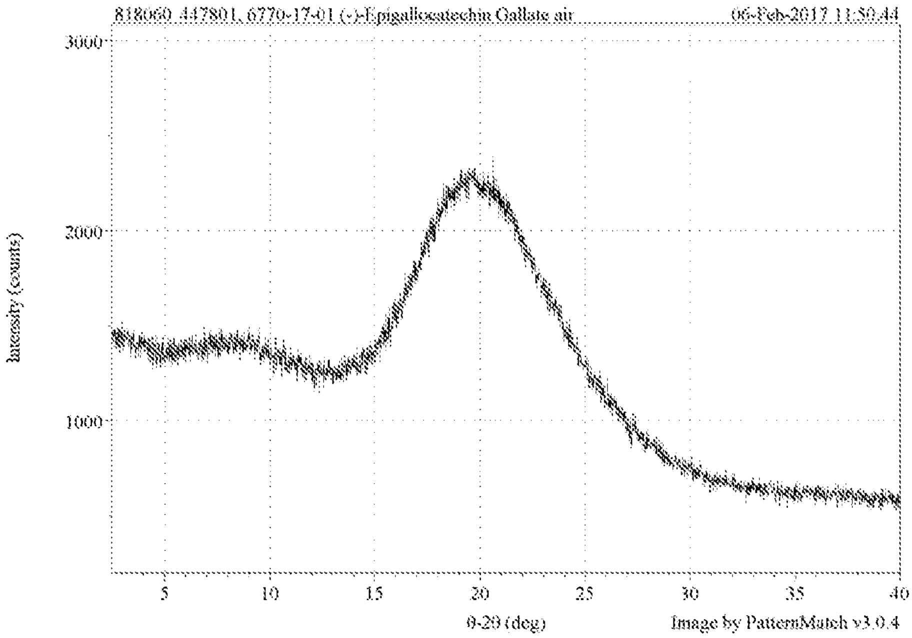


FIG. 12

SEM images of EGCG/HPMC-AS dispersion

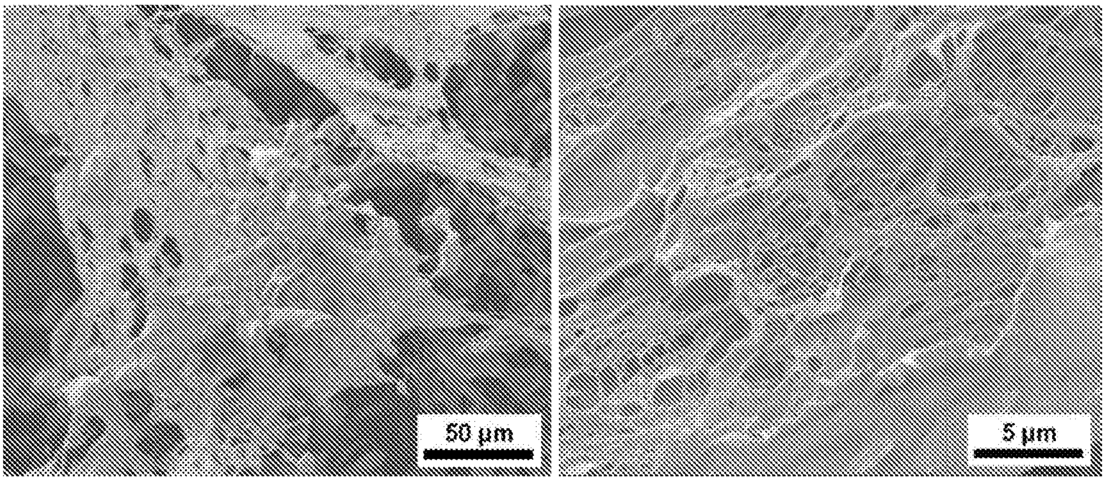


FIG. 13

TGA thermogram of EGCG/HPMC-AS dispersion

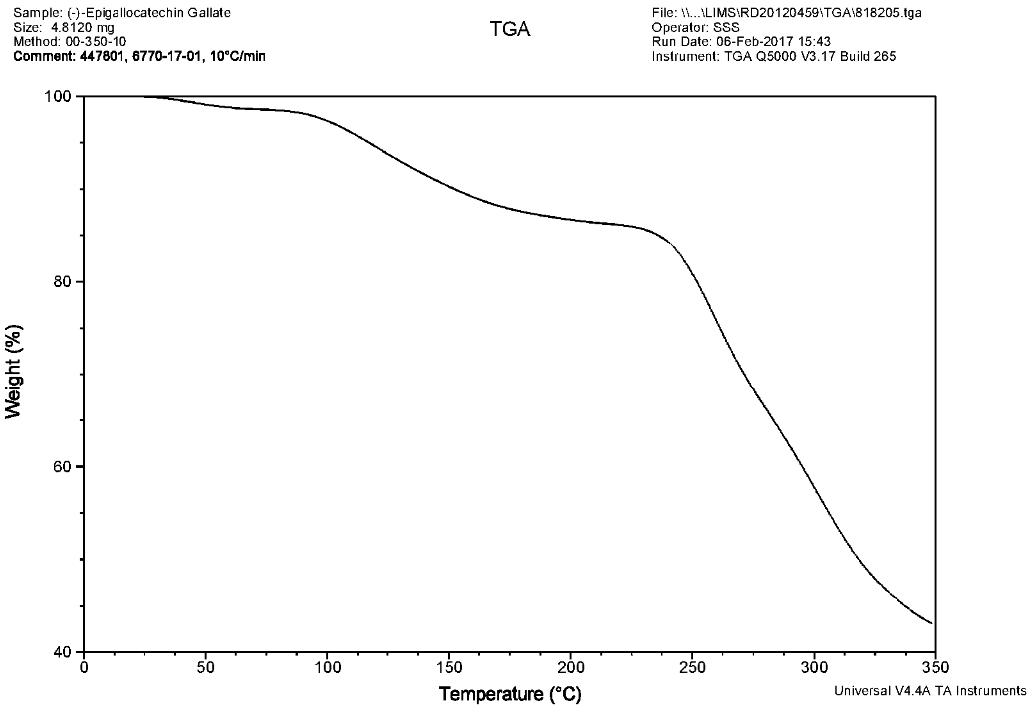


FIG. 14

mDSC of EGCG/HPMC-AS dispersion

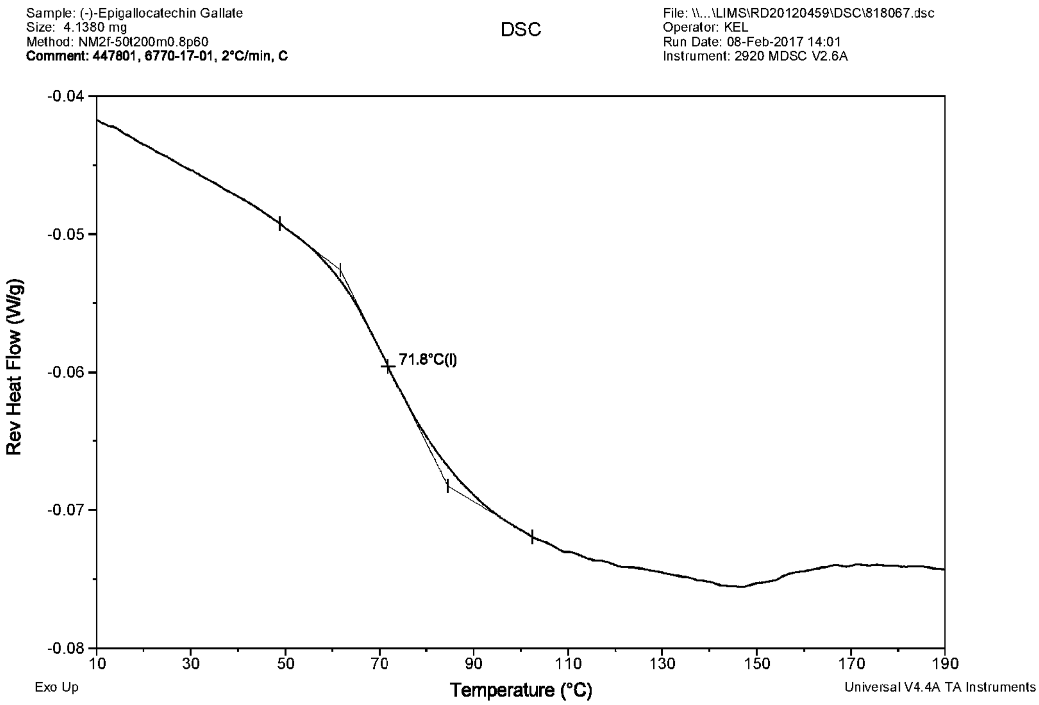


FIG. 15

XRPD pattern of EGCG/HPMC-AS dispersion at about 40°C/75%RH for 11 d

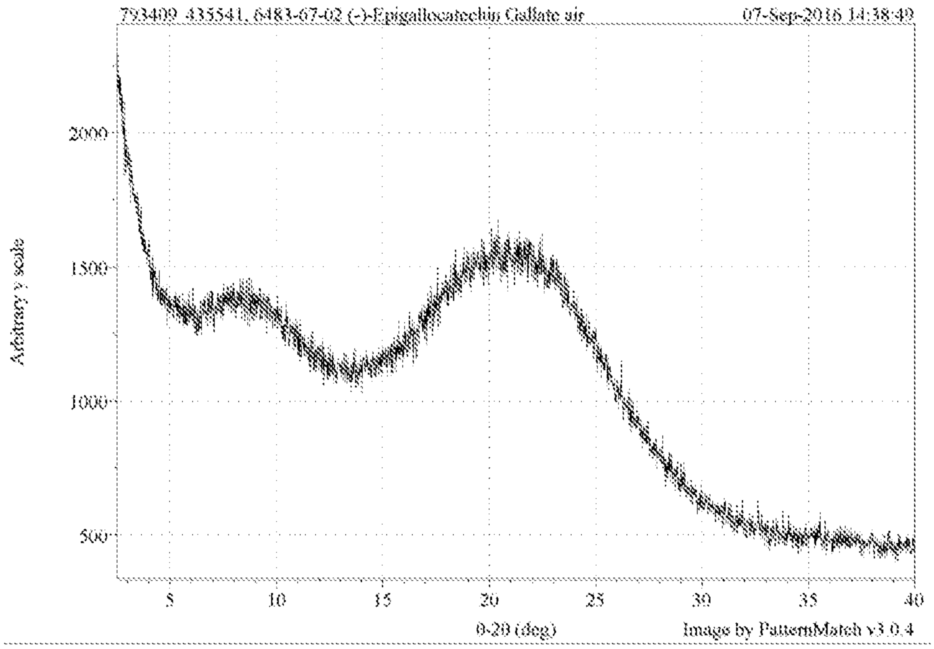


FIG. 16

EGCG release profile of EGCG/HPMC-AS dispersion in pH 7.4 PBS medium at 37°C

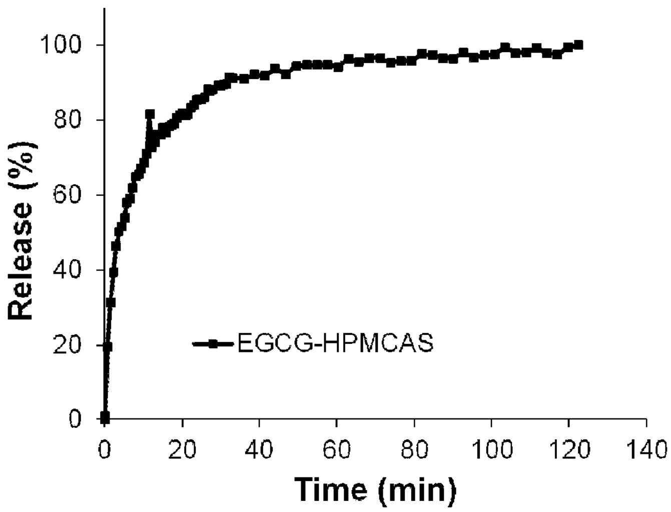


FIG. 17

XRPD pattern of EGCG/HPMC-P dispersion

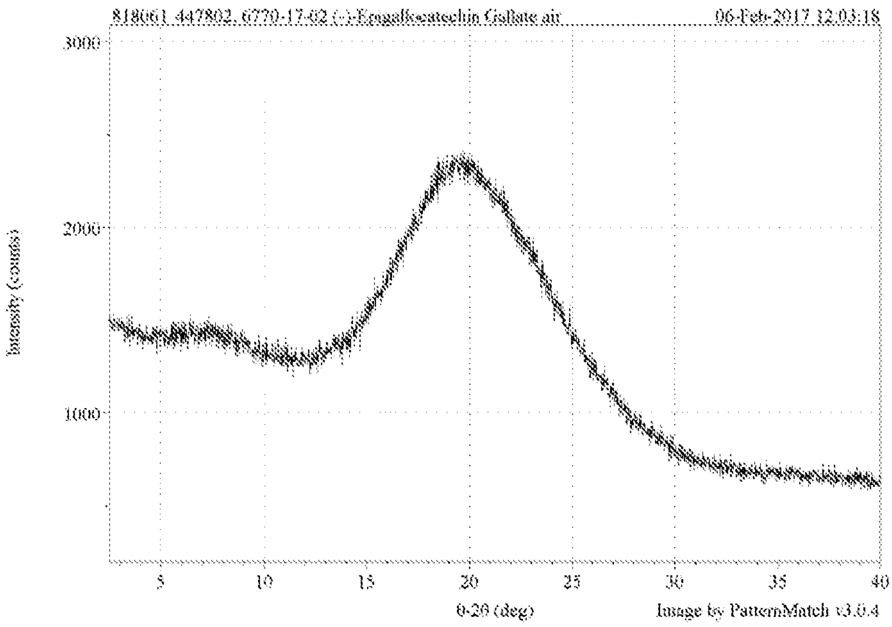


FIG. 18

SEM images of EGCG/HPMC-P dispersion

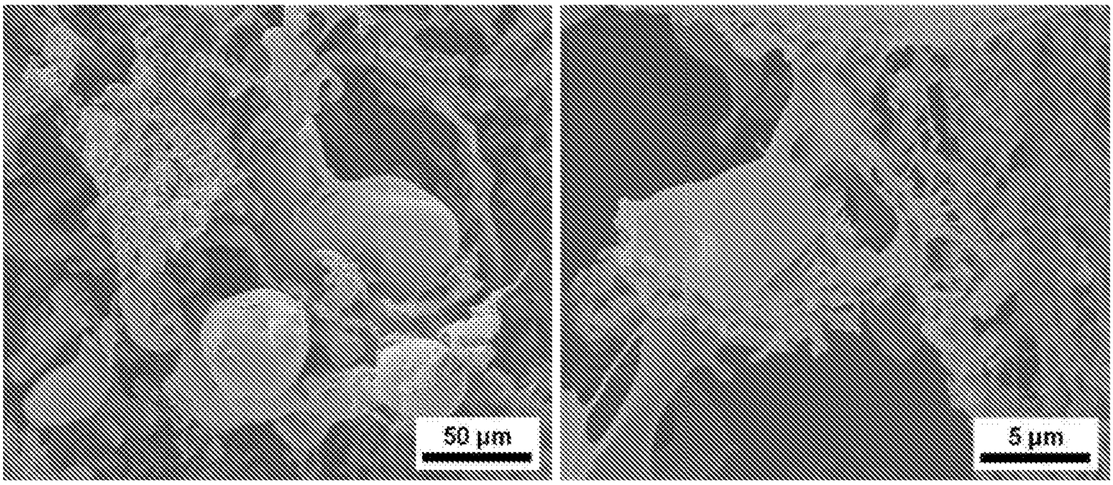


FIG. 19

TGA thermogram of EGCG/HPMC-P dispersion

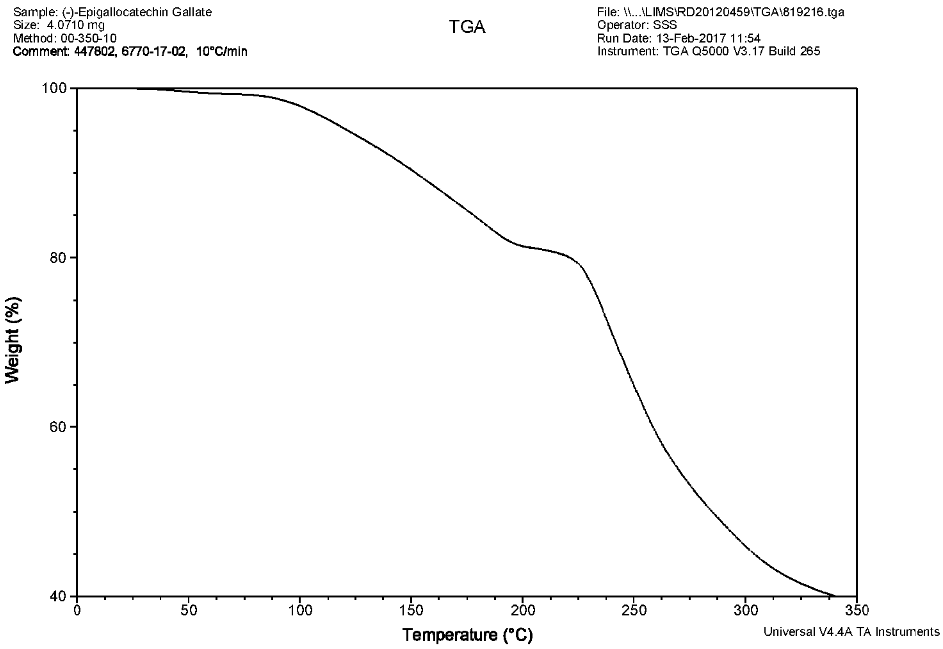


FIG. 20

mDSC of EGCG/HPMC-P dispersion

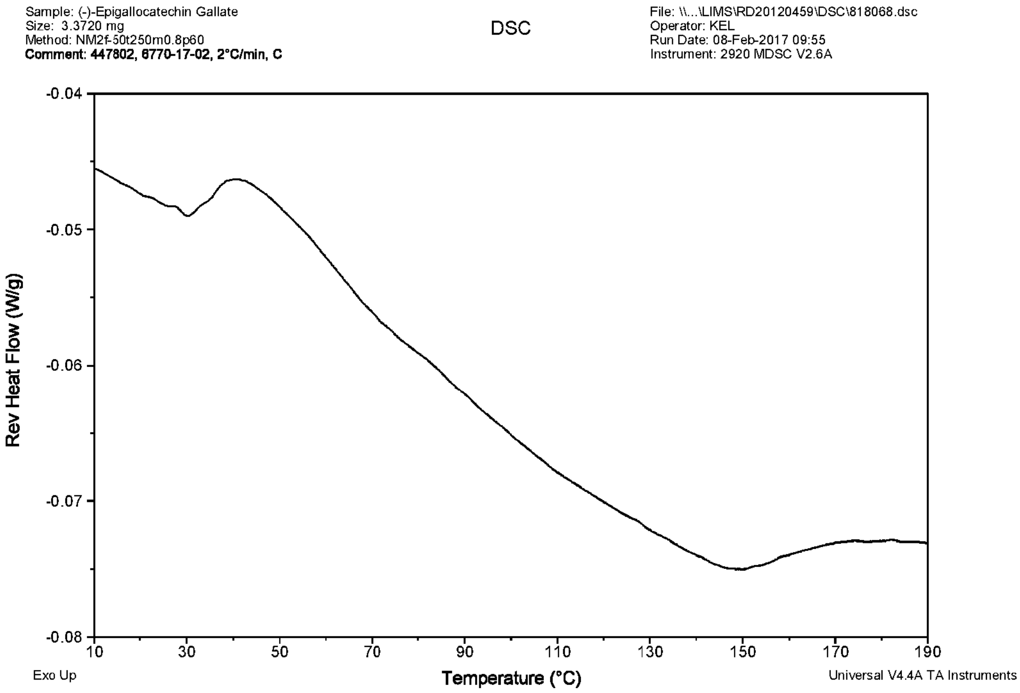


FIG. 21

XRPD pattern of EGCG/ HPMC-P dispersion at about 40°C/75%RH for 11 d

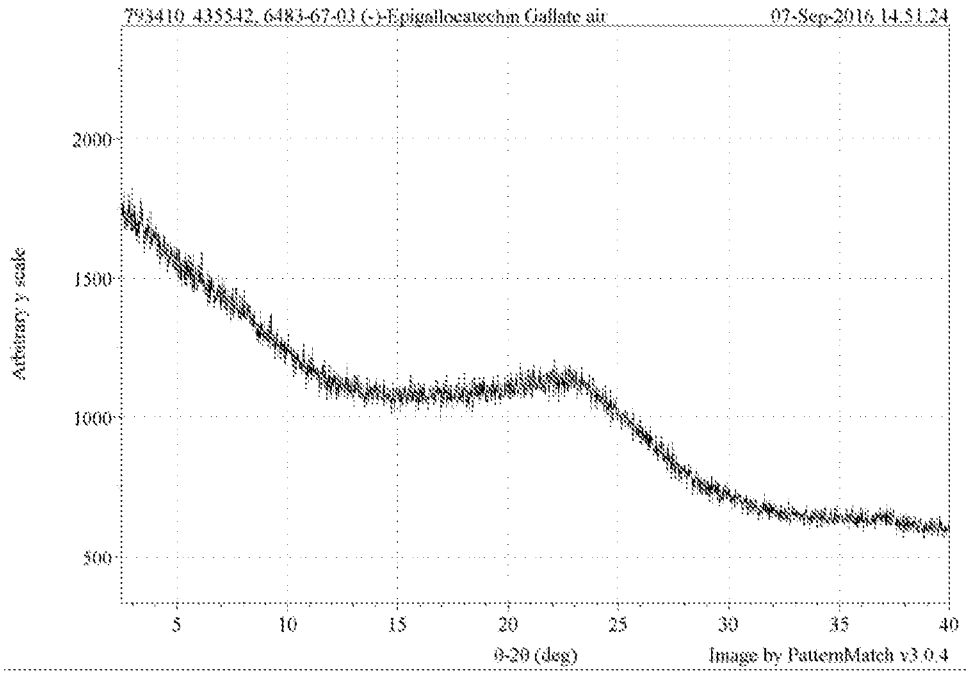


FIG. 22

EGCG release profile of EGCG/HPMC-P dispersion in pH 7.4 PBS medium at 37°C

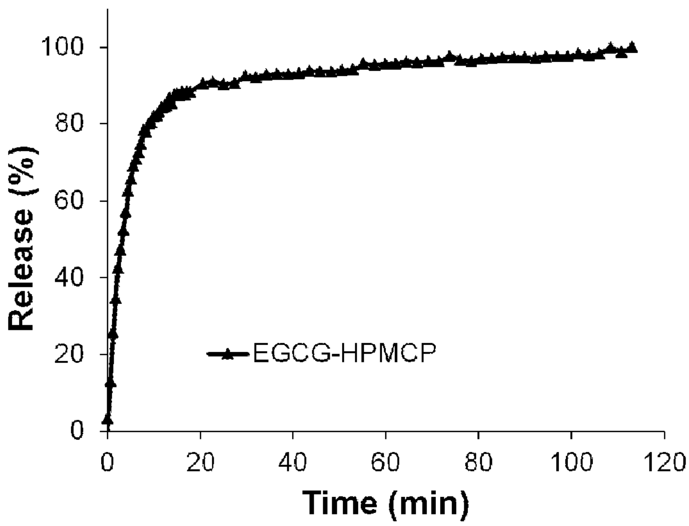


FIG. 23

XRPD pattern of EGCG/Soluplus® dispersion

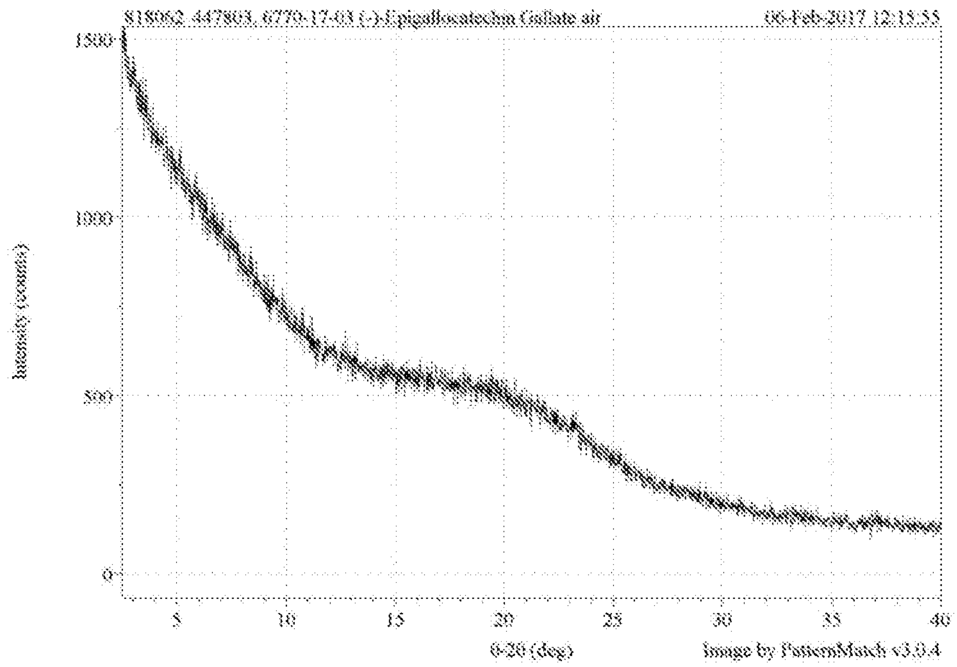


FIG. 24

SEM images of EGCG/Soluplus® dispersion

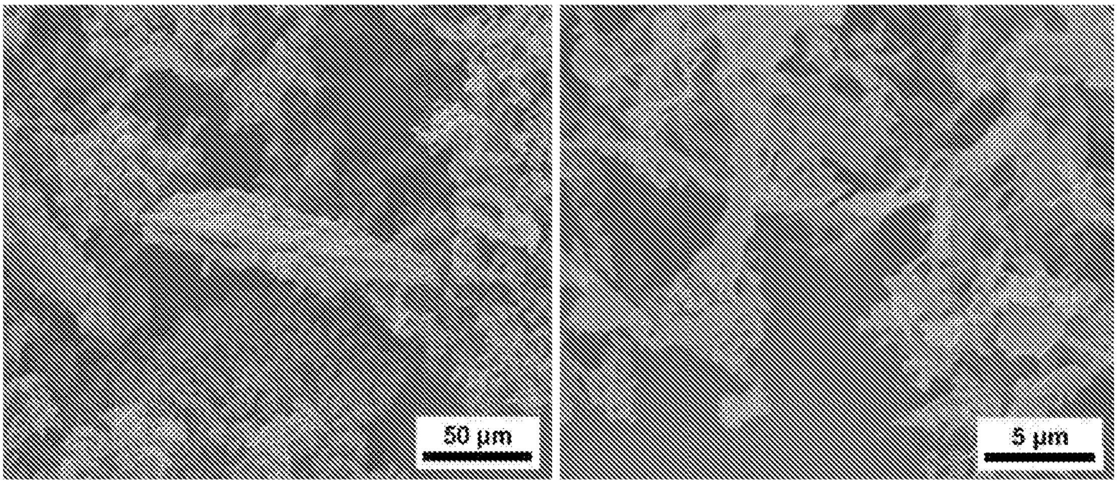


FIG. 25

TGA thermogram of EGCG/Soluplus® dispersion

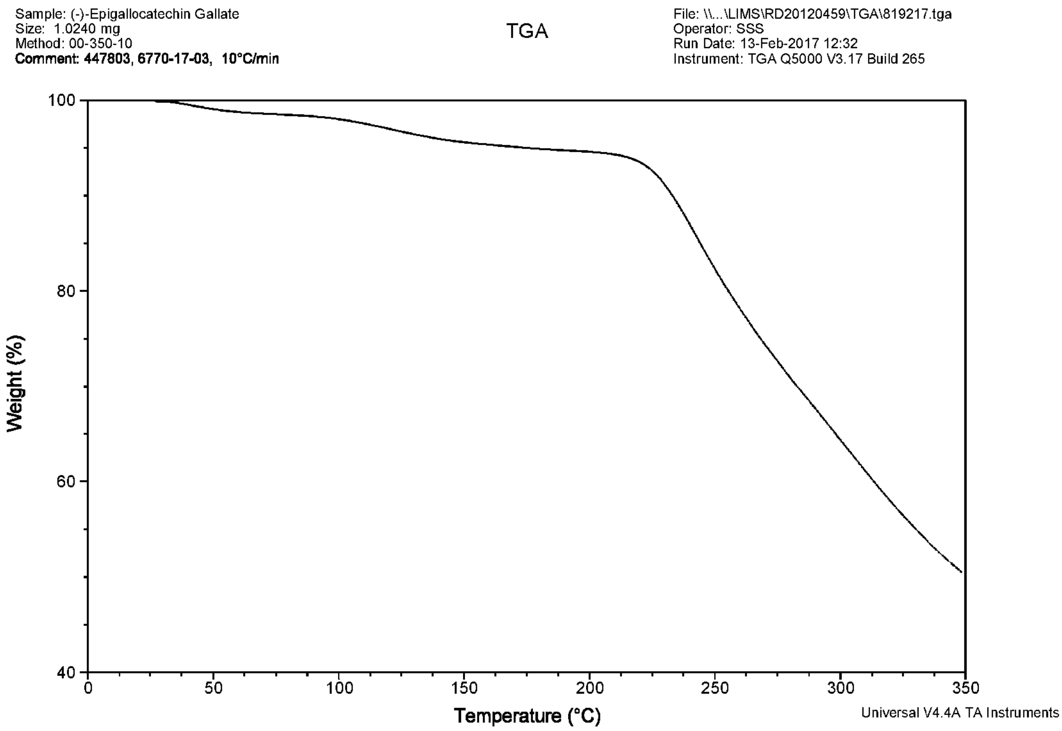


FIG. 26

mDSC of EGCG/Soluplus® dispersion

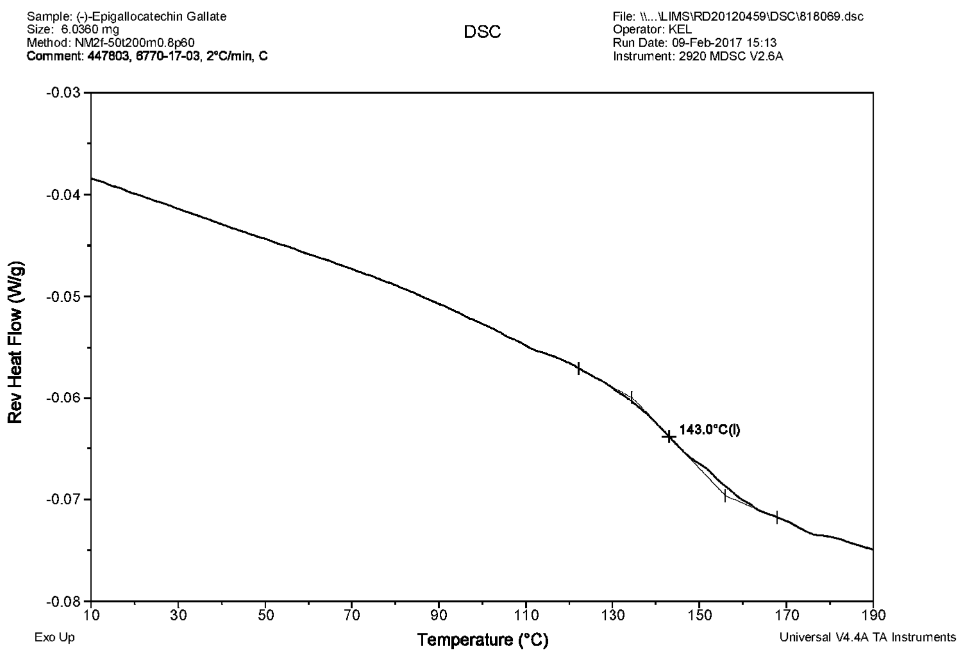


FIG. 27

XRPD pattern of EGCG/Soluplus® dispersion at about 40ºC/75%RH for 11 d

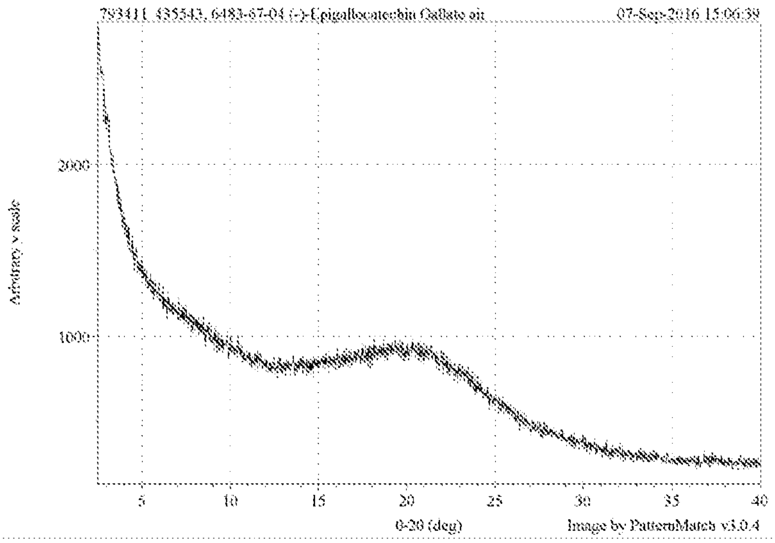


FIG. 28

EGCG release profile of EGCG/Soluplus® dispersion in pH 7.4 PBS medium at 37°C

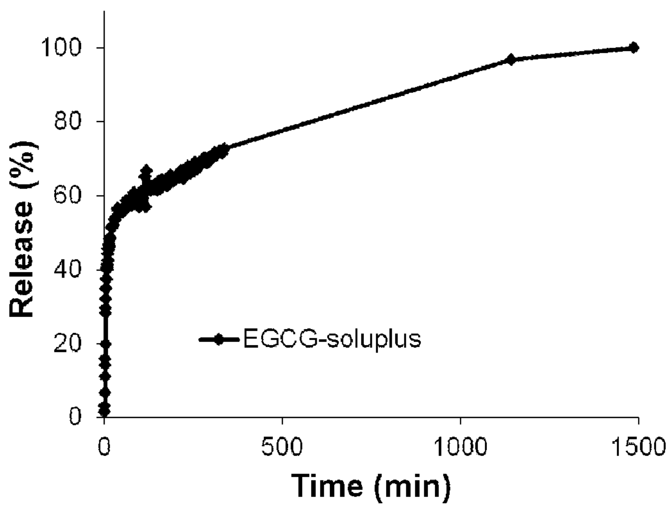


FIG. 29

XRPD pattern of EGCG/cellulose acetate dispersion

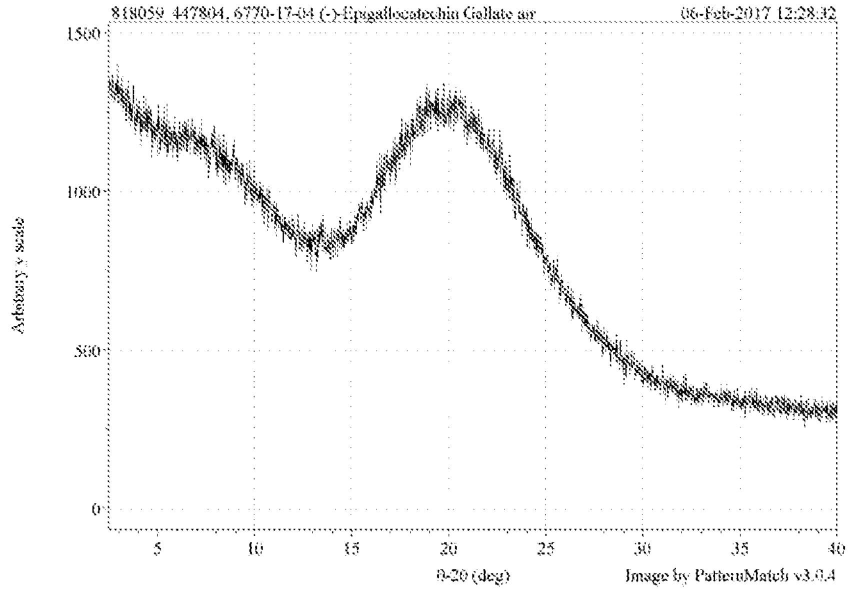


FIG. 30

SEM images of EGCG/cellulose acetate dispersion

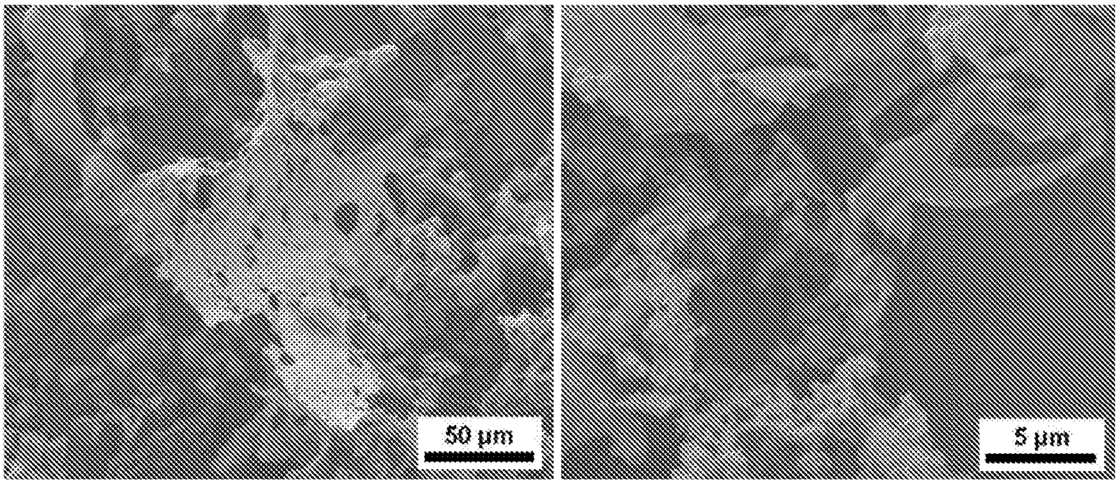


FIG. 31

TGA thermogram of EGCG/cellulose acetate dispersion

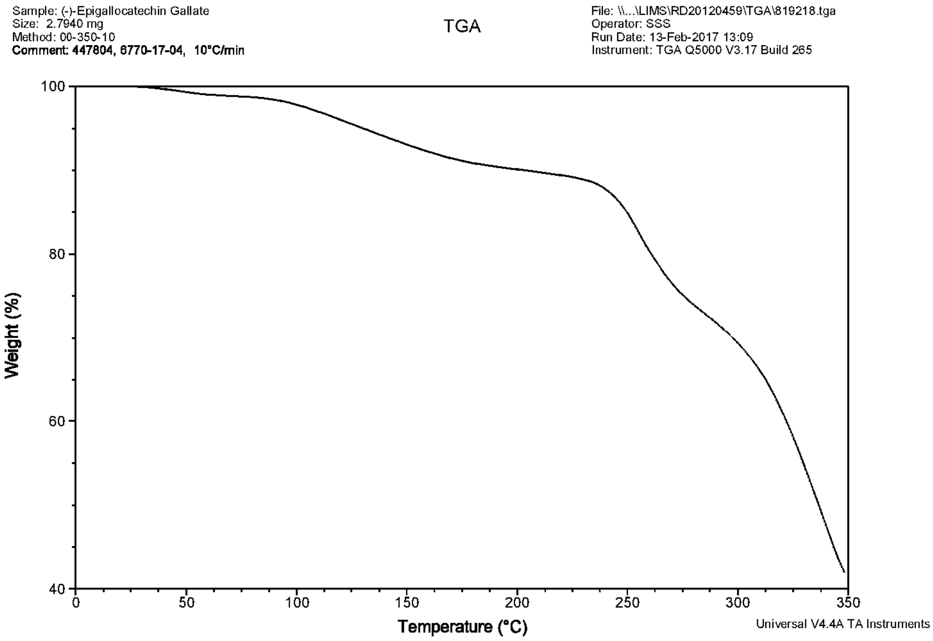


FIG. 32

mDSC of EGCG/cellulose acetate dispersion

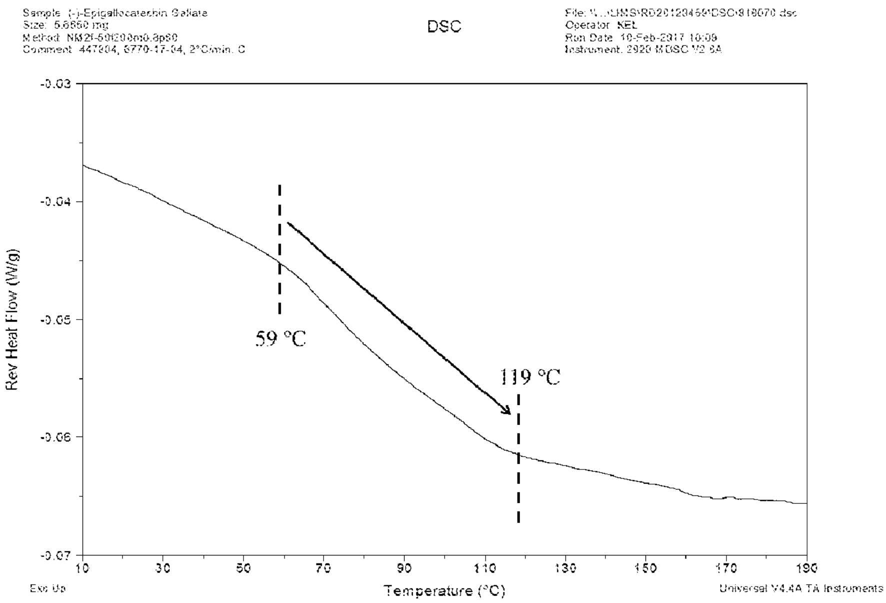


FIG. 33

XRPD pattern of EGCG/cellulose acetate dispersion at about 40°C/75%RH for 11 d

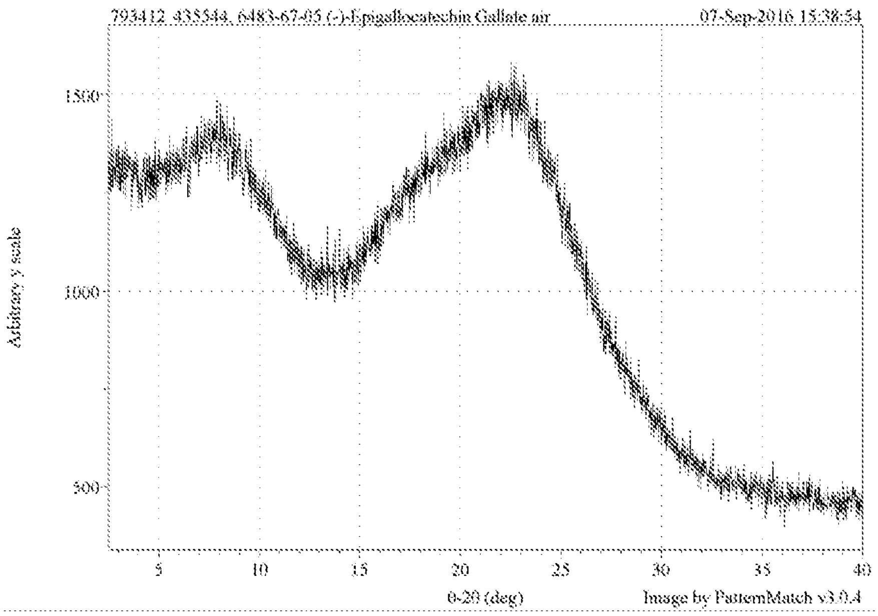


FIG. 34

EGCG release profile of EGCG/cellulose acetate dispersion in pH 7.4 PBS medium at 37°C

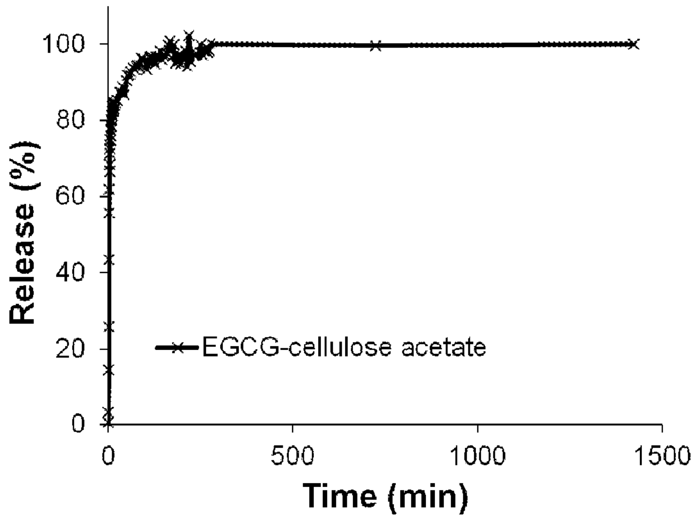


FIG. 35

XRPD pattern overlays

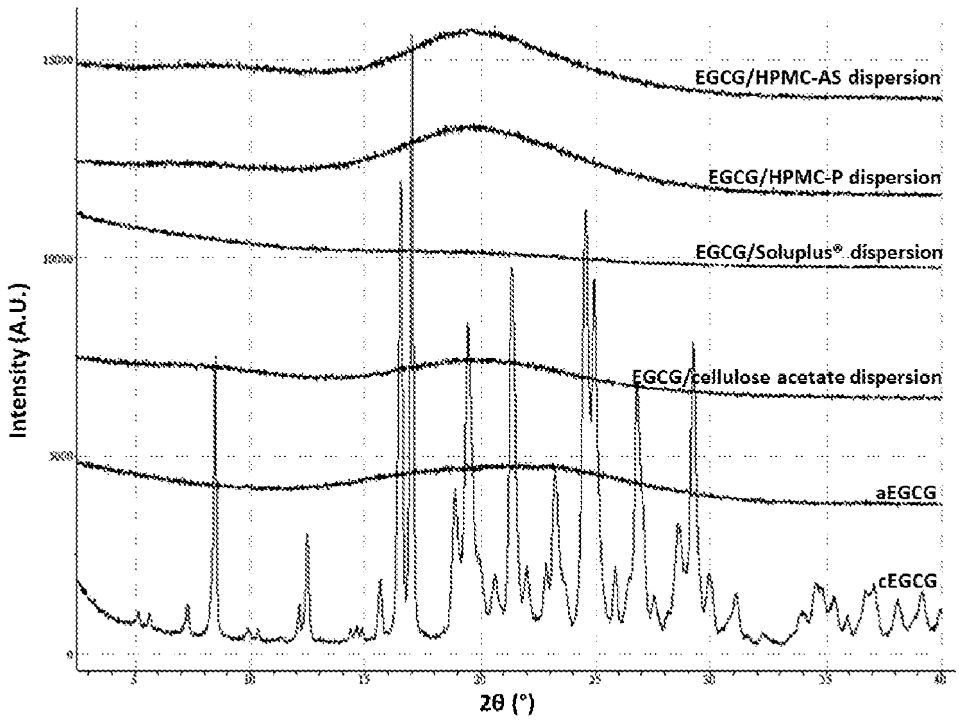


FIG. 36

TGA overlays

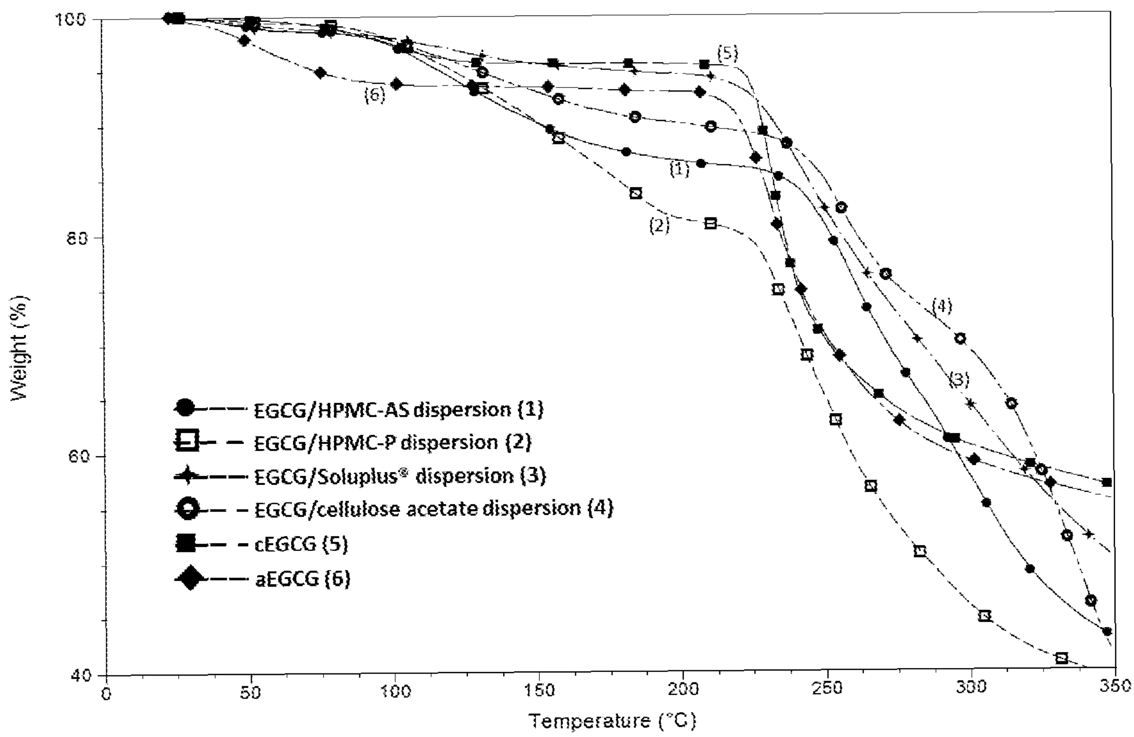


FIG. 37

mDSC overlays

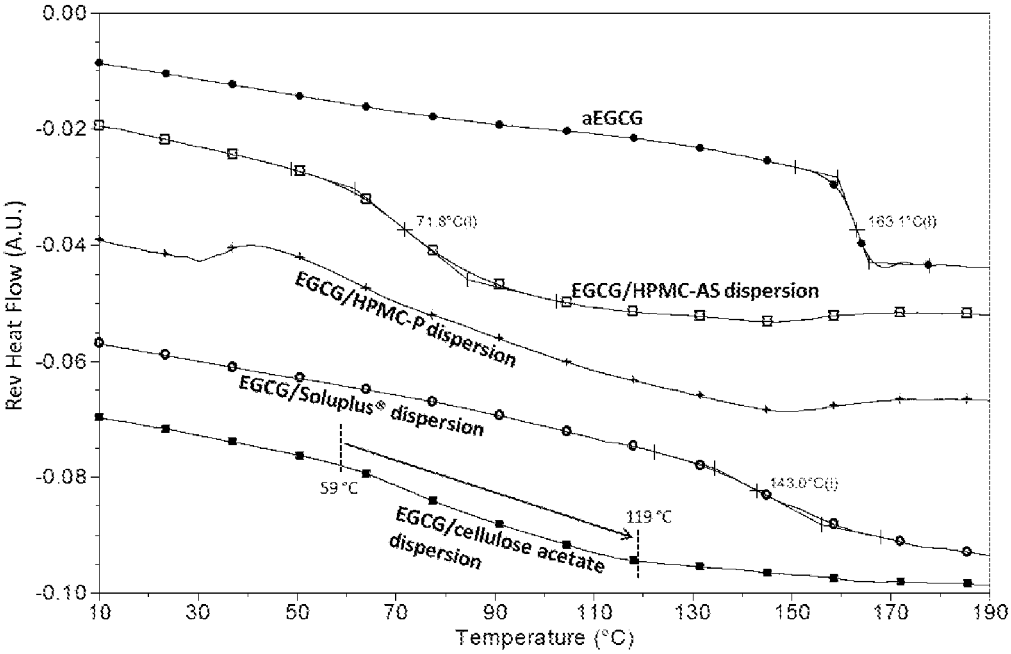


FIG. 38

XRPD pattern overlays

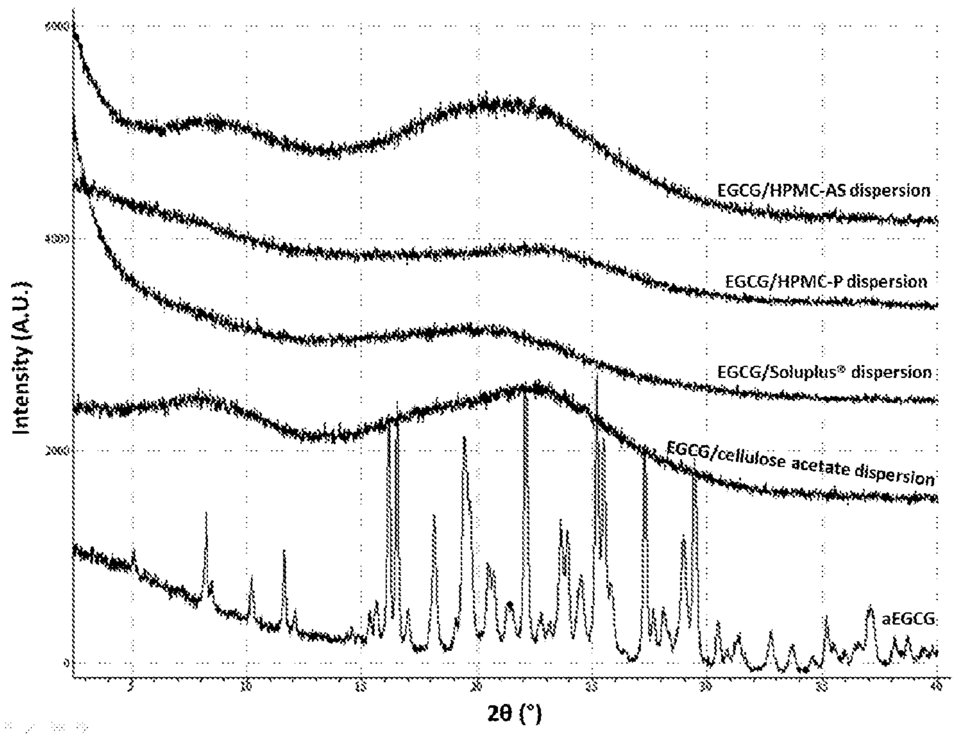


FIG. 39

EGCG release profile overlays in pH 7.4 PBS medium at 37°C

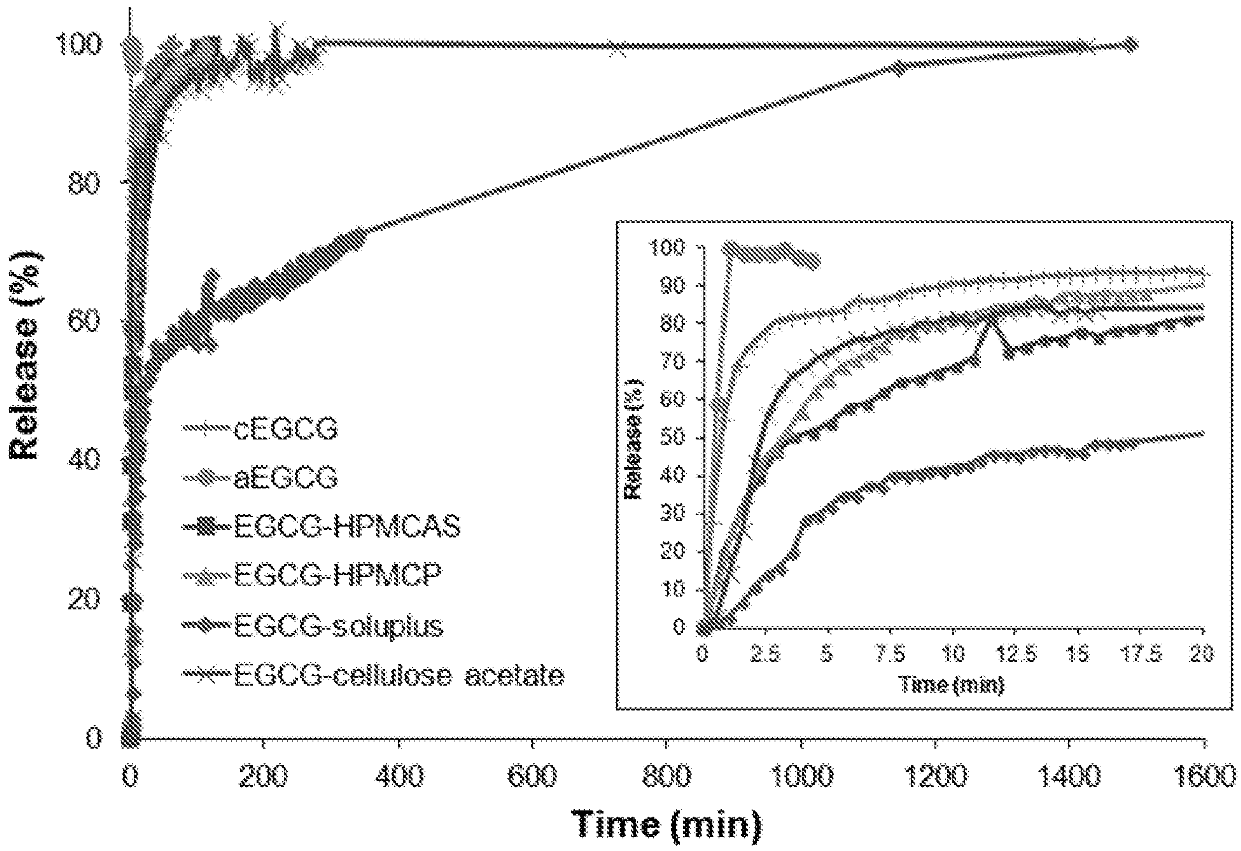


FIG. 40

XRPD pattern overlays

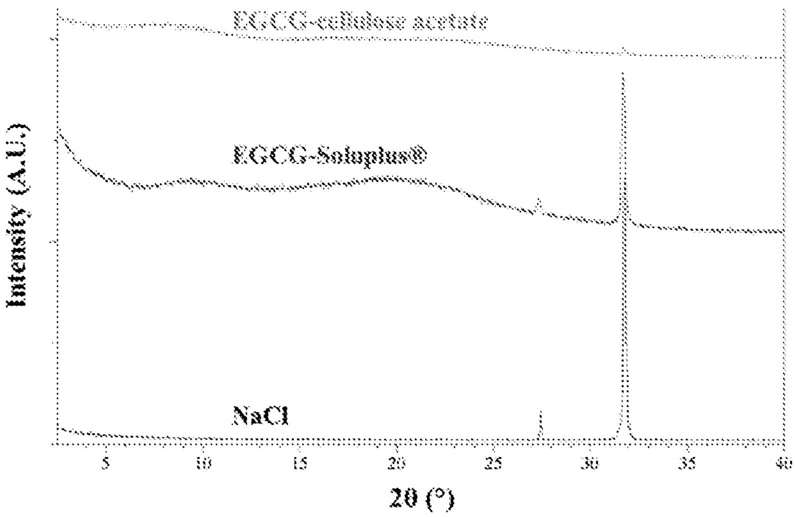


FIG. 41

EGCG release in the first 20 min in pH 7.4 PBS medium at 37 ºC

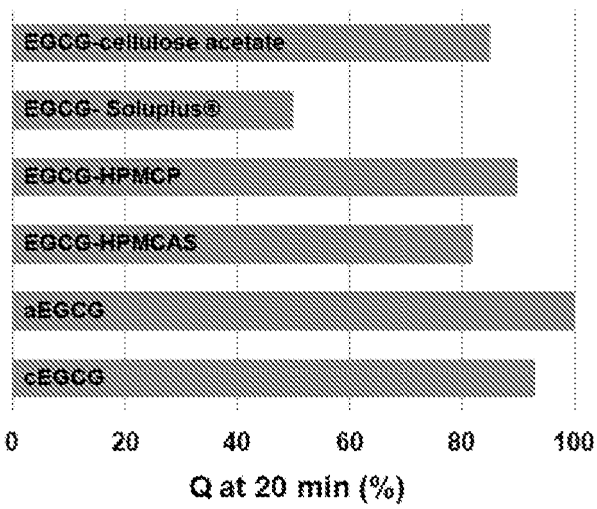


FIG. 42

Kinetic release model fit for cEGCG

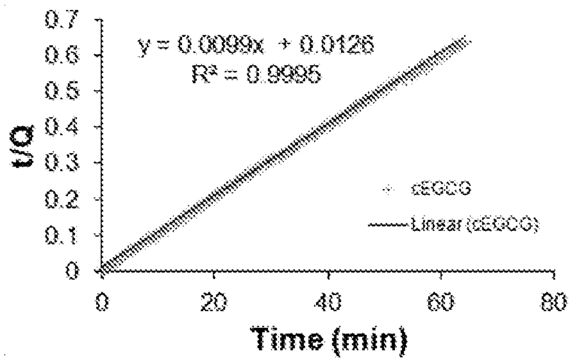


FIG. 43

Kinetic release model fit for aEGCG

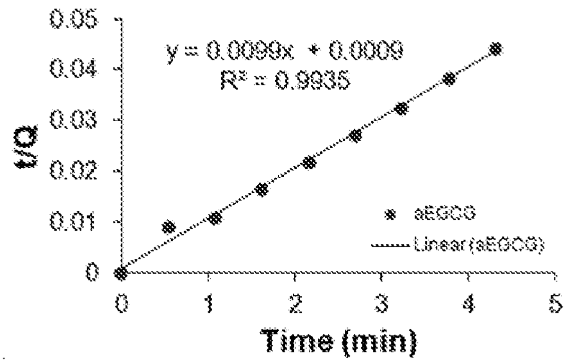


FIG. 44

Kinetic release model fit for EGCG/HPMC-AS

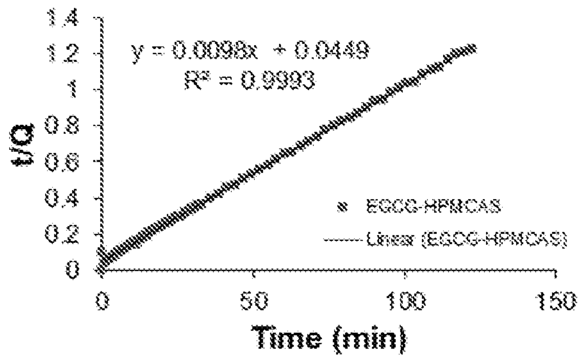


FIG. 45

Kinetic release model fit for EGCG/HPMC-P

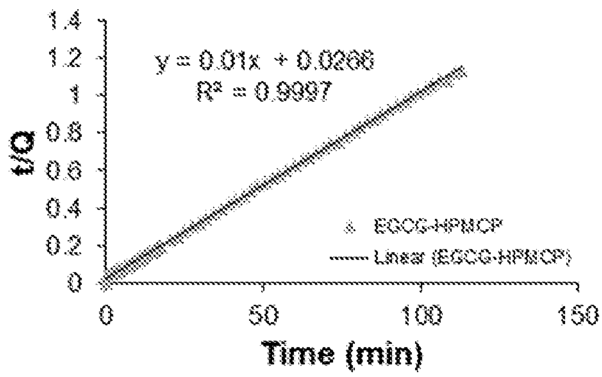


FIG. 46

Kinetic release model fit for EGCG/cellulose acetate

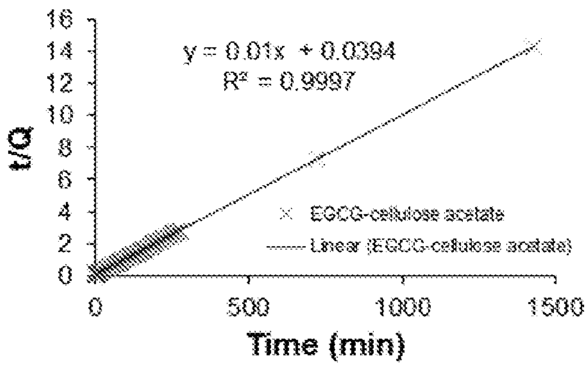


FIG. 47

Kinetic release model fit for EGCG/Soluplus®

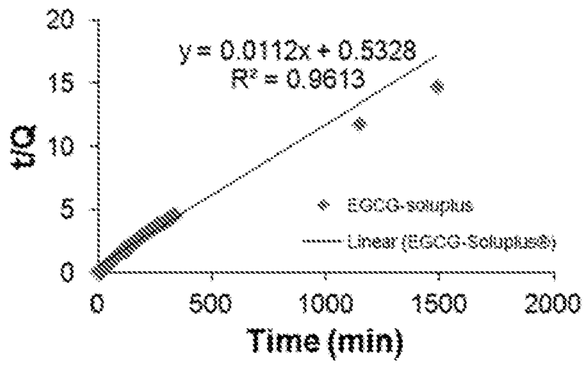


FIG. 48

Bi-phasic release profile of EGCG/Soluplus® dispersion (PSO-FO model fit)

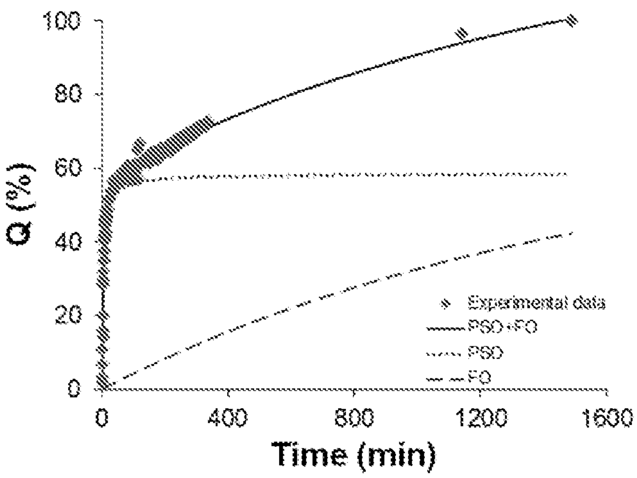


FIG. 49

XRPD pattern for 91:9 (w/w) EGCG/Soluplus® dispersion

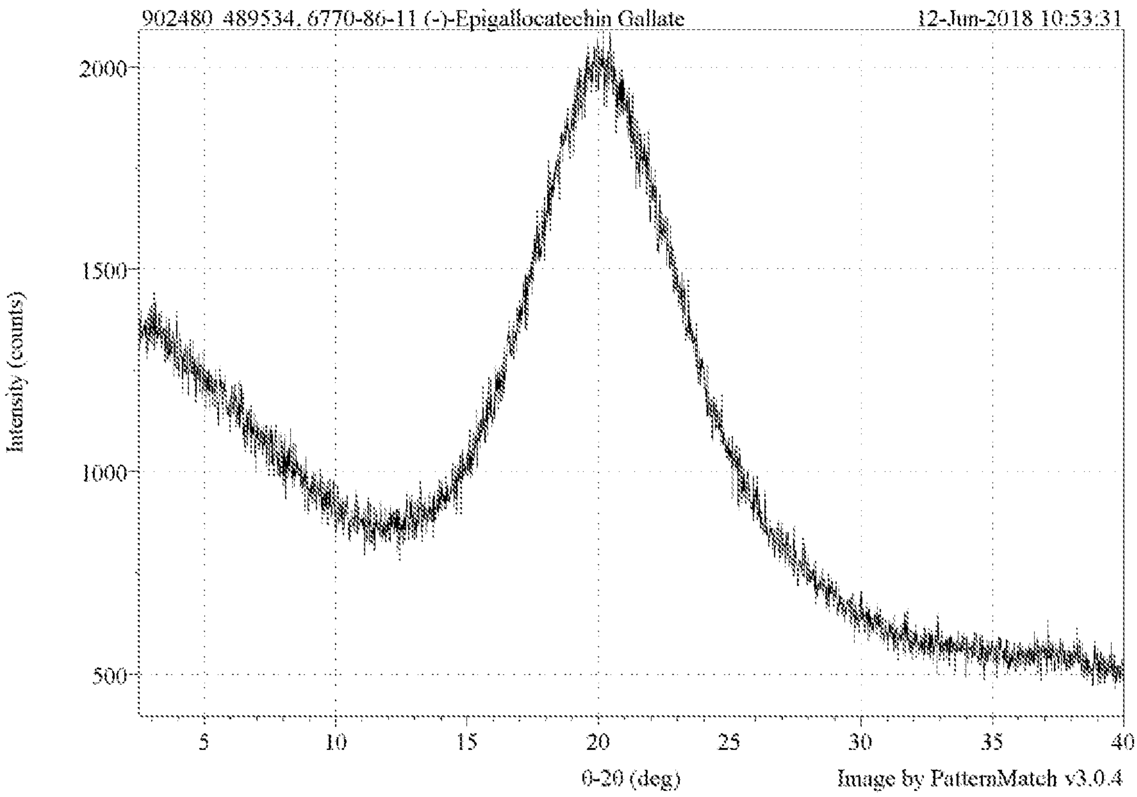


FIG. 50

XRPD pattern for 83:17 (w/w) EGCG/Soluplus® dispersion

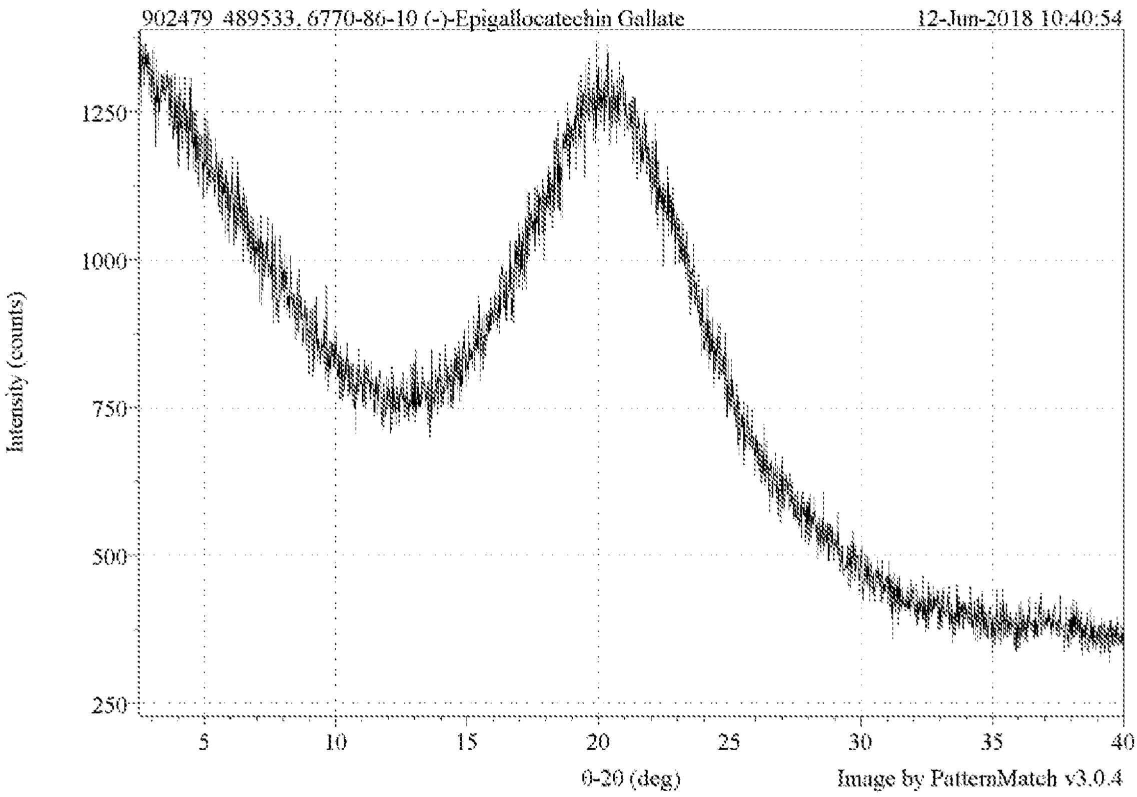


FIG. 51

XRPD pattern for 67:33 (w/w) EGCG/Soluplus® dispersion

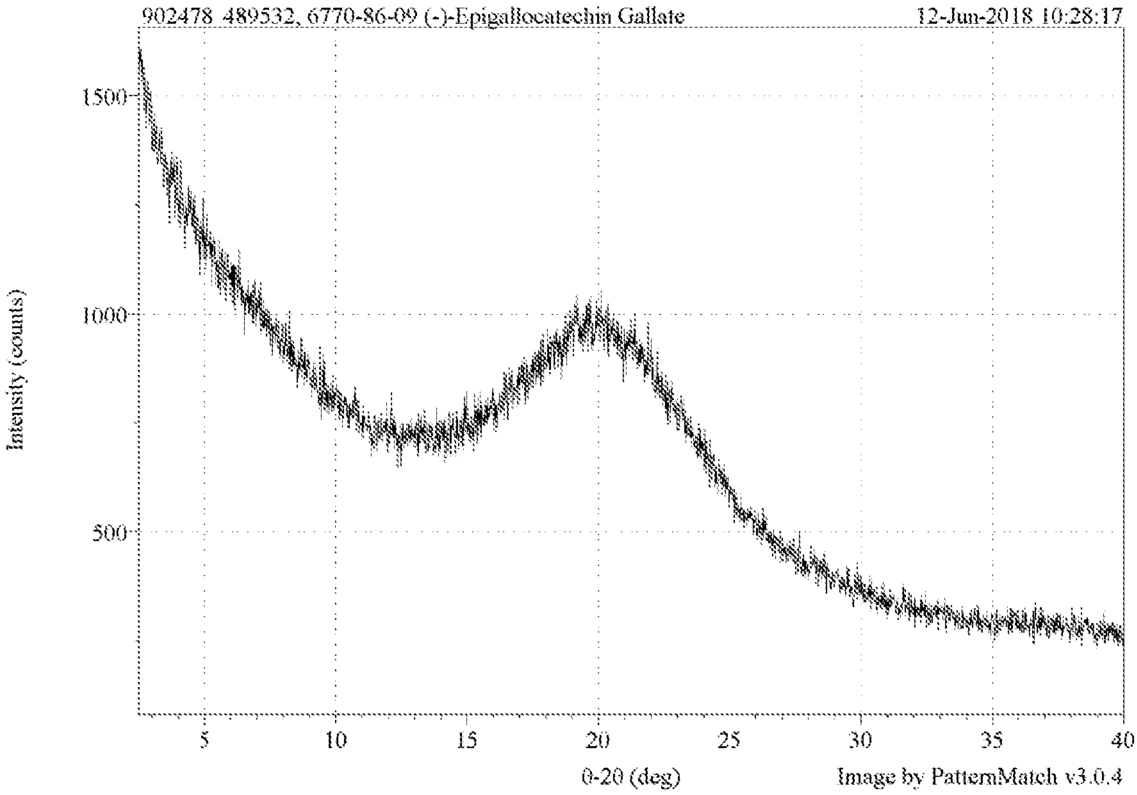


FIG. 52

XRPD pattern for 50:50 (w/w) EGCG/Soluplus® dispersion.

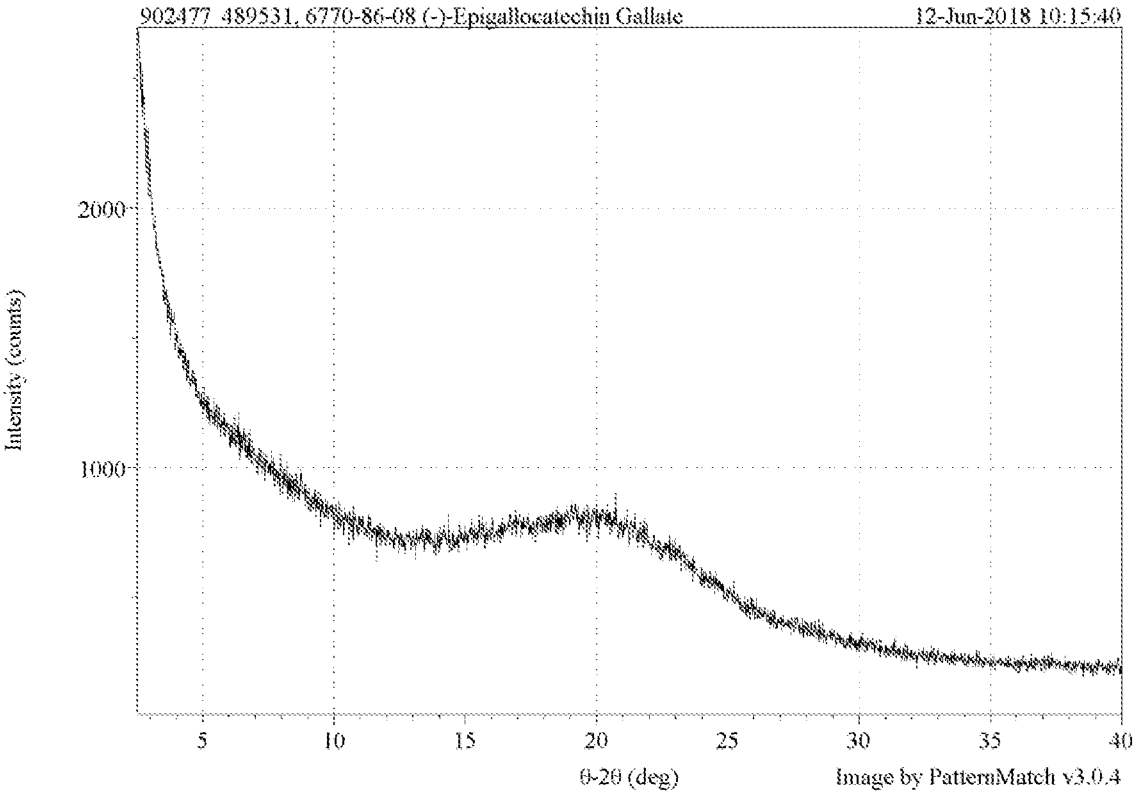


FIG. 53

XRPD pattern for 9:91 (w/w) EGCG/Soluplus® dispersion

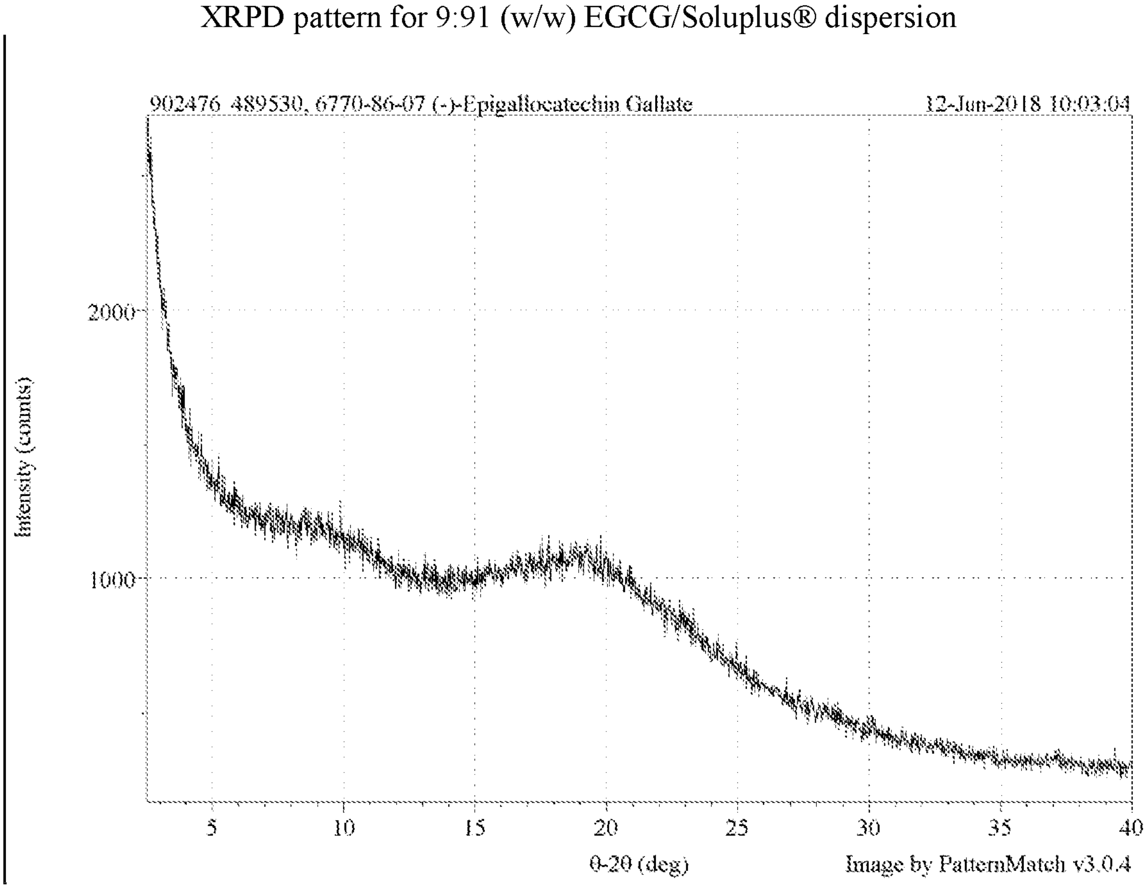


FIG. 54

XRPD pattern for 91:9 (w/w) EGCG/HPMC-AS dispersion

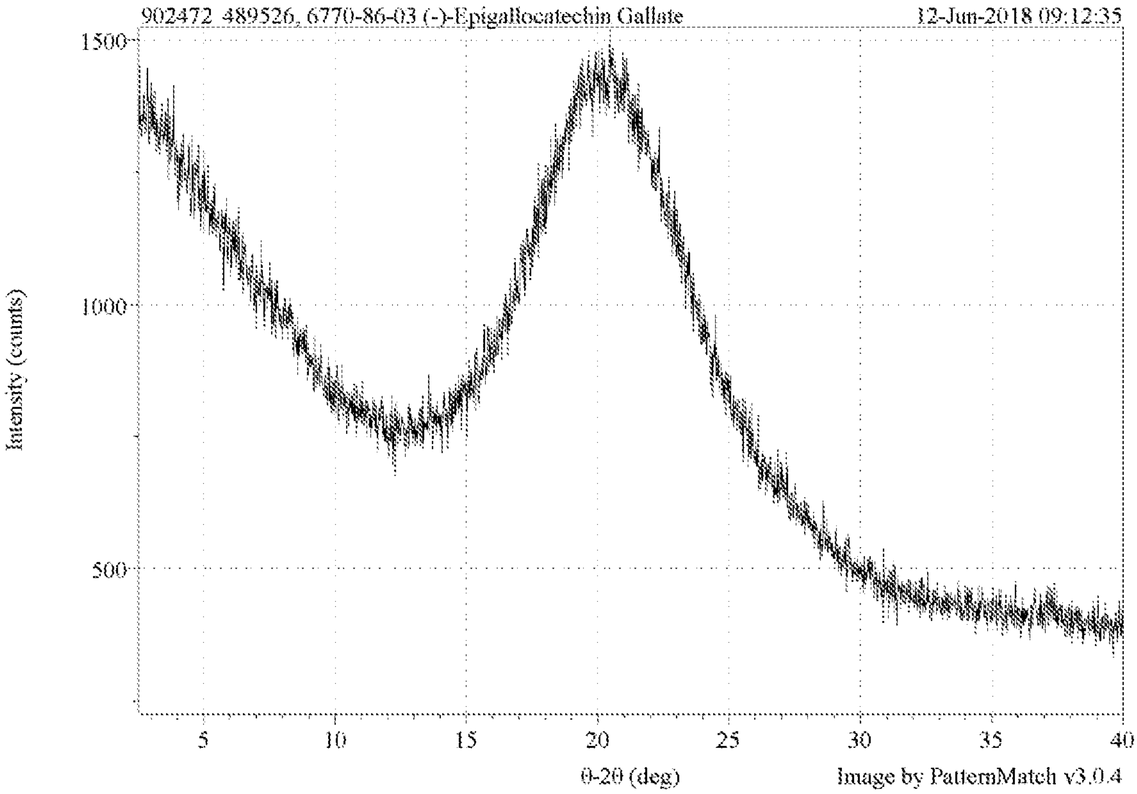


FIG. 55

XRPD pattern for 83:17 (w/w) EGCG/HPMC-AS dispersion

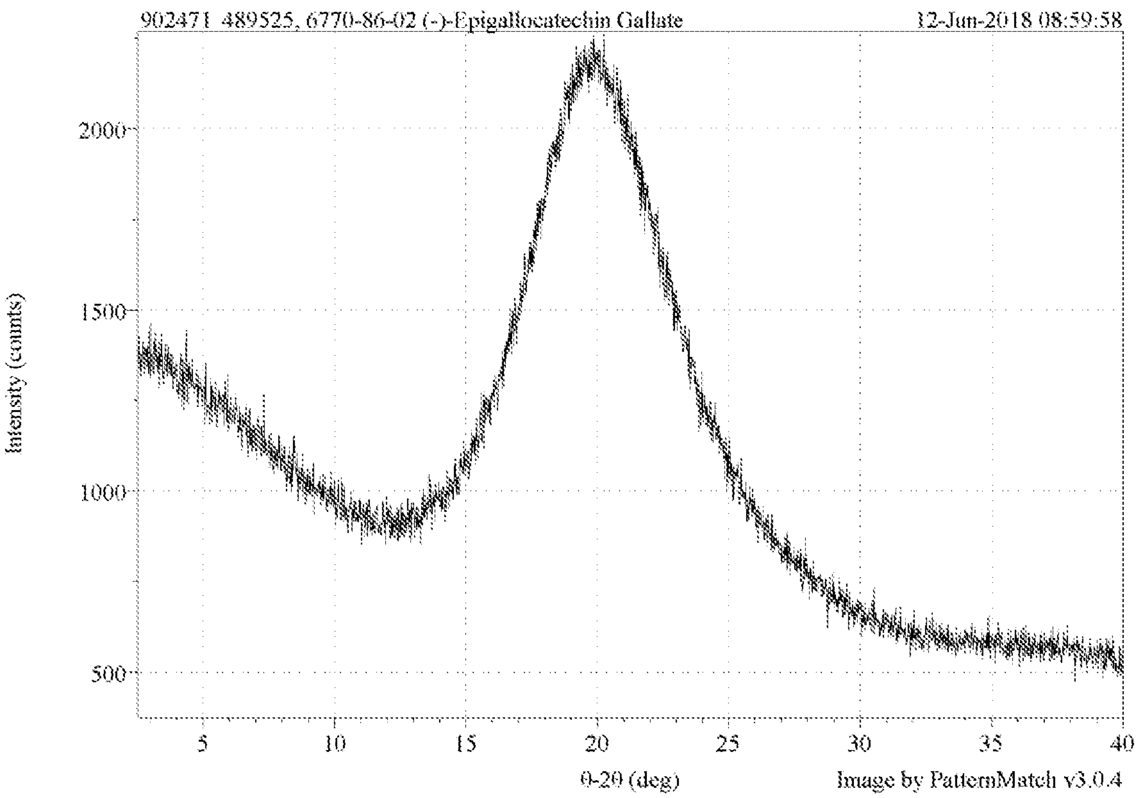


FIG. 56

XRPD pattern for 67:33 (w/w) EGCG/HPMC-AS dispersion

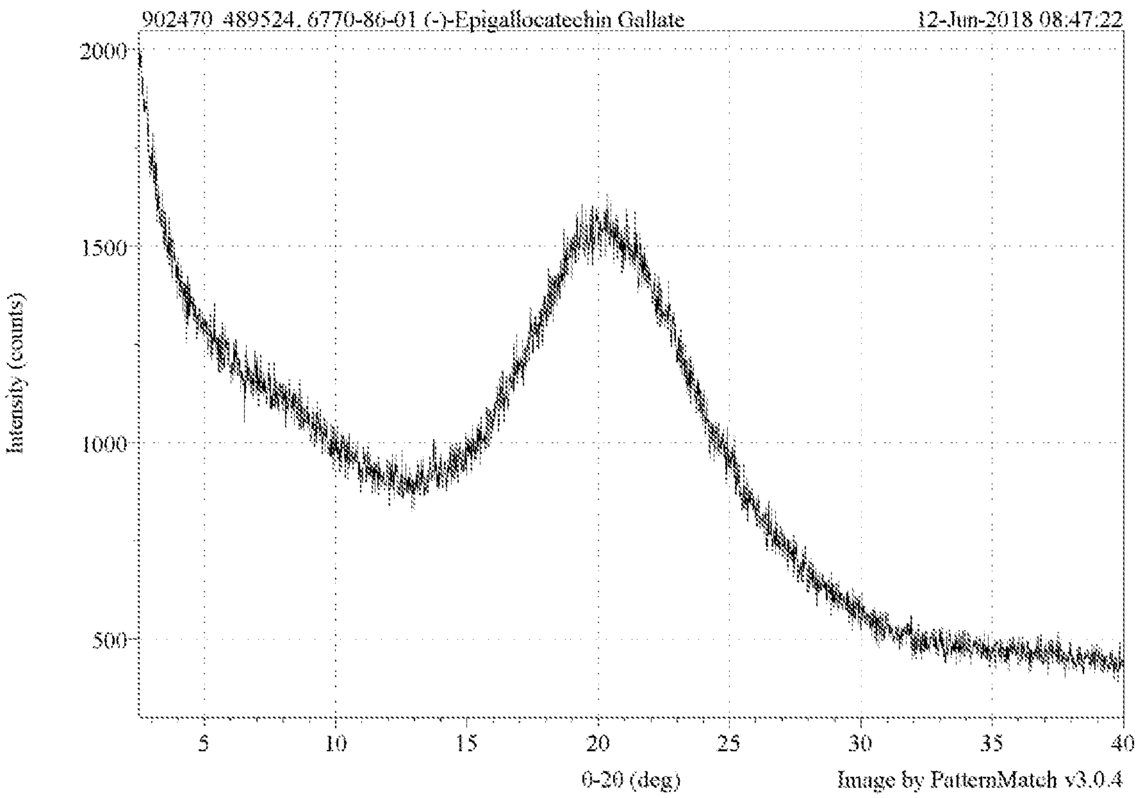


FIG. 57

XRPD pattern for 91:9 (w/w) EGCG/HPMC-P dispersion

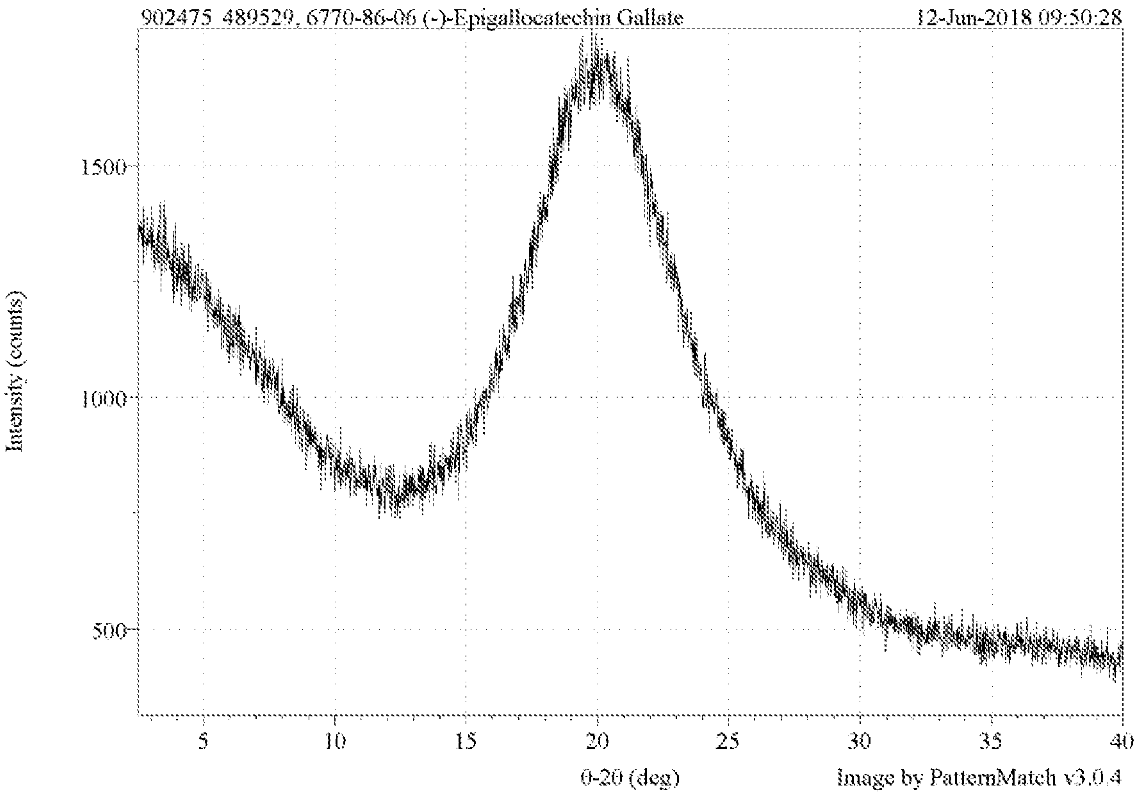


FIG. 58

XRPD pattern for 83:17 (w/w) EGCG/HPMC-P dispersion

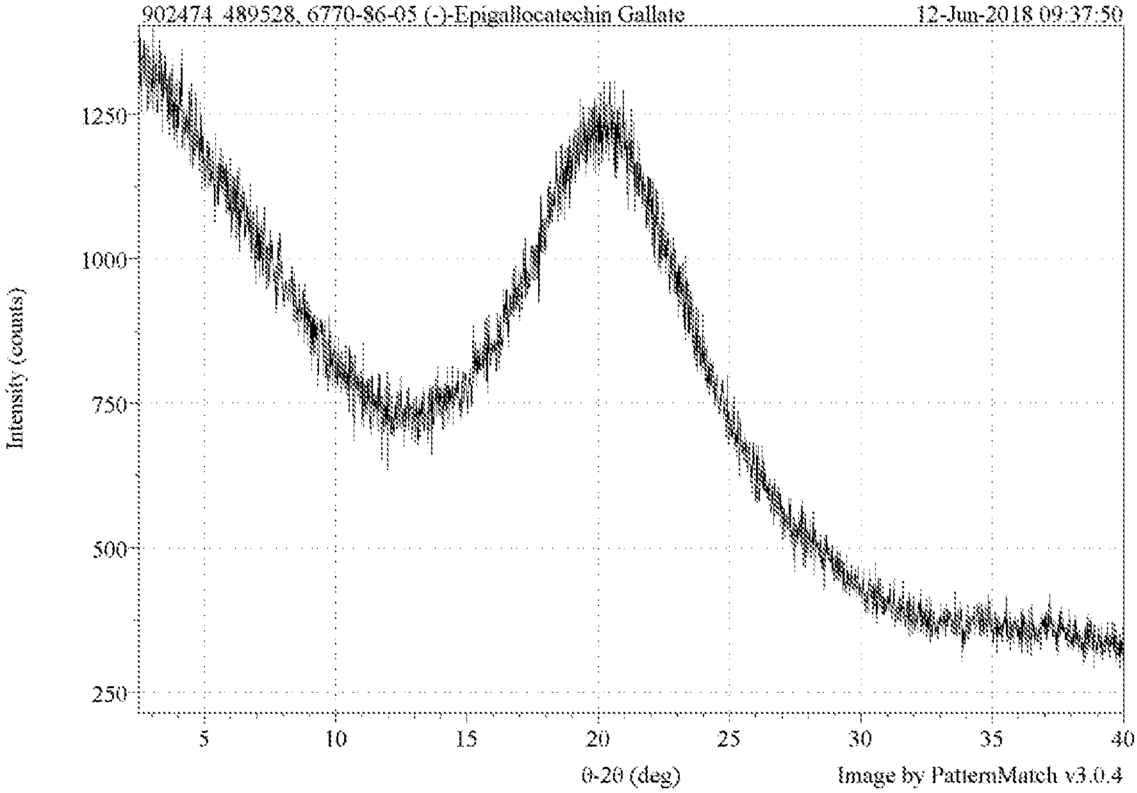


FIG. 59

XRPD pattern for 67:33 (w/w) EGCG/HPMC-P dispersion

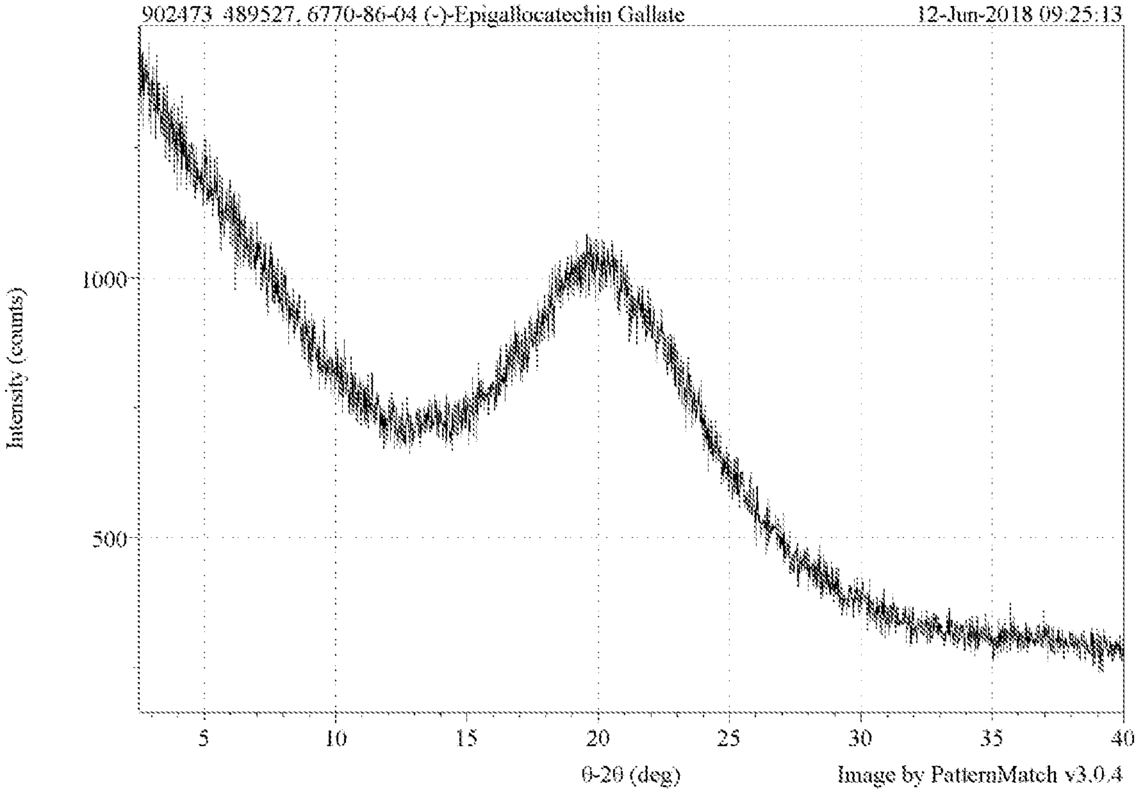


FIG. 60

XRPD pattern for 91:9 (w/w) EGCG/cellulose acetate dispersion

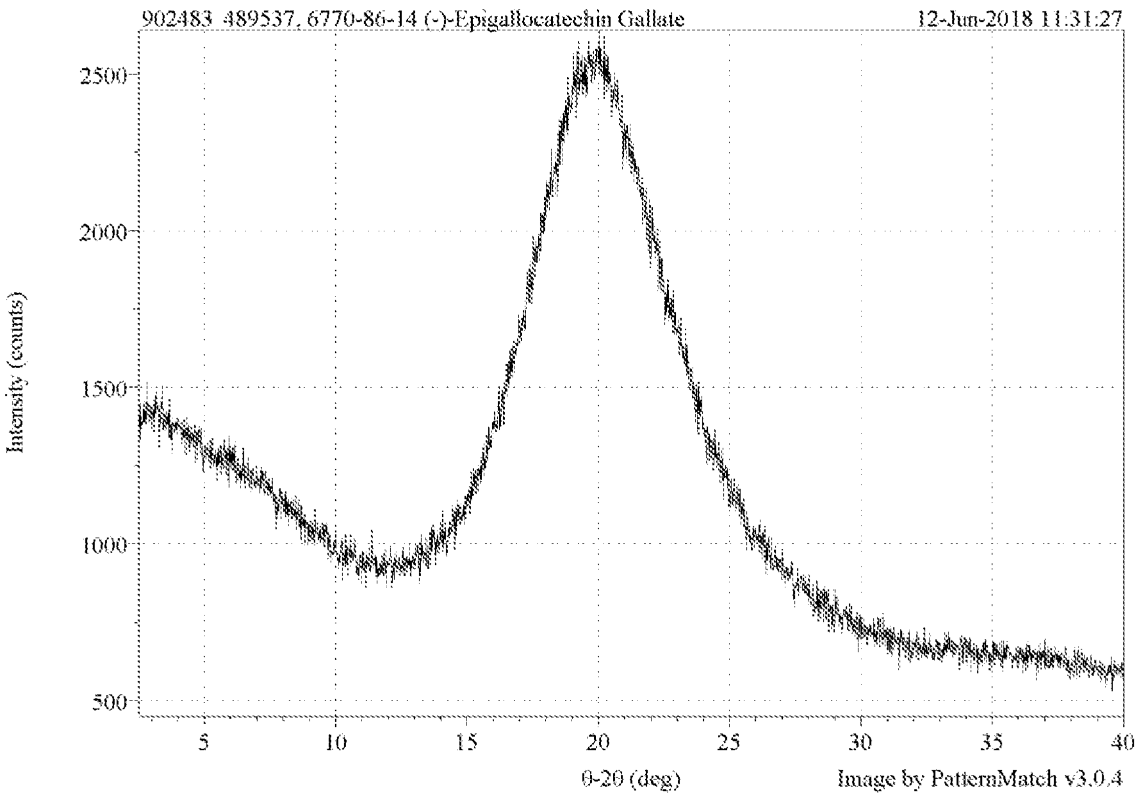


FIG. 61

XRPD pattern for 83:17 (w/w) EGCG/cellulose acetate dispersion

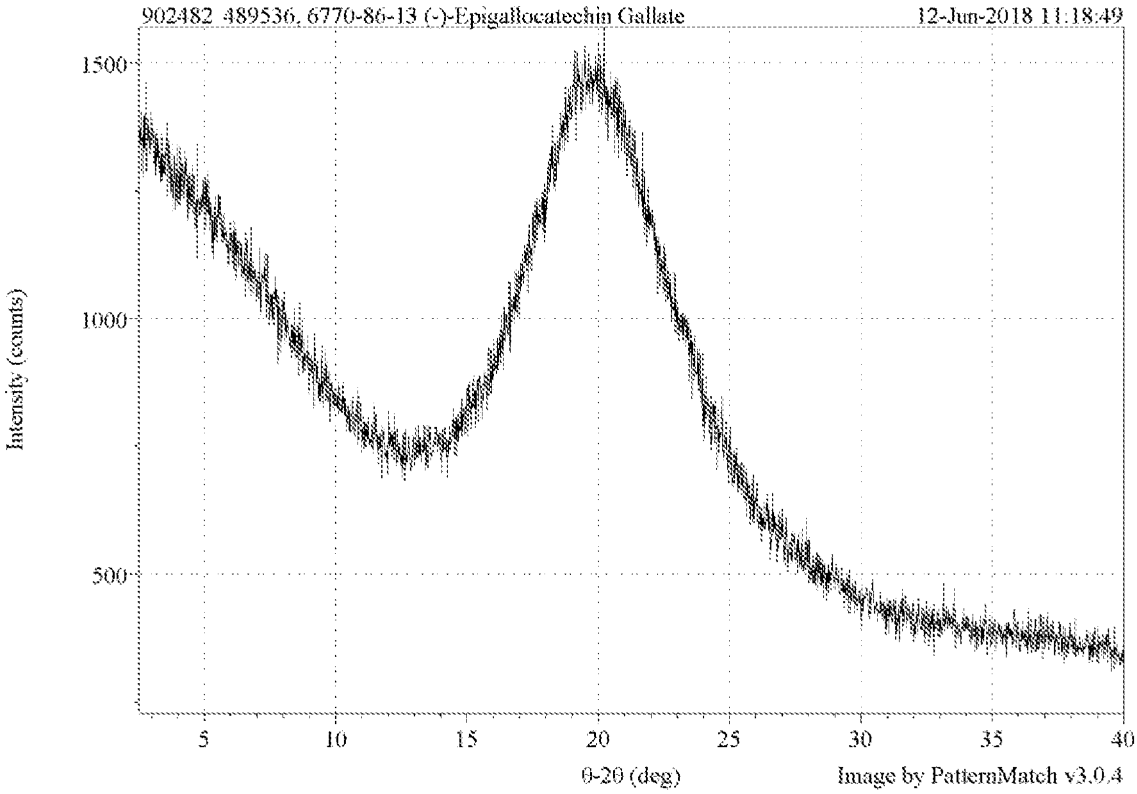


FIG. 62

XRPD pattern for 67:33 (w/w) EGCG/cellulose acetate dispersion

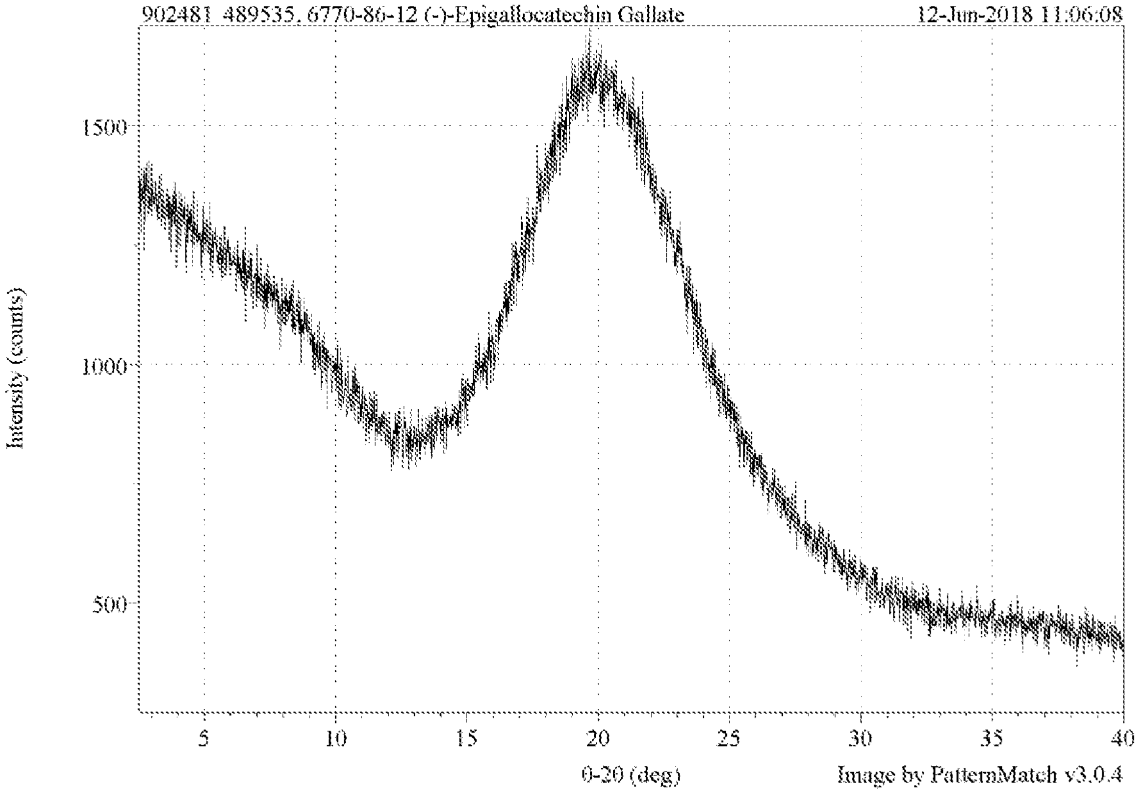


FIG. 63

XRPD pattern for amorphous EGCG

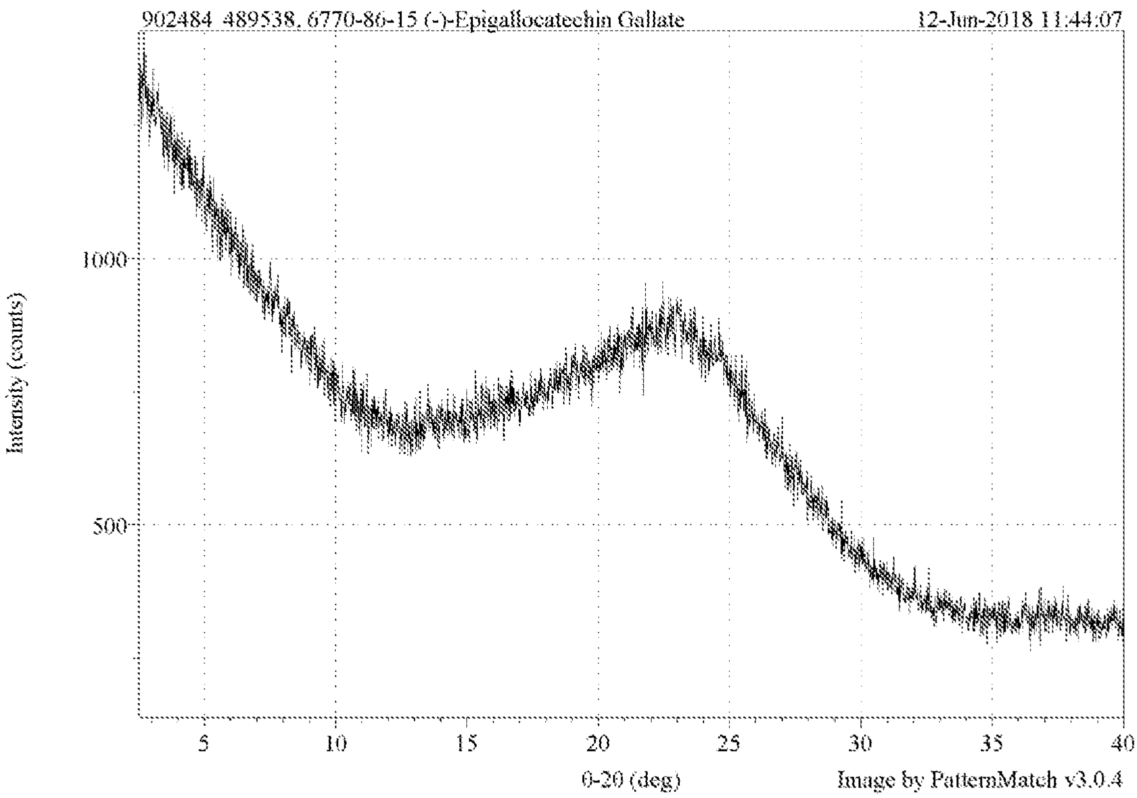


FIG. 64

XRPD pattern for the post-stressing solids of 91:9 (w/w) EGCG/Soluplus® dispersion at about 40°C/75% RH/30 d

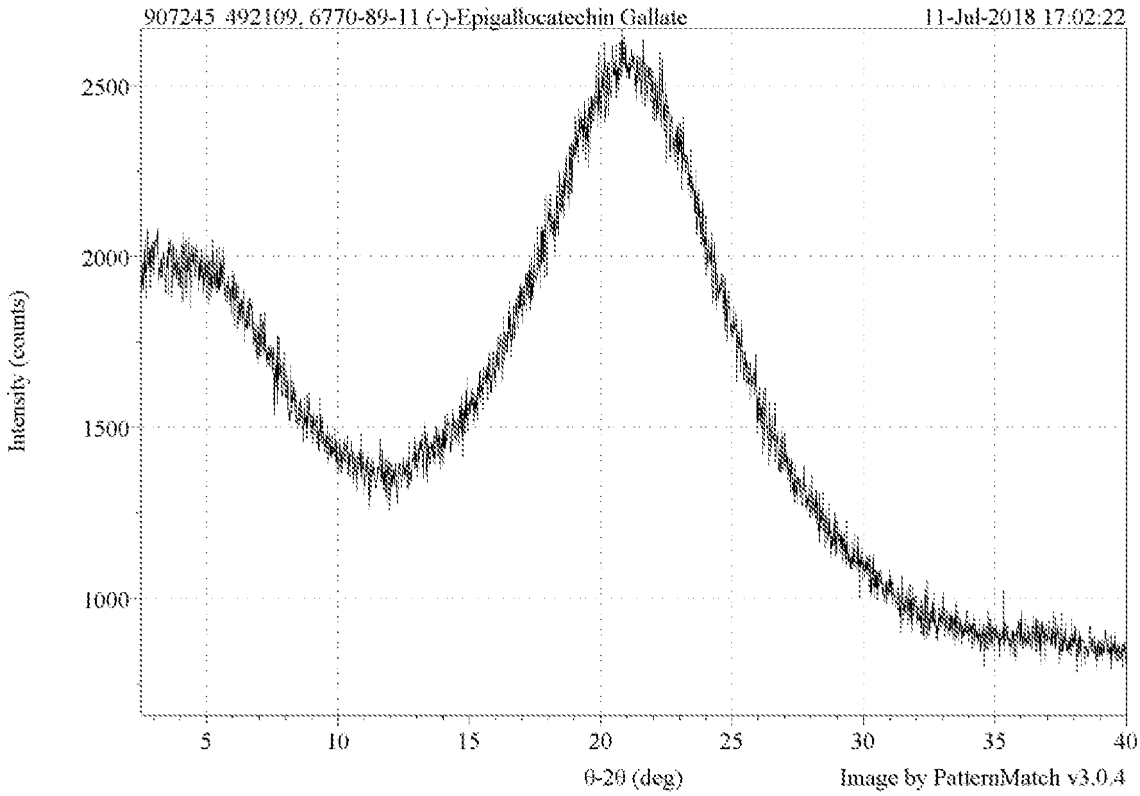


FIG. 65

XRPD pattern for the post-stressing solids of 83:17 (w/w) EGCG/Soluplus® dispersion at about 40°C/75% RH/30 d

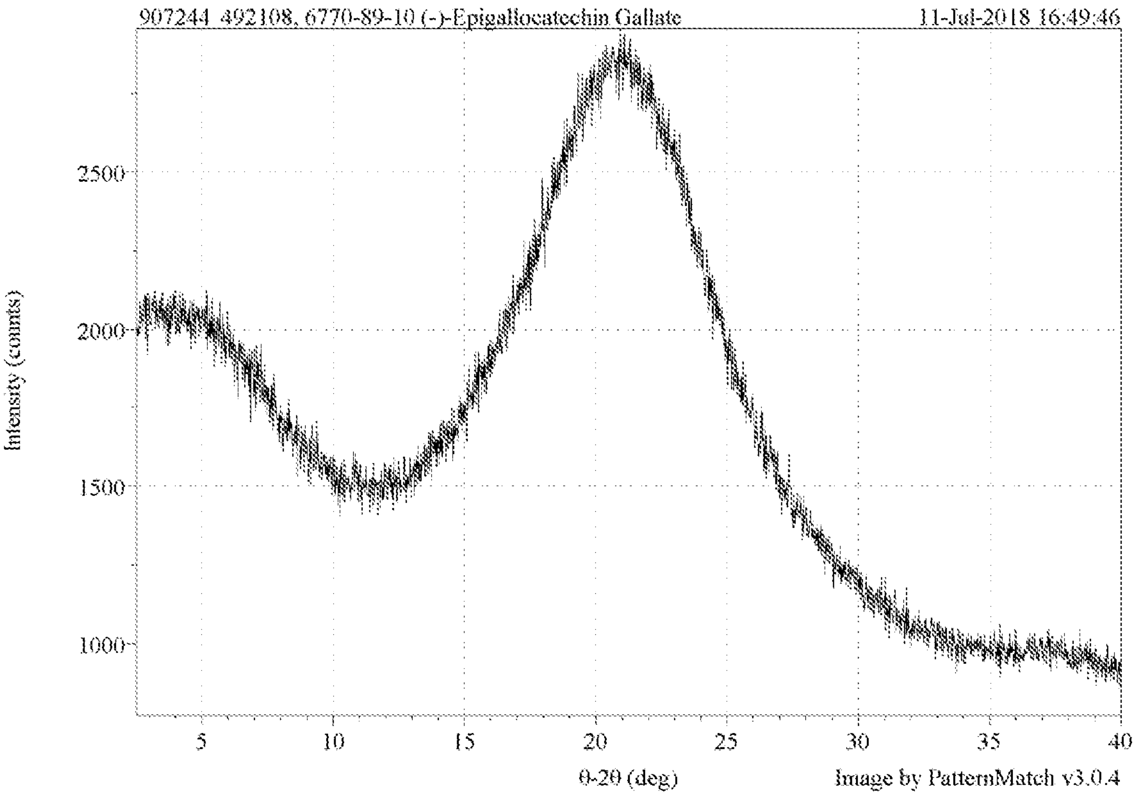


FIG. 66

XRPD pattern for the post-stressing solids of 67:33 (w/w) EGCG/Soluplus® dispersion at about 40°C/75% RH/30 d

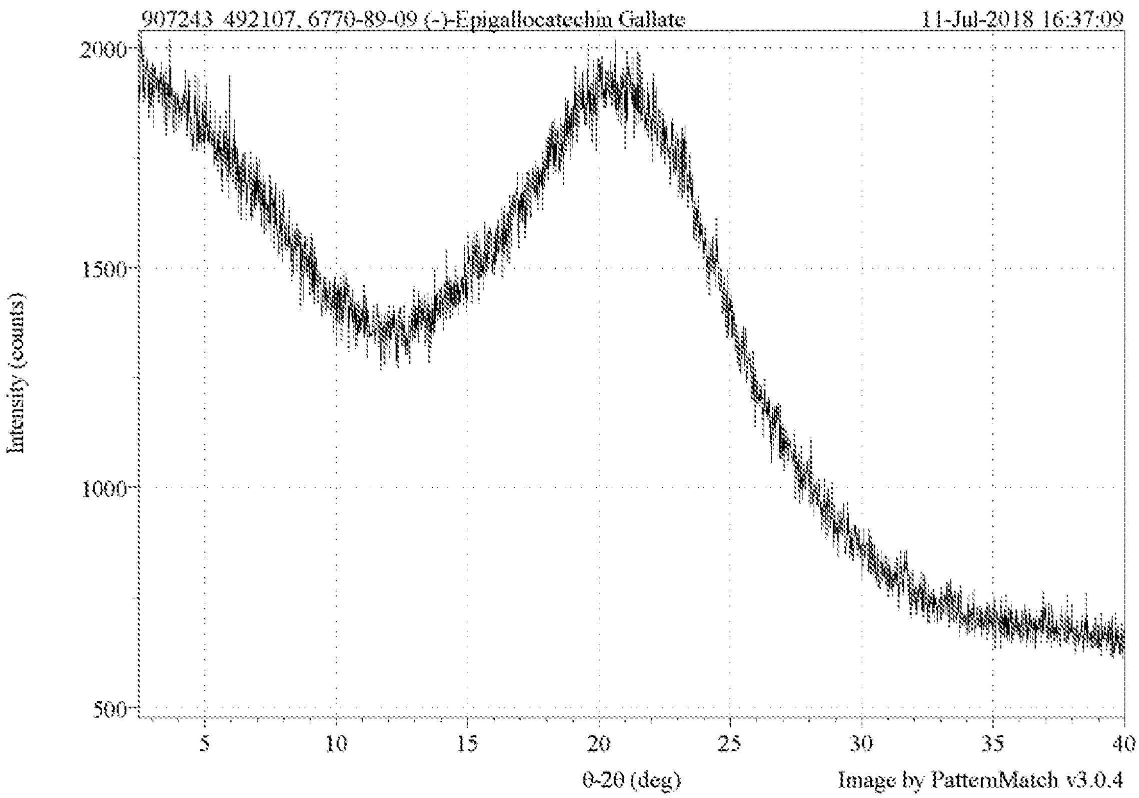


FIG. 67

XRPD pattern for the post-stressing solids of 50:50 (w/w) EGCG/Soluplus® dispersion at about 40°C/75% RH/30 d

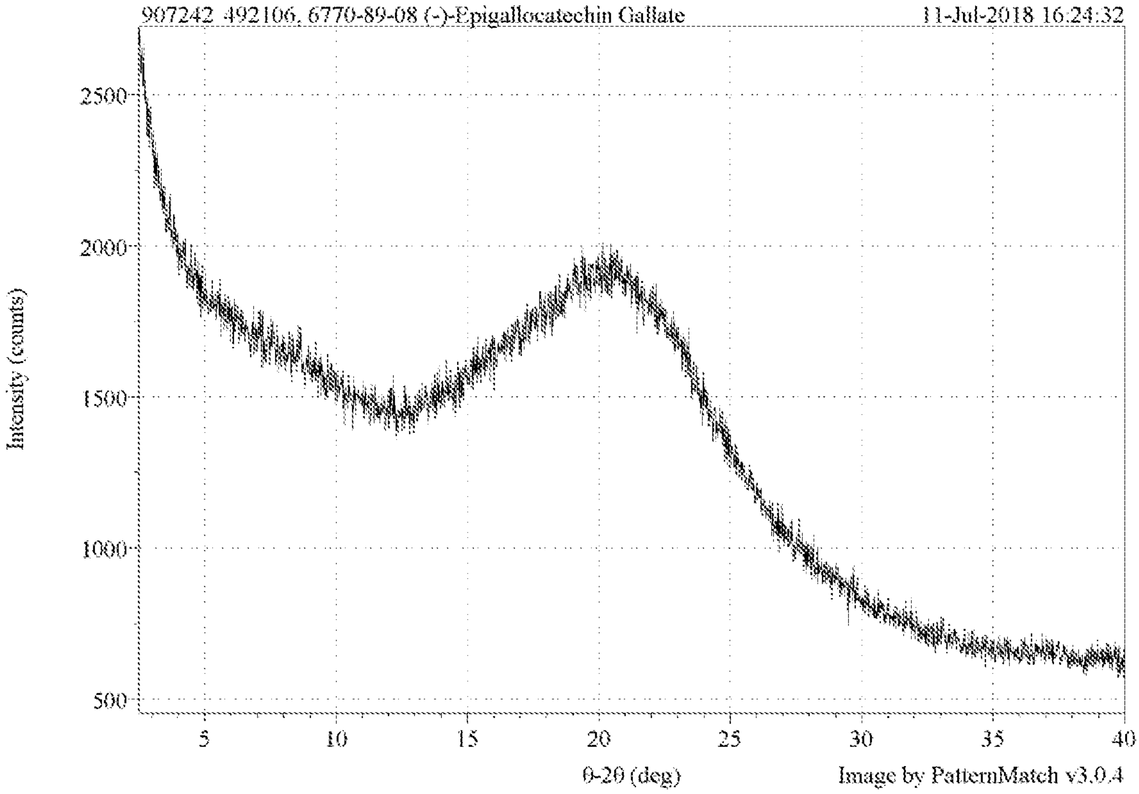


FIG. 68

XRPD pattern for the post-stressing solids of 9:91 (w/w) EGCG/Soluplus® dispersion at about 40°C/75% RH/30 d

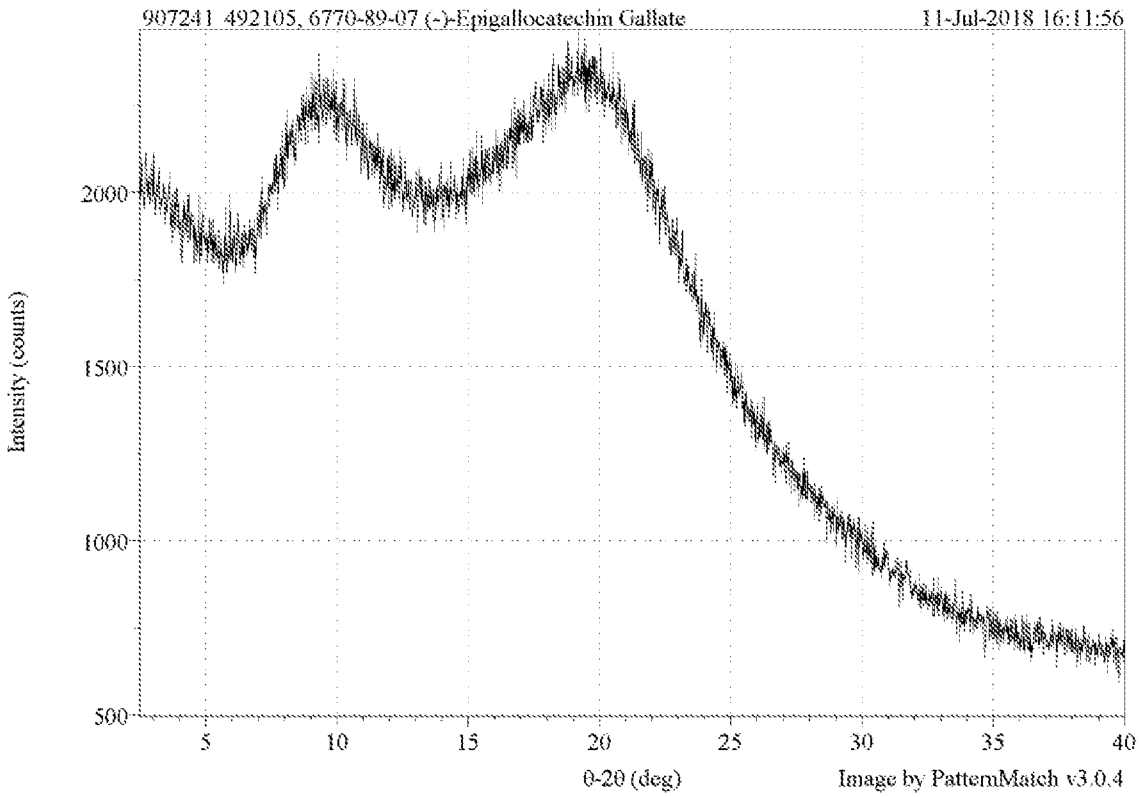


FIG. 69

XRPD pattern for the post-stressing solids of 91:9 (w/w) EGCG/HPMC-AS dispersion at about 40°C/75% RH/30 d

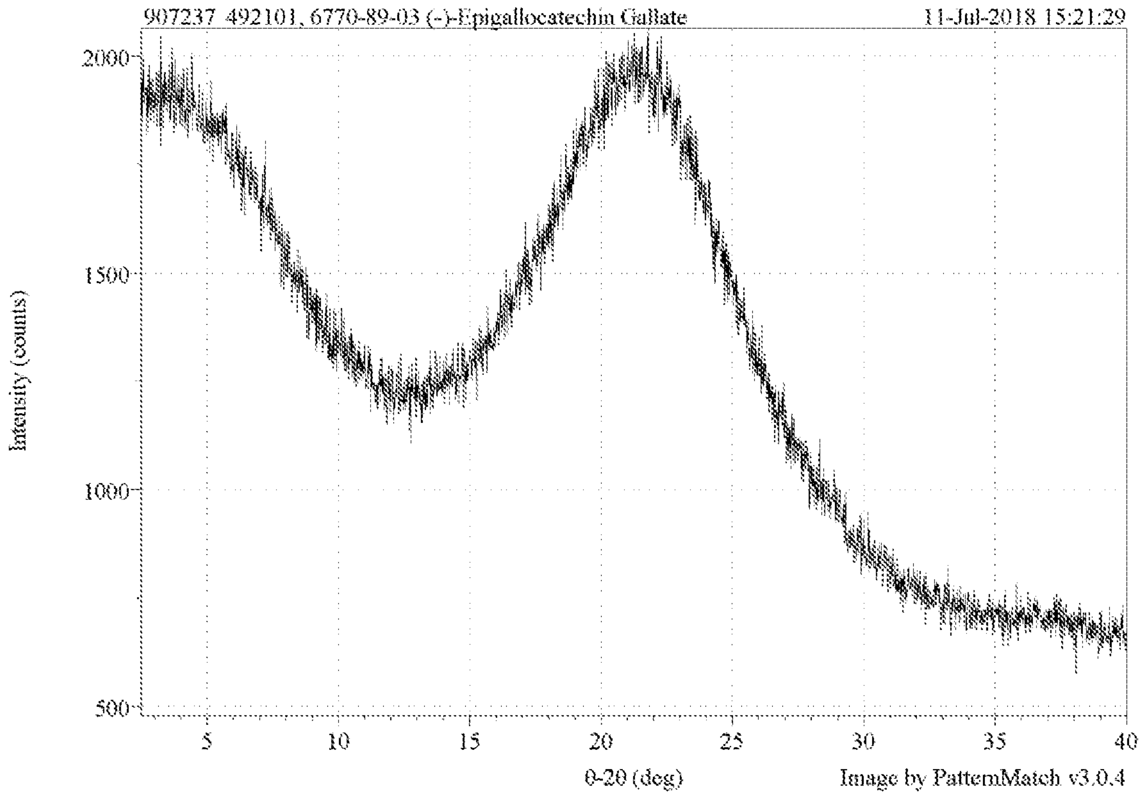


FIG. 70

XRPD pattern for the post-stressing solids of 83:17 (w/w) EGCG/HPMC-AS dispersion at about 40°C/75% RH/30 d

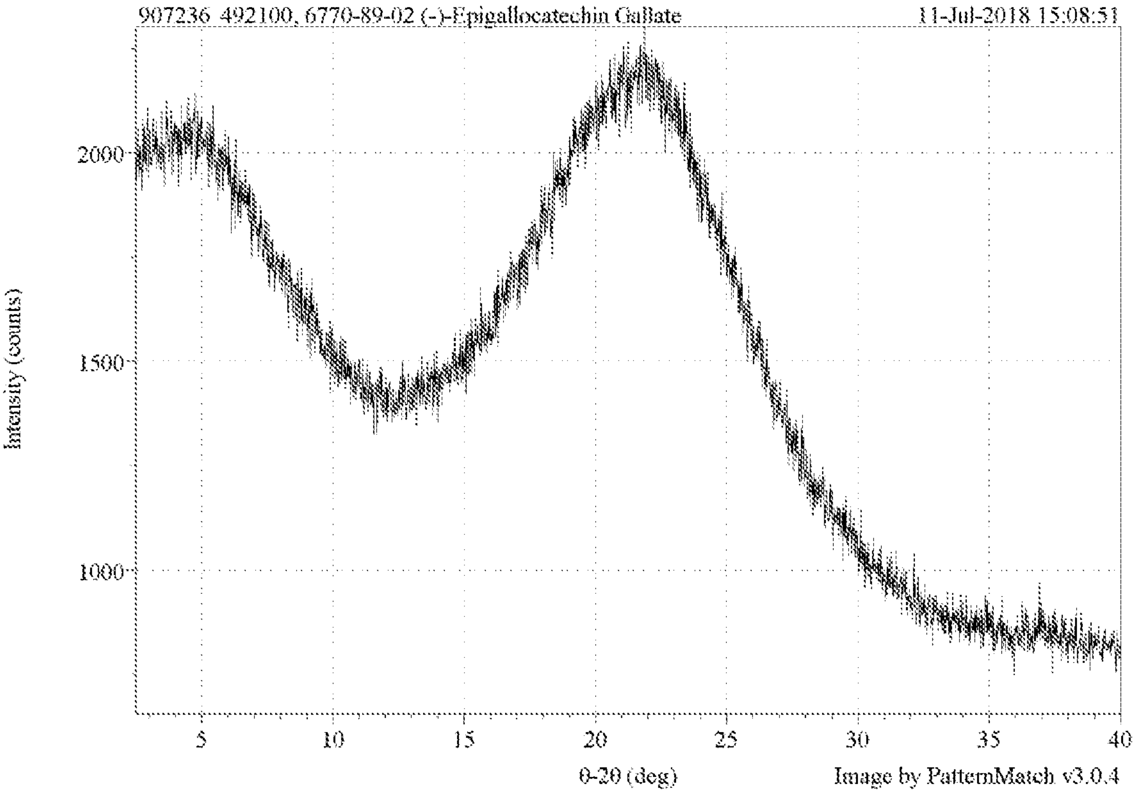


FIG. 71

XRPD pattern for the post-stressing solids of 67:33 (w/w) EGCG/HPMC-AS dispersion at about 40°C/75% RH/30 d

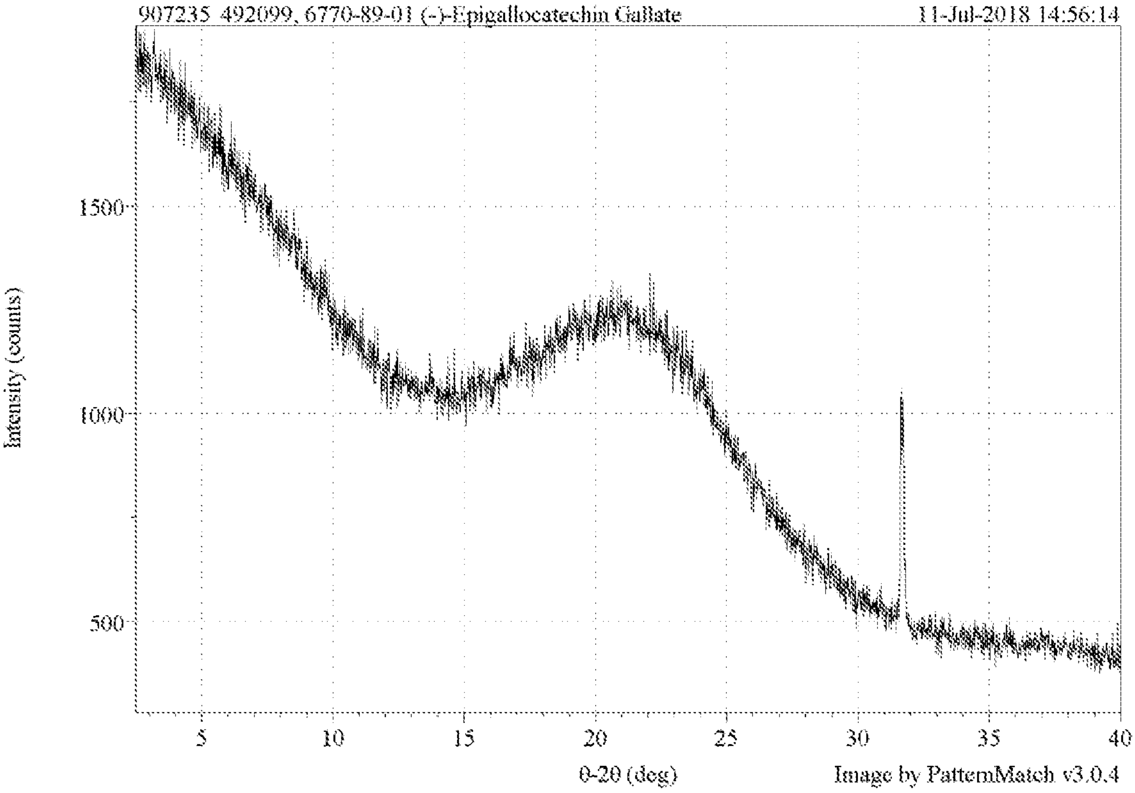


FIG. 72

XRPD pattern for the post-stressing solids of 91:9 (w/w) EGCG/HPMC-P dispersion at about 40°C/75% RH/30 d

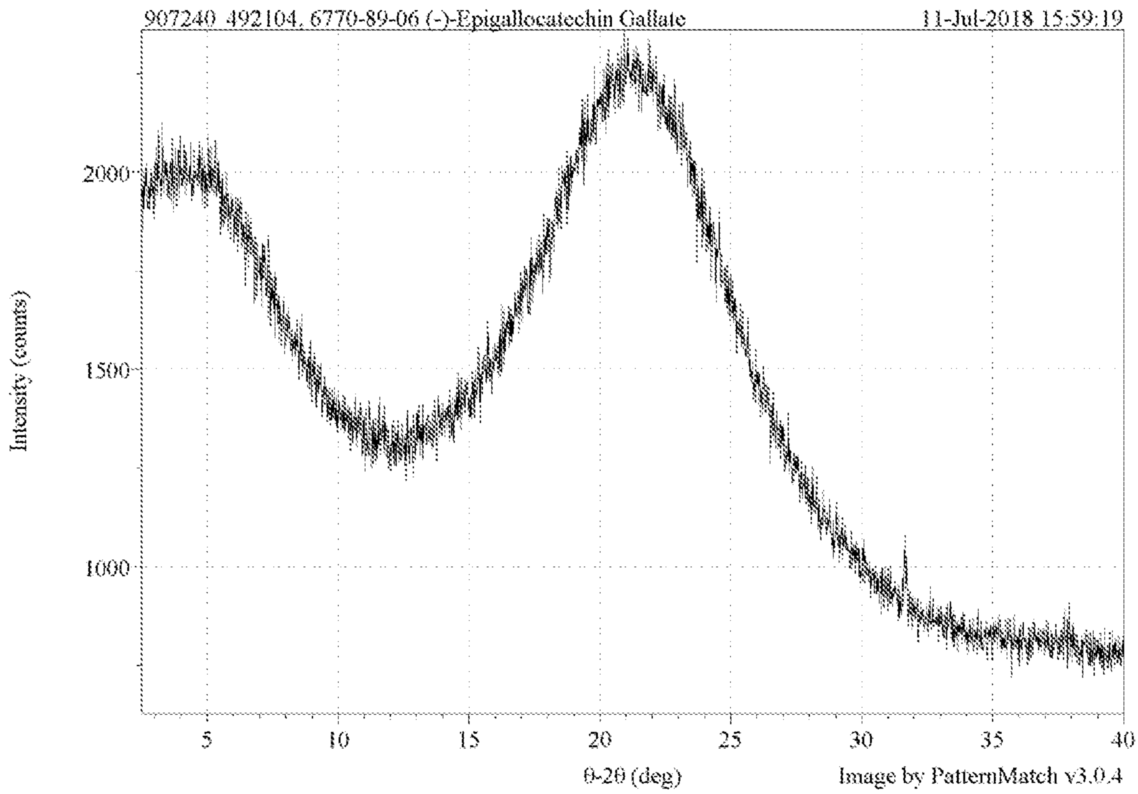


FIG. 73

XRPD pattern for the post-stressing solids of 83:17 (w/w) EGCG/HPMC-P dispersion at about 40°C/75% RH/30 d

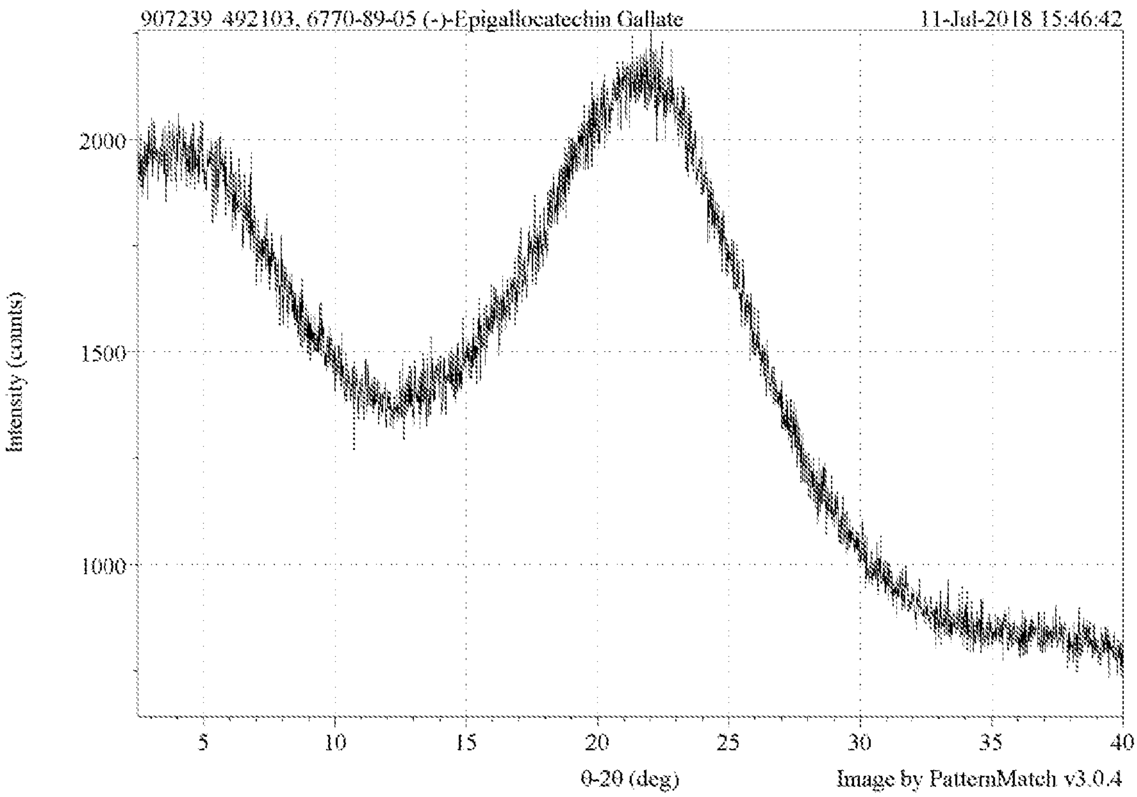


FIG. 74

XRPD pattern for the post-stressing solids of 67:33 (w/w) EGCG/HPMC-P dispersion dispersion at about 40°C/75% RH/30 d

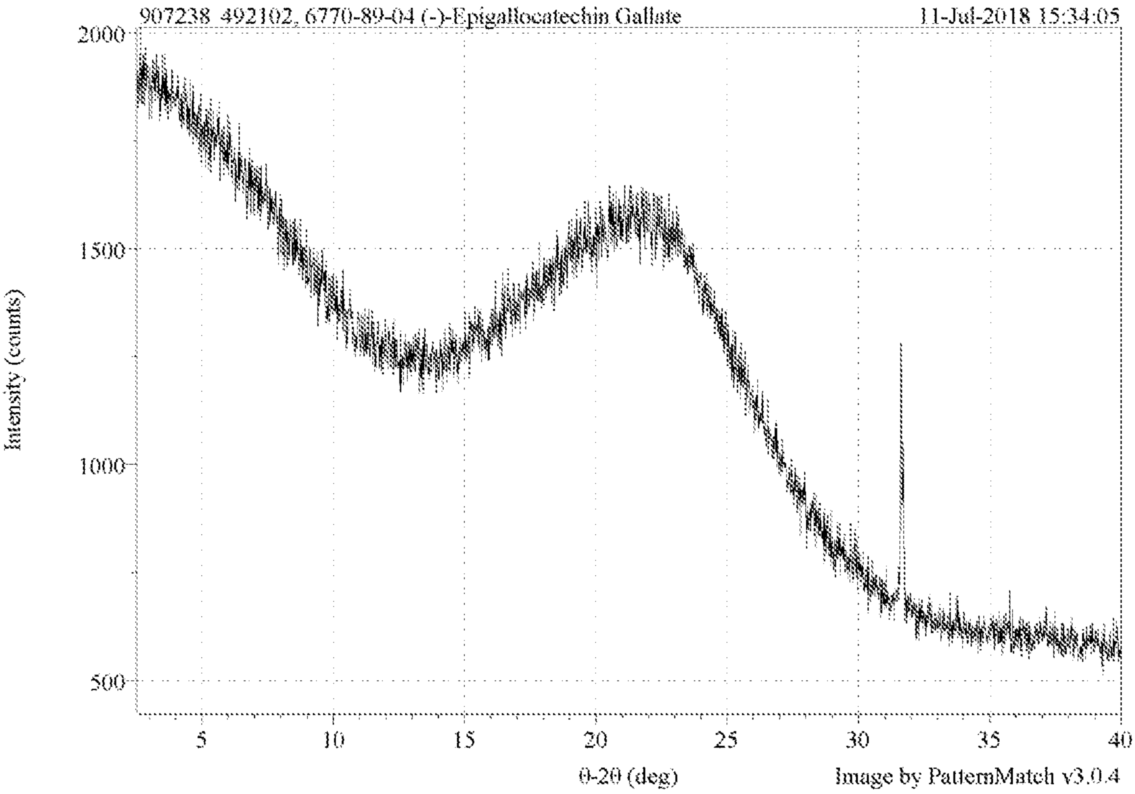


FIG. 75

XRPD pattern for the post-stressing solids of 91:9 (w/w) EGCG/ cellulose acetate dispersion dispersion at about 40°C/75% RH/30 d

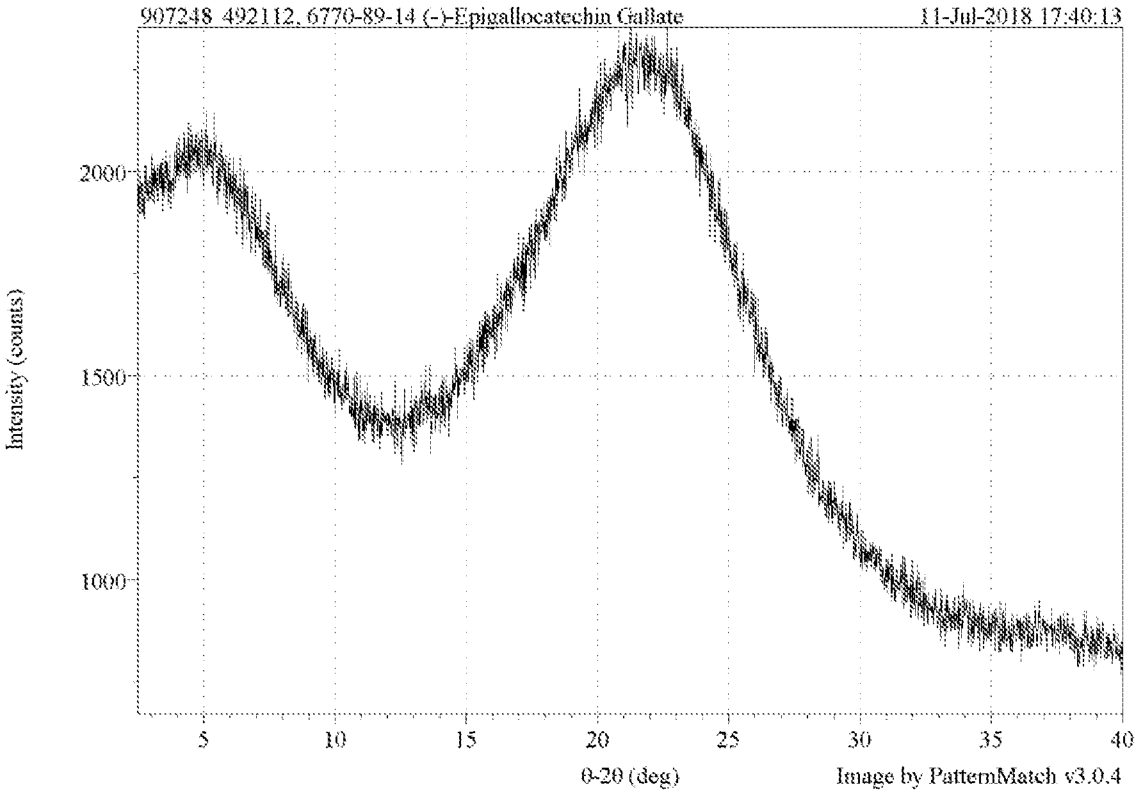


FIG. 76

XRPD pattern for the post-stressing solids of 83:17 (w/w) EGCG/cellulose acetate dispersion at about 40°C/75% RH/30 d

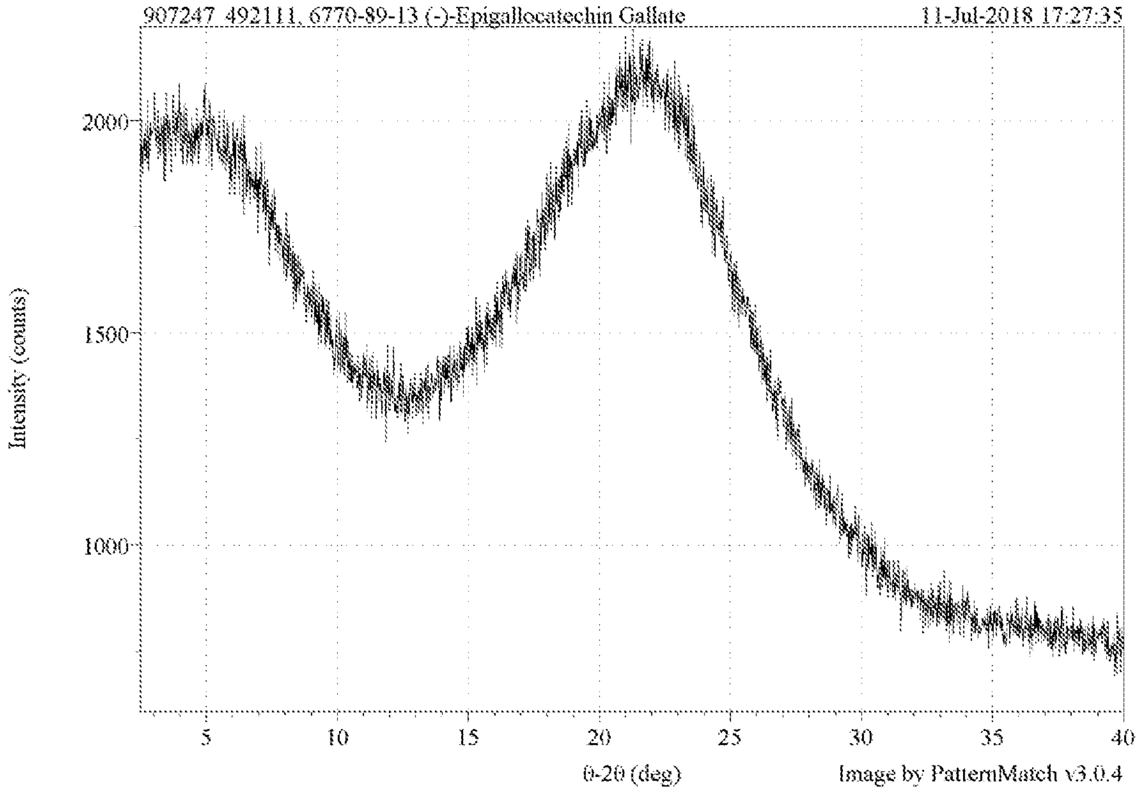


FIG. 77

XRPD pattern for the post-stressing solids of 67:33 (w/w) EGCG/cellulose acetate dispersion at about 40°C/75% RH/30 d

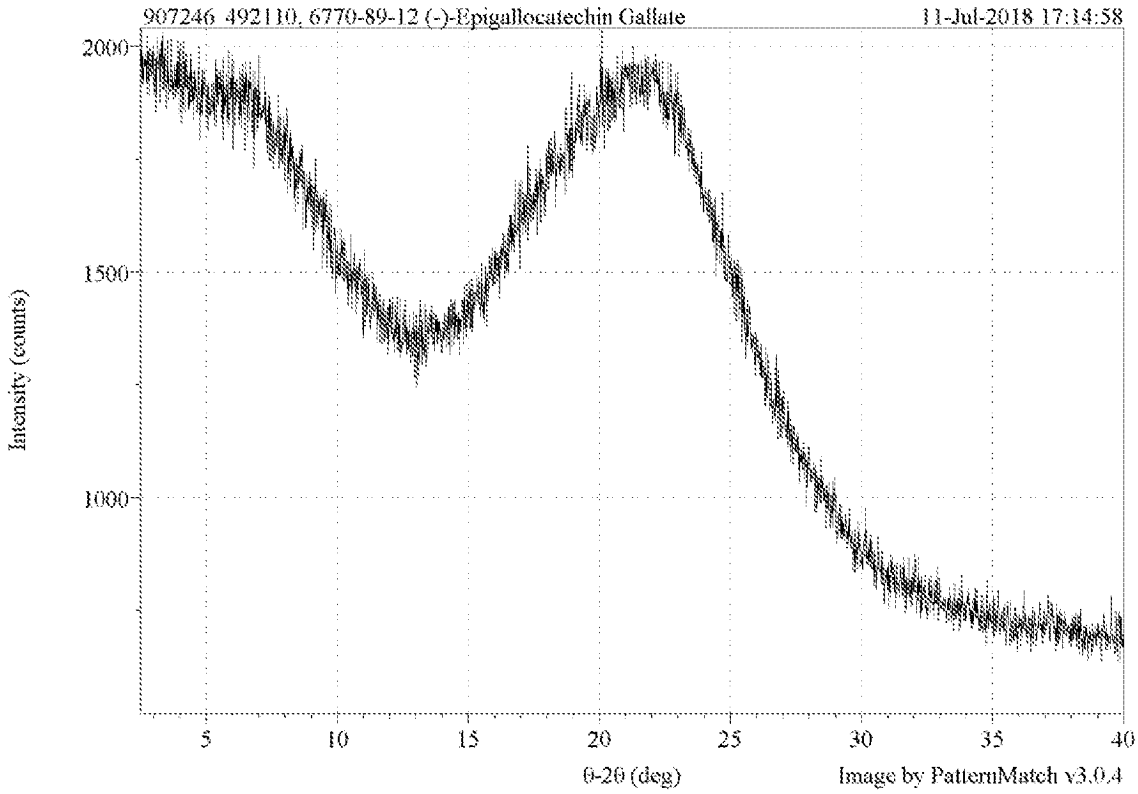


FIG. 78

XRPD pattern for the post-stressing solids of amorphous EGCG at about 40°C/75% RH/30 d

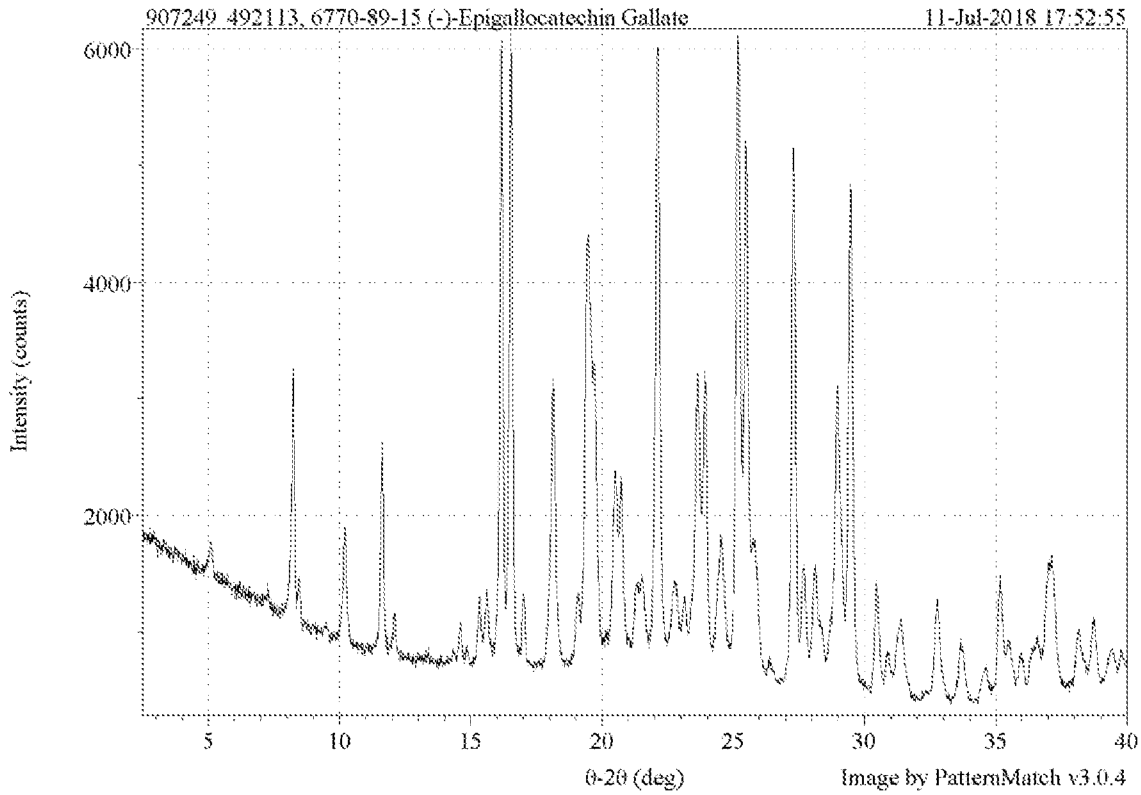


FIG. 79

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62535075
- US 20150265575 A